(19) Europäisches Patentamt
European Patent Office
Office européen des brevets

(11) EP 3 997 097 B1

(12) **EUROPEAN PATENT SPECIFICATION**

(45) Date of publication and mention of the grant of the patent:
**20.03.2024 Bulletin 2024/12**

(21) Application number: **20739606.0**

(22) Date of filing: **07.07.2020**

(51) International Patent Classification (IPC):
***C07D 513/22*** *(2006.01)* ***A61P 35/00*** *(2006.01)*
***A61K 31/553*** *(2006.01)* ***C07D 513/20*** *(2006.01)*

(52) Cooperative Patent Classification (CPC):
**C07D 513/20; A61P 35/00; C07D 513/22**

(86) International application number:
**PCT/EP2020/069097**

(87) International publication number:
**WO 2021/005043 (14.01.2021 Gazette 2021/02)**

(54) **MACROCYCLIC SPIROCYCLE DERIVATIVES AS MCL-1 INHIBITORS**

MAKROCYCLISCHE SPIROCYCLUS-DERIVATE ALS MCL-1-INHIBITOREN

DÉRIVÉS SPIROCYCLIQUES MACROCYCLIQUES EN TANT QU'INHIBITEURS DE MCL-1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.07.2019 EP 19185177**
**19.12.2019 EP 19218032**

(43) Date of publication of application:
**18.05.2022 Bulletin 2022/20**

(73) Proprietor: **Janssen Pharmaceutica NV**
**2340 Beerse (BE)**

(72) Inventors:
- **ROMBOUTS, Frederik, Jan, Rita**
**2340 Beerse (BE)**
- **DIELS, Gaston, Stanislas, Marcella**
**2340 Beerse (BE)**
- **JERHAOUI, Soufyan**
**2340 Beerse (BE)**
- **SURKYN, Michel**
**2340 Beerse (BE)**
- **VAN ROOSBROECK, Yves, Emiel, Maria**
**2340 Beerse (BE)**
- **JOUFFROY, Matthieu, Dominique**
**2340 Beerse (BE)**

(74) Representative: **Lenaerts, Philip**
**Johnson & Johnson Patent Law Department**
**Turnhoutseweg 30**
**2340 Beerse (BE)**

(56) References cited:
**WO-A1-2018/183418 WO-A1-2019/046150**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

FIELD OF THE INVENTION

The present invention relates to pharmaceutical agents useful for therapy and/or prophylaxis in a subject, pharmaceutical composition comprising such compounds, and their use as MCL-1 inhibitors, useful for treating or preventing diseases such as cancer.

BACKGROUND OF THE INVENTION

Cellular apoptosis or programmed cell death is critical to the development and homeostasis of many organs including the hematopoietic system. Apoptosis can be initiated via the extrinsic pathway, which is mediated by death receptors, or by the intrinsic pathway using the B cell lymphoma (BCL-2) family of proteins. Myeloid cell leukemia-1 (MCL-1) is a member of the BCL-2 family of cell survival regulators and is a critical mediator of the intrinsic apoptosis pathway. MCL-1 is one of five principal anti-apoptotic BCL-2 proteins (MCL-1, BCL-2, BCL-XL, BCL-w, and BFL1/A1) responsible for maintaining cell survival. MCL-1 continuously and directly represses the activity of the pro-apoptotic BCL-2 family proteins Bak and Bax and indirectly blocks apoptosis by sequestering BH3 only apoptotic sensitizer proteins such as Bim and Noxa. The activation of Bak/Bax following various types of cellular stress leads to aggregation on the mitochondrial outer membrane and this aggregation facilitates pore formation, loss of mitochondrial outer membrane potential, and subsequent release of cytochrome C into the cytosol. Cytosolic cytochrome C binds Apaf-1 and initiates recruitment of procaspase 9 to form apoptosome structures (Cheng et al. eLife 2016; 5: e17755). The assembly of apoptosomes activates the executioner cysteine proteases 3/7 and these effector caspases then cleave a variety of cytoplasmic and nuclear proteins to induce cell death (Julian et al. Cell Death and Differentiation 2017; 24, 1380-1389).

Avoiding apoptosis is an established hallmark of cancer development and facilitates the survival of tumor cells that would otherwise be eliminated due to oncogenic stresses, growth factor deprivation, or DNA damage (Hanahan and Weinberg. Cell 2011;1-44). Thus, unsurprisingly, MCL-1 is highly upregulated in many solid and hematologic cancers relative to normal non-transformed tissue counterparts. The overexpression of MCL-1 has been implicated in the pathogenesis of several cancers where it correlated with poor outcome, relapse, and aggressive disease. Additionally, overexpression of MCL-1 has been implicated in the pathogenesis of the following cancers: prostate, lung, pancreatic, breast, ovarian, cervical, melanoma, B-cell chronic lymphocytic leukemia (CLL), acute myeloid leukemia (AML), and acute lymphoblastic leukemia (ALL). The human MCL-1 genetic locus (1q21) is frequently amplified in tumors and quantitatively increases total MCL-1 protein levels (Beroukhim et al. Nature 2010;463 (7283) 899-905). MCL-1 also mediates resistance to conventional cancer therapeutics and is transcriptionally upregulated in response to inhibition of BCL-2 function (Yecies et al. Blood 2010;115 (16)3304-3313).

A small molecule BH3 inhibitor of BCL-2 has demonstrated clinical efficacy in patients with chronic lymphocytic leukemia and is FDA approved for patients with CLL or AML (Roberts et al. NEJM 2016;374:311-322). The clinical success of BCL-2 antagonism led to the development of several MCL-1 BH3 mimetics that show efficacy in preclinical models of both hematologic malignancies and solid tumors (Kotschy et al. Nature 2016;538 477-486, Merino et al. Sci. Transl. Med;2017 (9)).

MCL-1 regulates several cellular processes in addition to its canonical role in mediating cell survival including mitochondrial integrity and non-homologous end joining following DNA damage (Chen et al. JCI 2018;128(1):500-516). The genetic loss of MCL-1 shows a range of phenotypes depending on the developmental timing and tissue deletion. MCL-1 knockout models reveal there are multiple roles for MCL-1 and loss of function impacts a wide range of phenotypes. Global MCL-1-deficient mice display embryonic lethality and studies using conditional genetic deletion have reported mitochondrial dysfunction, impaired activation of autophagy, reductions in B and T lymphocytes, increased B and T cell apoptosis, and the development of heart failure/ cardiomyopathy (Wang et al. Genes and Dev 2013;27 1351-1364, Steimer et al. Blood 2009;(113) 2805-2815).

WO2018178226 discloses MCL-1 inhibitors and methods of use thereof.

WO2017182625 discloses macrocyclic MCL-1 inhibitors for treating cancer.

WO2018178227 discloses the synthesis of MCL-1 inhibitors.

WO2019046150 discloses macrocyclic compounds that inhibit mcl-1 protein.

WO2016033486 discloses tetrahydronaphthalene derivatives that inhibit mcl-1 protein.

WO2019036575, WO2017147410, and WO2018183418 disclose compounds that inhibit mcl-1 protein.

WO2019222112 discloses MCL-1 inhibitors for treating cancer.

WO2019046150 discloses macrocyclic compounds that inhibit mcl-1 protein.

WO2019036575, WO2017147410, and WO2018183418 disclose compounds that inhibit mcl-1 protein.

WO2020097577 discloses spiro-sulfonamide derivatives as inhibitors of myeloid cell leukemia-1 (MCL-1) protein.

There remains a need for MCL-1 inhibitors, useful for the treatment or prevention of cancers such as prostate, lung, pancreatic, breast, ovarian, cervical, melanoma, B-cell chronic lymphocytic leukemia (CLL), acute myeloid leukemia (AML), and acute lymphoblastic leukemia (ALL).

SUMMARY OF THE INVENTION

The present invention concerns novel compounds of Formula (I):

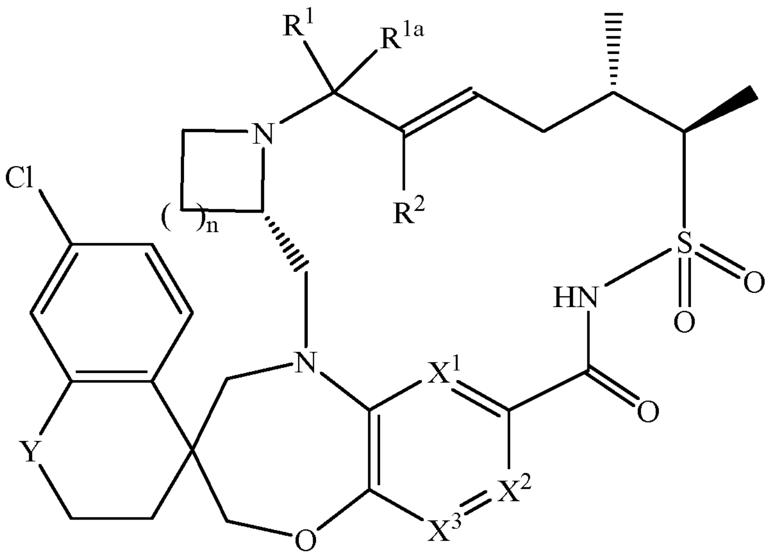

(I)

wherein

$R^1$ represents hydrogen; $-CH_2OR^a$; $-CH_2F$; or $-CH_2NR^bR^c$;

$R^{1a}$ represents hydrogen; $-CH_2OR^a$; $-CH_2F$; or $-CH_2NR^bR^c$;

$R^a$ is selected from the group consisting of hydrogen; $C_{1-4}$alkyl; $Het^a$; $C_{1-4}$alkyl substituted with one substitutent selected from the group consisting of $-C(=O)-NR^dR^e$, $-C(=O)-OR^f$, $C_{3-6}$cycloalkyl, $Het^b$, and $Het^c$; and $C_{2-4}$alkyl substituted with one substitutent selected from the group consisting of $-(OCH_2CH_2)_m-OCH_3$, $Het^d$, and $-NR^dR^e$;

$R^b$ and $R^c$ are each independently selected from the group consisting of hydrogen, $C_{1-4}$alkyl, and $C_{2-4}$alkyl substituted with one $Het^1$; or $R^b$ and $R^c$ are taken together to form together with the N-atom to which they are attached a monocyclic 4- to 7-membered fully saturated heterocyclyl containing at least one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing at least one N-atom and optionally one or two additional heteroatoms each independently selected from O, S, and N, wherein said S-atom might be substituted to form S(=O) or $S(=O)_2$; wherein said monocyclic or fused bicyclic heterocyclyl is optionally substituted on one nitrogen with one $C_{1-4}$alkyl; wherein said monocyclic or fused bicyclic heterocyclyl is optionally substituted on one carbon atom with one or two substituents each independently selected from the group consisting of $C_{1-4}$alkyl, oxo and halo;

$R^d$ and $R^e$ are each independently selected from the group consisting of hydrogen, $C_{1-4}$alkyl, and $C_{2-4}$alkyl substituted with one $Het^1$; or $R^d$ and $R^e$ are taken together to form together with the N-atom to which they are attached a monocyclic 4- to 7-membered fully saturated heterocyclyl containing at least one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing at least one N-atom and optionally one or two additional heteroatoms each independently selected from O, S, and N, wherein said S-atom might be substituted to form S(=O) or $S(=O)_2$; wherein said monocyclic or fused bicyclic heterocyclyl is optionally substituted on one nitrogen with one $C_{1-4}$alkyl; wherein said monocyclic or fused bicyclic heterocyclyl is optionally substituted on one carbon atom with one or two substituents each independently selected from the group consisting of $C_{1-4}$alkyl, oxo and halo;

$R^f$ represents hydrogen or $C_{1-4}$alkyl;

$R^2$ represents hydrogen or fluoro;

$Het^a$ represents a C-linked 5- or 6-membered monocyclic aromatic heterocyclyl containing one, two or three heteroatoms each independently selected from O, S, and N; wherein one carbon atom is optionally substituted with one $C_{1-4}$alkyl;

$Het^b$ represents a C-linked 4- to 7-membered monocyclic fully saturated heterocyclyl containing one or two heteroatoms each independently selected from O, S, and N; wherein said S-atom might be substituted to form S(=O) or $S(=O)_2$;

$Het^c$ represents a C-linked 5- or 6-membered monocyclic aromatic heterocyclyl containing one, two or three heteroatoms each independently selected from O, S, and N; wherein one carbon atom is optionally substituted with one $C_{1-4}$alkyl;

$Het^d$ represents a N-linked 5-membered monocyclic aromatic heterocyclyl containing one, two or three N-atoms; wherein one carbon atom is optionally substituted with one $C_{1-4}$alkyl;

$Het^1$ represents a 4- to 7-membered monocyclic fully saturated heterocyclyl containing one or two heteroatoms each independently selected from O, S, and N; wherein said S-atom might be substituted to form S(=O) or $S(=O)_2$;

n is 1 or 2;

m is 0, 1 or 2;

Y represents O or $CH_2$;

$X^1$ represents CH;

$X^2$ represents CH;

$X^3$ represents CH;

and the pharmaceutically acceptable salts and the solvates thereof.

The present invention also relates to a pharmaceutical composition comprising a therapeutically effective amount of a compound of Formula (I), a pharmaceutically acceptable salt, or a solvate thereof, and a pharmaceutically acceptable carrier or excipient.

Additionally, the invention relates to a compound of Formula (I), a pharmaceutically acceptable salt, or a solvate thereof, for use as a medicament, and to a compound of Formula (I), a pharmaceutically acceptable salt, or a solvate thereof, for use in the treatment or in the prevention of cancer.

In a particular embodiment, the invention relates to a compound of Formula (I), a pharmaceutically acceptable salt, or a solvate thereof, for use in the treatment or in the prevention of cancer.

The invention also relates to the use of a compound of Formula (I), a pharmaceutically acceptable salt, or a solvate thereof, in combination with an additional pharmaceutical agent for use in the treatment or prevention of cancer.

Furthermore, the invention relates to a process for preparing a pharmaceutical composition according to the invention, characterized in that a pharmaceutically acceptable carrier is intimately mixed with a therapeutically effective amount of a compound of Formula (I), a pharmaceutically acceptable salt, or a solvate thereof.

The invention also relates to a product comprising a compound of Formula (I), a pharmaceutically acceptable salt, or a solvate thereof, and an additional pharmaceutical agent, as a combined preparation for simultaneous, separate or sequential use in the treatment or prevention of cancer.

Additionally, the invention relates to a compound of Formula (I) for use in a method of treating or preventing a cell proliferative disease in a subject which comprises administering to the said subject an effective amount of a compound of Formula (I), a pharmaceutically acceptable salt, or a solvate thereof, as defined herein, or a pharmaceutical composition or combination as defined herein.

DETAILED DESCRIPTION OF THE INVENTION

The term ‘halo’ or ‘halogen’ as used herein represents fluoro, chloro, bromo and iodo.

The prefix ’$C_{x-y}$’ (where x and y are integers) as used herein refers to the number of carbon atoms in a given group. Thus, a $C_{1-6}$alkyl group contains from 1 to 6 carbon atoms, and so on.

The term ’$C_{1-4}$alkyl’ as used herein as a group or part of a group represents a straight or branched chain fully saturated hydrocarbon radical having from 1 to 4 carbon atoms, such as methyl, ethyl, *n*-propyl, isopropyl, *n*-butyl, *s*-butyl, *t*-butyl and the like.

The term ’$C_{2-4}$alkyl’ as used herein as a group or part of a group represents a straight or branched chain fully saturated hydrocarbon radical having from 2 to 4 carbon atoms, for example ethyl, *n*-propyl, isopropyl, *n*-butyl, *s*-butyl, and *t*-butyl.

The term ’$C_{3-6}$cycloalkyl’ as used herein as a group or part of a group defines a saturated, cyclic hydrocarbon radical having from 3 to 6 carbon atoms, for example cyclopropyl, cyclobutyl, cyclopentyl and cyclohexyl.

Non-limiting examples of two R groups taken together to from together with the N-atom to which they are attached a monocyclic 4- to 7-membered fully saturated heterocyclyl containing at least one N-atom and optionally one additional heteroatom selected from O, S, and N, include, but are not limited to azetidinyl, pyrrolidinyl, morpholinyl and piperidinyl. A skilled person will understand these examples are N-linked.

Fused bicyclic groups are two cycles that share two atoms and the bond between these atoms.

Non-limiting examples of two R groups taken together to from together with the N-atom to which they are attached a fused bicyclic 6- to 11-membered fully saturated heterocyclyl containing at least one N-atom and optionally one or two additional heteroatoms each independently selected from O, S, and N, include, but are not limited to

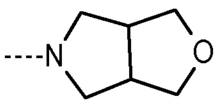
,
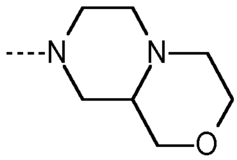
, and
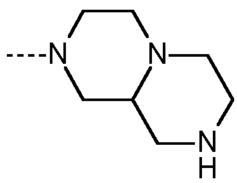
.

Non-limiting examples of ’C-linked 5-or 6-membered monocyclic aromatic heterocyclyl containing one, two or three heteroatoms each independently selected from O, S, and N’, include, but are not limited to C-linked pyrazolyl, C-linked imidazolyl, C-linked pyridinyl, C-linked triazolyl, C-linked pyridazinyl, C-linked pyrimidinyl, C-linked oxazolyl, C-linked furanyl, C-linked isothiazolyl, C-linked 1,2,4-oxadiazolyl, and C-linked pyrazinyl.

Examples of ‘N-linked 5-membered monocyclic aromatic heterocyclyl containing one, two or three N-atoms’, include, N-linked pyrazolyl, N-linked imidazolyl, and N-linked triazolyl.

The term ‘C-linked 4- to 7-membered monocyclic fully saturated heterocyclyl containing one or two heteroatoms each independently selected from O, S, and N’, defines a fully saturated, cyclic hydrocarbon radical having from 4 to 7 ring members and containing one or two heteroatoms each independently selected from O, S, and N, attached to the remainder of the molecule of Formula (I) through any available ring carbon atom (C-linked), for example C-linked azetidinyl, C-linked pyrrolidinyl, C-linked morpholinyl, C-linked tetrahydropyranyl, and C-linked piperidinyl.

The term ‘4- to 7-membered monocyclic fully saturated heterocyclyl containing one or two heteroatoms each independently selected from O, S, and N’, defines a fully saturated, cyclic hydrocarbon radical having from 4 to 7 ring members and containing one or two heteroatoms each independently selected from O, S, and N, for example azetidinyl, pyrrolidinyl, morpholinyl and piperidinyl.

Unless otherwise specified or clear from the context, heterocyclyl goups, can be attached to the remainder of the molecule of Formula (I) through any available ring carbon atom (C-linked) or nitrogen atom (N-linked).

In general, whenever the term ’substituted’ is used in the present invention, it is meant, unless otherwise indicated or clear from the context, to indicate that one or more hydrogens, in particular from 1 to 4 hydrogens, more in particular from 1 to 3 hydrogens, preferably 1 or 2 hydrogens, more preferably 1 hydrogen, on the atom or radical indicated in the expression using ’substituted’ are replaced with a selection from the indicated group, provided that the normal valency is not exceeded, and that the substitution results in a chemically stable compound, i.e. a compound that is sufficiently robust to survive isolation to a useful degree of purity from a reaction mixture.

Combinations of substituents and/or variables are permissible only if such combinations result in chemically stable compounds. ‘Stable compound’ is meant to indicate a compound that is sufficiently robust to survive isolation to a useful degree of purity from a reaction mixture.

The skilled person will understand that the term ’optionally substituted’ means that the atom or radical indicated in the expression using ’optionally substituted’ may or may not be substituted (this means substituted or unsubstituted

respectively).

When two or more substituents are present on a moiety they may, where possible and unless otherwise indicated or clear from the context, replace hydrogens on the same atom or they may replace hydrogen atoms on different atoms in the moiety.

When any variable occurs more than one time in any constituent, each definition is independent.

The term "subject" as used herein, refers to an animal, preferably a mammal (e.g. cat, dog, primate or human), more preferably a human, who is or has been the object of treatment, observation or experiment.

The term "therapeutically effective amount" as used herein, means that amount of active compound or pharmaceutical agent that elicits the biological or medicinal response in a tissue system, or subject (e.g., human) that is being sought by a researcher, veterinarian, medicinal doctor or other clinician, which includes alleviation or reversal of the symptoms of the disease or disorder being treated.

The term "composition" is intended to encompass a product comprising the specified ingredients in the specified amounts, as well as any product which results, directly or indirectly, from combinations of the specified ingredients in the specified amounts. The term "treatment", as used herein, is intended to refer to all processes wherein there may be a slowing, interrupting, arresting or stopping of the progression of a disease, but does not necessarily indicate a total elimination of all symptoms.

The term "compound(s) of the (present) invention" or "compound(s) according to the (present) invention" as used herein, is meant to include the compounds of Formula (I) and the pharmaceutically acceptable salts, and the solvates thereof.

As used herein, any chemical formula with bonds shown only as solid lines and not as solid wedged or hashed wedged bonds, or otherwise indicated as having a particular configuration (e.g. *R*, *S*) around one or more atoms, contemplates each possible stereoisomer, or mixture of two or more stereoisomers. Where the stereochemistry of any particular chiral atom is not specified in the structures shown herein, then all stereoisomers are contemplated and included as the compounds of the invention, either as a pure stereoisomer or as a mixture of two or more stereoisomers.

Hereinbefore and hereinafter, the term "compound of Formula (I)" is meant to include the stereoisomers thereof and the tautomeric forms thereof. However where stereochemistry, as mentioned in the previous paragraph, is specified by bonds which are shown as solid wedged or hashed wedged bonds, or are otherwise indicated as having a particular configuration (e.g. *R*, *S*), or when the stereochemistry around a double bond is shown (e.g. in Formula (I)), then that stereoisomer is so specified and defined. It will be clear this also applies to subgroups of Formula (I).

It follows that a single compound may, where possible, exist in both stereoisomeric and tautomeric form.

The terms "stereoisomers", "stereoisomeric forms" or "stereochemically isomeric forms" hereinbefore or hereinafter are used interchangeably.

The invention includes all stereoisomers of the compounds of the invention either as a pure stereoisomer or as a mixture of two or more stereoisomers.

Enantiomers are stereoisomers that are non-superimposable mirror images of each other. A 1:1 mixture of a pair of enantiomers is a racemate or racemic mixture.

Diastereomers (or diastereoisomers) are stereoisomers that are not enantiomers, i.e. they are not related as mirror images. If a compound contains a double bond, the substituents may be in the *E* or the *Z* configuration.

Substituents on bivalent cyclic saturated or partially saturated radicals may have either the cis- or trans-configuration; for example if a compound contains a disubstituted cycloalkyl group, the substituents may be in the cis or trans configuration.

Therefore, the invention includes enantiomers, atropisomers, diastereomers, racemates, *E* isomers, *Z* isomers, cis isomers, trans isomers and mixtures thereof, unless the context indicates otherwise and whenever chemically possible.

The meaning of all those terms, i.e. enantiomers, atropisomers, diastereomers, racemates, *E* isomers, *Z* isomers, cis isomers, trans isomers and mixtures thereof are known to the skilled person.

The absolute configuration is specified according to the Cahn-Ingold-Prelog system. The configuration at an asymmetric atom is specified by either *R* or *S*. Resolved stereoisomers whose absolute configuration is not known can be designated by (+) or (-) depending on the direction in which they rotate plane polarized light. For instance, resolved enantiomers whose absolute configuration is not known can be designated by (+) or (-) depending on the direction in which they rotate plane polarized light.

When a specific stereoisomer is identified, this means that said stereoisomer is substantially free, i.e. associated with less than 50 %, preferably less than 20 %, more preferably less than 10 %, even more preferably less than 5 %, in particular less than 2 % and most preferably less than 1 %, of the other stereoisomers. Thus, when a compound of Formula (I) is for instance specified as (*R*), this means that the compound is substantially free of the (S) isomer; when a compound of Formula (I) is for instance specified as *E*, this means that the compound is substantially free of the *Z* isomer; when a compound of Formula (I) is for instance specified as cis, this means that the compound is substantially free of the trans isomer.

Pharmaceutically acceptable salts, in particular pharmaceutically acceptable additions salts, include acid addition salts and base addition salts. Such salts may be formed by conventional means, for example by reaction of a free acid or a free base form with one or more equivalents of an appropriate base or acid, optionally in a solvent, or in a medium in which the salt is insoluble, followed by removal of said solvent, or said medium, using standard techniques (e.g. *in vacuo*, by freeze-drying or by filtration). Salts may also be prepared by exchanging a counter-ion of a compound of the invention in the form of a salt with another counter-ion, for example using a suitable ion exchange resin.

The pharmaceutically acceptable salts as mentioned hereinabove or hereinafter are meant to comprise the therapeutically active non-toxic acid and base salt forms which the compounds of Formula (I), and solvates thereof, are able to form.

Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids. Conversely said salt forms can be converted by treatment with an appropriate base into the free base form.

The compounds of Formula (I) and solvates thereof containing an acidic proton may also be converted into their non-toxic metal or amine salt forms by treatment with appropriate organic and inorganic bases.

Appropriate base salt forms comprise, for example, the ammonium salts, the alkali and earth alkaline metal salts, e.g. the lithium, sodium, potassium, cesium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline; the benzathine, N-methyl-D-glucamine, hydrabamine salts, and salts with amino acids such as, for example, arginine, lysine and the like. Conversely the salt form can be converted by treatment with acid into the free acid form.

The term solvate comprises the solvent addition forms as well as the salts thereof, which the compounds of Formula (I) are able to form. Examples of such solvent addition forms are e.g. hydrates, alcoholates and the like.

The compounds of the invention as prepared in the processes described below may be synthesized in the form of mixtures of enantiomers, in particular racemic mixtures of enantiomers, that can be separated from one another following art-known resolution procedures. A manner of separating the enantiomeric forms of the compounds of Formula (I), and pharmaceutically acceptable salts, N-oxides and solvates thereof, involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

The term "enantiomerically pure" as used herein means that the product contains at least 80 % by weight of one enantiomer and 20 % by weight or less of the other enantiomer. Preferably the product contains at least 90 % by weight of one enantiomer and 10 % by weight or less of the other enantiomer. In the most preferred embodiment the term "enantiomerically pure" means that the composition contains at least 99 % by weight of one enantiomer and 1 % or less of the other enantiomer.

The present invention also embraces isotopically-labeled compounds of the present invention which are identical to those recited herein, but for the fact that one or more atoms are replaced by an atom having an atomic mass or mass number different from the atomic mass or mass number usually found in nature (or the most abundant one found in nature).

All isotopes and isotopic mixtures of any particular atom or element as specified herein are contemplated within the scope of the compounds of the invention, either naturally occurring or synthetically produced, either with natural abundance or in an isotopically enriched form. Exemplary isotopes that can be incorporated into compounds of the invention include isotopes of hydrogen, carbon, nitrogen, oxygen, phosphorus, sulfur, fluorine, chlorine and iodine, such as $^{2}H$, $^{3}H$, $^{11}C$, $^{13}C$, $^{14}C$, $^{13}N$, $^{15}O$, $^{17}O$, $^{18}O$, $^{32}P$, $^{33}P$, $^{35}S$, $^{18}F$, $^{36}Cl$, $^{122}I$, $^{123}I$, $^{125}I$, $^{131}I$, $^{75}Br$, $^{76}Br$, $^{77}Br$ and $^{82}Br$. Preferably, the isotope is selected from the group of $^{2}H$, $^{3}H$, $^{11}C$ and $^{18}F$. More preferably, the isotope is $^{2}H$. In particular, deuterated compounds are intended to be included within the scope of the present invention.

Certain isotopically-labeled compounds of the present invention (e.g., those labeled with $^{3}H$ and $^{14}C$) may be useful for example in substrate tissue distribution assays. Tritiated ($^{3}H$) and carbon-14 ($^{14}C$) isotopes are useful for their ease of preparation and detectability. Further, substitution with heavier isotopes such as deuterium (i.e., $^{2}H$) may afford certain therapeutic advantages resulting from greater metabolic stability (e.g., increased *in vivo* half-life or reduced dosage requirements) and hence may be preferred in some circumstances. Positron emitting isotopes such as $^{15}O$, $^{13}N$, $^{11}C$ and $^{18}F$ are useful for positron emission tomography (PET) studies. PET imaging in cancer finds utility in helping locate and identify tumours, stage the disease and determine suitable treatment. Human cancer cells overexpress many receptors or proteins that are potential disease-specific molecular targets. Radiolabelled tracers that bind with high affinity and specificity to such receptors or proteins on tumour cells have great potential for diagnostic imaging and

targeted radionuclide therapy (Charron, Carlie L. et al. Tetrahedron Lett. 2016, 57(37), 4119-4127). Additionally, target-specific PET radiotracers may be used as biomarkers to examine and evaluate pathology, by for example, measuring target expression and treatment response (Austin R. et al. Cancer Letters (2016), doi: 10.1016/j.canlet.2016.05.008).

In an embodiment, the present invention concerns novel compounds of Formula (I), wherein

$R^1$ represents hydrogen; $-CH_2OR^a$; $-CH_2F$; or $-CH_2NR^bR^c$;

$R^{1a}$ represents hydrogen or $-CH_2OR^a$;

$R^a$ is selected from the group consisting of hydrogen; $C_{1-4}$alkyl; $Het^a$; $C_{1-4}$alkyl substituted with one substitutent selected from the group consisting of $-C(=O)-NR^dR^e$, $-C(=O)-OR^f$, $C_{3-6}$cycloalkyl, $Het^b$, and $Het^c$; and $C_{2-4}$alkyl substituted with one substitutent selected from the group consisting of $-(OCH_2CH_2)_m-OCH_3$, and $-NR^dR^e$;

$R^b$ and $R^c$ are taken together to form together with the N-atom to which they are attached a monocyclic 4- to 7-membered fully saturated heterocyclyl containing at least one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing at least one N-atom and optionally one or two additional heteroatoms each independently selected from O, S, and N, wherein said S-atom might be substituted to form S(=O) or $S(=O)_2$; wherein said monocyclic or fused bicyclic heterocyclyl is optionally substituted on one nitrogen with one $C_{1-4}$alkyl;

$R^d$ and $R^e$ are taken together to form together with the N-atom to which they are attached a monocyclic 4- to 7-membered fully saturated heterocyclyl containing at least one N-atom and optionally one additional heteroatom selected from O, S, and N, wherein said S-atom might be substituted to form S(=O) or $S(=O)_2$; wherein said monocyclic heterocyclyl is optionally substituted on one carbon atom with one or two halo substituents;

$R^f$ represents hydrogen;

$R^2$ represents hydrogen or fluoro;

$Het^a$ represents a C-linked 5- or 6-membered monocyclic aromatic heterocyclyl containing one, two or three heteroatoms each independently selected from O, S, and N;

$Het^b$ represents a C-linked 4- to 7-membered monocyclic fully saturated heterocyclyl containing one or two heteroatoms each independently selected from O, S, and N; wherein said S-atom might be substituted to form S(=O) or $S(=O)_2$;

$Het^c$ represents a C-linked 5- or 6-membered monocyclic aromatic heterocyclyl containing one, two or three heteroatoms each independently selected from O, S, and N; wherein one carbon atom is optionally substituted with one $C_{1-4}$alkyl;

n is 1 or 2;

m is 0 or 1;

Y represents O or $CH_2$;

$X^1$ represents CH;

$X^2$ represents CH;

$X^3$ represents CH;

and the pharmaceutically acceptable salts and the solvates thereof.

In an embodiment, the present invention concerns novel compounds of Formula (I), wherein

$R^1$ represents hydrogen; $-CH_2OR^a$; $-CH_2F$; or $-CH_2NR^bR^c$;

$R^{1a}$ represents hydrogen;

$R^a$ is selected from the group consisting of hydrogen; $C_{1-4}$alkyl; $Het^a$;

$C_{1-4}$alkyl substituted with one substitutent selected from the group consisting of -C(=O)-$NR^dR^e$, -C(=O)-$OR^f$, $C_{3-6}$cycloalkyl, $Het^b$, and $Het^c$; and

$C_{2-4}$alkyl substituted with one substitutent selected from the group consisting of -$(OCH_2CH_2)_m$-$OCH_3$, and -$NR^dR^e$;

$R^b$ and $R^c$ are each independently selected from the group consisting of hydrogen, $C_{1-4}$alkyl, and $C_{2-4}$alkyl substituted with one $Het^1$;

or $R^b$ and $R^c$ are taken together to form together with the N-atom to which they are attached a monocyclic 4- to 7-membered fully saturated heterocyclyl containing at least one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing at least one N-atom and optionally one or two additional heteroatoms each independently selected from O, S, and N, wherein said S-atom might be substituted to form S(=O) or $S(=O)_2$;

wherein said monocyclic or fused bicyclic heterocyclyl is optionally substituted on one nitrogen with one $C_{1-4}$alkyl;

wherein said monocyclic or fused bicyclic heterocyclyl is optionally substituted on one carbon atom with one or two substituents each independently selected from the group consisting of $C_{1-4}$alkyl, oxo and halo;

$R^d$ and $R^e$ are each independently selected from the group consisting of hydrogen, $C_{1-4}$alkyl, and $C_{2-4}$alkyl substituted with one $Het^1$;

or $R^d$ and $R^e$ are taken together to form together with the N-atom to which they are attached a monocyclic 4- to 7-membered fully saturated heterocyclyl containing at least one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing at least one N-atom and optionally one or two additional heteroatoms each independently selected from O, S, and N, wherein said S-atom might be substituted to form S(=O) or $S(=O)_2$;

wherein said monocyclic or fused bicyclic heterocyclyl is optionally substituted on one nitrogen with one $C_{1-4}$alkyl;

wherein said monocyclic or fused bicyclic heterocyclyl is optionally substituted on one carbon atom with one or two substituents each independently selected from the group consisting of $C_{1-4}$alkyl, oxo and halo;

$R^f$ represents hydrogen or $C_{1-4}$alkyl;

$R^2$ represents hydrogen or fluoro;

$Het^a$ represents a C-linked 5- or 6-membered monocyclic aromatic heterocyclyl containing one, two or three heteroatoms each independently selected from O, S, and N; wherein one carbon atom is optionally substituted with one $C_{1-4}$alkyl;

$Het^b$ represents a C-linked 4- to 7-membered monocyclic fully saturated heterocyclyl containing one or two heteroatoms each independently selected from O, S, and N; wherein said S-atom might be substituted to form S(=O) or $S(=O)_2$;

$Het^c$ represents a C-linked 5- or 6-membered monocyclic aromatic heterocyclyl containing one, two or three heteroatoms each independently selected from O, S, and N; wherein one carbon atom is optionally substituted with one $C_{1-4}$alkyl;

$Het^1$ represents a 4- to 7-membered monocyclic fully saturated heterocyclyl containing one or two heteroatoms each independently selected from O, S, and N; wherein said S-atom might be substituted to form S(=O) or $S(=O)_2$;

n is 1 or 2;

m is 0, 1 or 2;

Y represents O or $CH_2$;

$X^1$ represents CH;

$X^2$ represents CH;

$X^3$ represents CH;

and the pharmaceutically acceptable salts and the solvates thereof.

In an embodiment, the present invention concerns novel compounds of Formula (I), wherein

$R^1$ represents hydrogen or $-CH_2OR^a$;

$R^{1a}$ represents hydrogen;

$R^a$ is selected from the group consisting of hydrogen; $C_{1-4}$alkyl; $Het^a$;

$C_{1-4}$alkyl substituted with one substitutent selected from the group consisting of $-C(=O)-NR^dR^e$, $-C(=O)-OR^f$, $C_{3-6}$cycloalkyl, $Het^b$, and $Het^c$; and

$C_{2-4}$alkyl substituted with one substitutent selected from the group consisting of $-(OCH_2CH_2)_m-OCH_3$, and $-NR^dR^e$;

$R^d$ and $R^e$ are taken together to form together with the N-atom to which they are attached a monocyclic 4- to 7-membered fully saturated heterocyclyl containing at least one N-atom and optionally one additional heteroatom selected from O, S, and N, wherein said S-atom might be substituted to form S(=O) or $S(=O)_2$;

wherein said monocyclic heterocyclyl is optionally substituted on one carbon atom with one or two halo substituents;

$R^f$ represents hydrogen;

$R^2$ represents hydrogen or fluoro;

$Het^a$ represents a C-linked 5- or 6-membered monocyclic aromatic heterocyclyl containing one, two or three heteroatoms each independently selected from O, S, and N;

$Het^b$ represents a C-linked 4- to 7-membered monocyclic fully saturated heterocyclyl containing one or two heteroatoms each independently selected from O, S, and N; wherein said S-atom might be substituted to form S(=O) or $S(=O)_2$;

$Het^c$ represents a C-linked 5- or 6-membered monocyclic aromatic heterocyclyl containing one, two or three heteroatoms each independently selected from O, S, and N; wherein one carbon atom is optionally substituted with one $C_{1-4}$alkyl;

n is 1 or 2;

m is 0 or 1;

Y represents O or $CH_2$;

$X^1$ represents CH;

$X^2$ represents CH;

$X^3$ represents CH;

and the pharmaceutically acceptable salts and the solvates thereof.

In an embodiment, the present invention concerns novel compounds of Formula (I), wherein

$R^1$ represents hydrogen; $-CH_2OR^a$; or $-CH_2NR^bR^c$;

$R^{1a}$ represents hydrogen;

$R^a$, $R^b$, and $R^c$, are each independently selected from the group consisting of hydrogen and $C_{1-4}$alkyl;

$R^2$ represents hydrogen or fluoro;

n is 1 or 2;

Y represents O or $CH_2$;

$X^1$ represents CH;

$X^2$ represents CH;

$X^3$ represents CH;

and the pharmaceutically acceptable salts and the solvates thereof.

In an embodiment, the present invention concerns novel compounds of Formula (I), wherein

$R^1$ represents hydrogen; $-CH_2OR^a$; or $-CH_2NR^bR^c$;

$R^{1a}$ represents hydrogen;

$R^a$, $R^b$, and $R^c$, are each independently selected from the group consisting of hydrogen and $C_{1-4}$alkyl;

$R^2$ represents hydrogen or fluoro;

n is 1;

Y represents O or $CH_2$;

$X^1$ represents CH;

$X^2$ represents CH;

$X^3$ represents CH;

and the pharmaceutically acceptable salts and the solvates thereof.

In an embodiment, the present invention concerns novel compounds of Formula (I), wherein

$R^1$ represents hydrogen or $-CH_2OR^a$;

$R^{1a}$ represents hydrogen;

$R^a$ represents $C_{1-4}$alkyl;

$R^2$ represents hydrogen or fluoro;

n is 1;

Y represents O or $CH_2$;

$X^1$ represents CH;

$X^2$ represents CH;

$X^3$ represents CH;

and the pharmaceutically acceptable salts and the solvates thereof.

In an embodiment, the present invention concerns novel compounds of Formula (I), wherein

$R^1$ represents hydrogen; $-CH_2OR^a$; or $-CH_2NR^bR^c$;

$R^{1a}$ represents hydrogen;

$R^a$, $R^b$, and $R^c$, are each independently selected from the group consisting of hydrogen and $C_{1-4}$alkyl;

$R^2$ represents hydrogen;

n is 1 or 2;

Y represents O or $CH_2$;

$X^1$ represents CH;

$X^2$ represents CH;

$X^3$ represents CH;

and the pharmaceutically acceptable salts and the solvates thereof.

In an embodiment, the present invention relates to those compounds of Formula (I) and the pharmaceutically acceptable salts, and the solvates thereof, or any subgroup thereof as mentioned in any of the other embodiments, wherein $R^{1a}$ represents hydrogen.

In an embodiment, the present invention relates to those compounds of Formula (I) and the pharmaceutically acceptable salts, and the solvates thereof, or any subgroup thereof as mentioned in any of the other embodiments, wherein Y represents O.

In an embodiment, the present invention relates to those compounds of Formula (I) and the pharmaceutically acceptable salts, and the solvates thereof, or any subgroup thereof as mentioned in any of the other embodiments, wherein Y represents $CH_2$.

In an embodiment, the present invention relates to those compounds of Formula (I) and the pharmaceutically acceptable salts, and the solvates thereof, or any subgroup thereof as mentioned in any of the other embodiments, wherein n is 1.

In an embodiment, the present invention relates to those compounds of Formula (I) and the pharmaceutically acceptable salts, and the solvates thereof, or any subgroup thereof as mentioned in any of the other embodiments, wherein n is 2.

In an embodiment, the present invention relates to those compounds of Formula (I) and the pharmaceutically acceptable salts, and the solvates thereof, or any subgroup thereof as mentioned in any of the other embodiments, wherein m is 0 or 1.

In an embodiment, the present invention relates to those compounds of Formula (I) and the pharmaceutically acceptable salts, and the solvates thereof, or any subgroup thereof as mentioned in any of the other embodiments, wherein $R^2$ represents hydrogen.

In an embodiment, the present invention relates to those compounds of Formula (I) and the pharmaceutically acceptable salts, and the solvates thereof, or any subgroup thereof as mentioned in any of the other embodiments, wherein $R^2$ represents fluoro.

In an embodiment, the present invention relates to those compounds of Formula (I) and the pharmaceutically acceptable salts, and the solvates thereof, or any subgroup thereof as mentioned in any of the other embodiments, wherein $R^1$ represents hydrogen.

In an embodiment, the present invention relates to those compounds of Formula (I) and the pharmaceutically acceptable salts, and the solvates thereof, or any subgroup thereof as mentioned in any of the other embodiments, wherein $R^1$ represents $-CH_2OR^a$, and wherein $R^a$ represents $C_{1-4}$alkyl.

In an embodiment, the present invention relates to those compounds of Formula (I) and the pharmaceutically acceptable salts, and the solvates thereof, or any subgroup thereof as mentioned in any of the other embodiments, wherein $R^1$ represents $-CH_2OR^a$.

In an embodiment, the present invention relates to those compounds of Formula (I) and the pharmaceutically acceptable salts, and the solvates thereof, or any subgroup thereof as mentioned in any of the other embodiments, wherein $R^1$ represents $-CH_2NR^bR^c$.

In an embodiment, the present invention relates to those compounds of Formula (I) and the pharmaceutically acceptable salts, and the solvates thereof, or any subgroup thereof as mentioned in any of the other embodiments, wherein $R^a$, $R^b$, and $R^c$, represent hydrogen.

In an embodiment, the present invention relates to those compounds of Formula (I) and the pharmaceutically acceptable salts, and the solvates thereof, or any subgroup thereof as mentioned in any of the other embodiments, wherein $R^a$, $R^b$, and $R^c$, represent $C_{1-4}$alkyl.

In an embodiment, the present invention relates to those compounds of Formula (I) and the pharmaceutically acceptable salts, and the solvates thereof, or any subgroup thereof as mentioned in any of the other embodiments, wherein $R^a$ represents $C_{1-4}$alkyl, in particular methyl.

In an embodiment, the present invention relates to those compounds of Formula (I) and the pharmaceutically acceptable salts, and the solvates thereof, or any subgroup thereof as mentioned in any of the other embodiments, wherein $R^a$ represents hydrogen.

In an embodiment, the present invention relates to those compounds of Formula (I) and the pharmaceutically acceptable salts, and the solvates thereof, or any subgroup thereof as mentioned in any of the other embodiments, wherein $R^a$ is selected from the group consisting of hydrogen; $C_{1-4}$alkyl; $Het^a$;

$C_{1-4}$alkyl substituted with one substitutent selected from the group consisting of $-C(=O)-NR^dR^e$, $-C(=O)-OR^f$, $C_{3-6}$cycloalkyl, $Het^b$, and $Het^c$; and
$C_{2-4}$alkyl substituted with one substitutent selected from the group consisting of $-(OCH_2CH_2)_m-OCH_3$, and $-NR^dR^e$.

In an embodiment, the present invention relates to those compounds of Formula (I) and the pharmaceutically acceptable salts, and the solvates thereof, or any subgroup thereof as mentioned in any of the other embodiments, wherein $R^{1a}$ represents $-CH_2OR^a$; $-CH_2F$; or $-CH_2NR^bR^c$.

In an embodiment, the present invention relates to those compounds of Formula (I) and the pharmaceutically acceptable salts, and the solvates thereof, or any subgroup thereof as mentioned in any of the other embodiments, wherein $R^{1a}$ represents hydrogen.

In an embodiment, the present invention relates to those compounds of Formula (I) and the pharmaceutically accptable salts, and the solvates thereof, or any subgroup thereof as mentioned in any of the other embodiments, wherein

$R^1$ represents $-CH_2OR^a$; $-CH_2F$; or $-CH_2NR^bR^c$;

$R^{1a}$ represents $-CH_2OR^a$; $-CH_2F$; or $-CH_2NR^bR^c$.

In an embodiment, the present invention relates to those compounds of Formula (I) and the pharmaceutically accptable salts, and the solvates thereof, or any subgroup thereof as mentioned in any of the other embodiments, wherein at least one of $R^1$ and $R^{1a}$ is other than hydrogen.In an embodiment, the present invention relates to those compounds of Formula (I) and the pharmaceutically accptable salts, and the solvates thereof, or any subgroup thereof as mentioned in any of the other embodiments, wherein

a heterocyclyl containing at least one N-atom and optionally one additional heteroatom selected from O, S, and N; is a heterocyclyl containing
1 or 2 N-atoms,
1 N-atom and 1 O-atom, or
1 N-atom and 1 S-atom.

In an embodiment, the present invention relates to those compounds of Formula (I) and the pharmaceutically

accptable salts, and the solvates thereof, or any subgroup thereof as mentioned in any of the other embodiments, wherein

a heterocyclyl containing at least one N-atom and optionally one or two additional heteroatoms each independently selected from O, S, and N;
is a heterocyclyl containing
1, 2 or 3 N-atoms,
1 N-atom and 1 or 2 O-atoms,
1 N-atom and 1 or 2 S-atoms,
1 N-atom, 1 O-atom, and 1 S-atom,
2 N-atoms and 1 O-atom, or
2 N-atoms and 1 S-atom.

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-a):

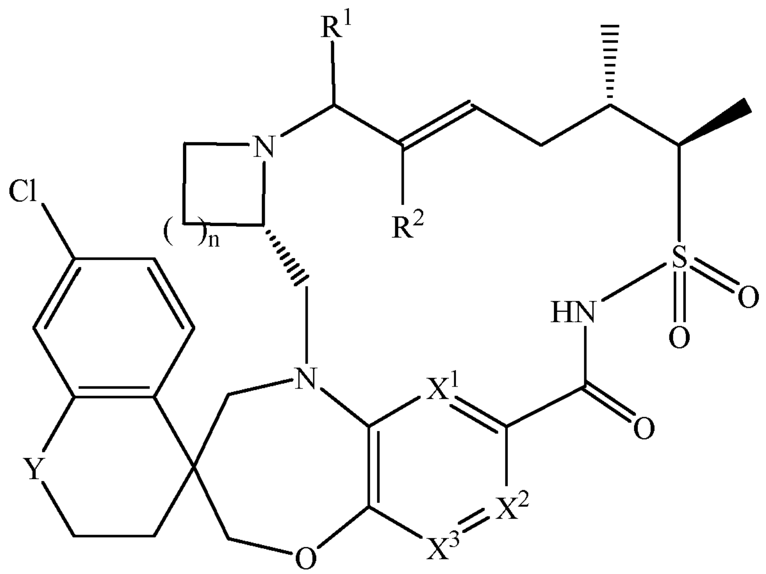

(I-a)

It will be clear that all variables in the structure of Formula (I-a), are defined as defined for the compounds of Formula (I) or any subgroup thereof as mentioned in any of the other embodiments.

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-x):

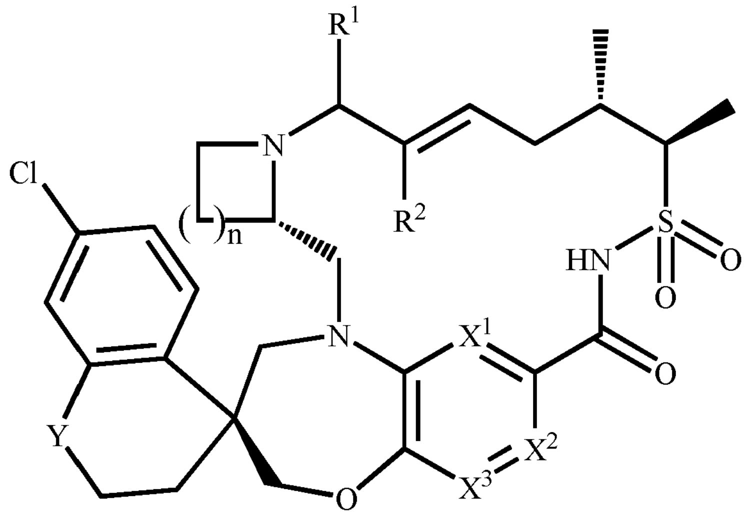

(I-x)

It will be clear that all variables in the structure of Formula (I-x), are defined as defined for the compounds of Formula (I) or any subgroup thereof as mentioned in any of the other embodiments.

In an embodiment, the present invention relates to those compounds of Formula (I) and the pharmaceutically acceptable salts, and the solvates thereof, or any subgroup thereof as mentioned in any of the other embodiments, wherein the compounds of Formula (I) are restricted to compounds of Formula (I-y):

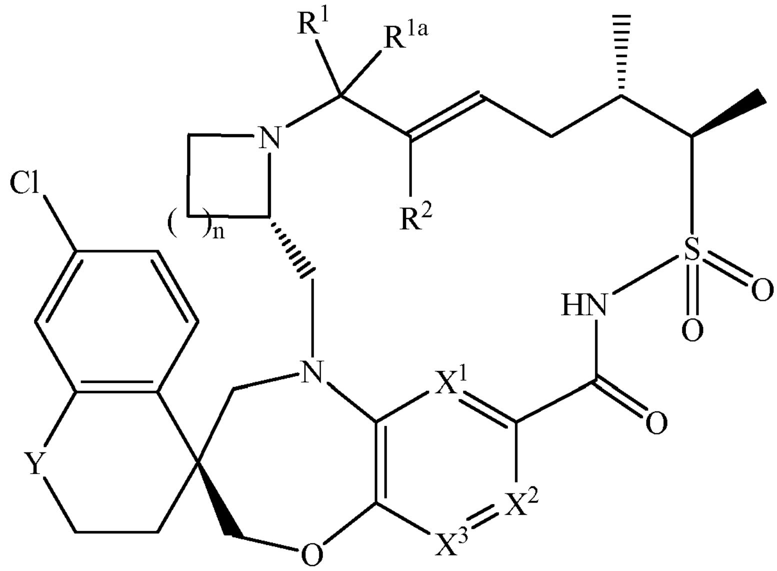

(I-y)

It will be clear that all variables in the structure of Formula (I-y), are defined as defined for the compounds of Formula (I) or any subgroup thereof as mentioned in any of the other embodiments.

In a particular embodiment, when $R^b$ and $R^c$, or $R^d$ and $R^e$, are taken together to form together with the N-atom to which they are attached a monocyclic 4- to 7-membered fully saturated heterocyclyl or a fused bicyclic 6- to 11-membered fully saturated heterocyclyl, said monocyclic or fused bicyclic heterocyclyl is substituted on a carbon atom in the 2-position of the N-atom with oxo to form together with the N-atom an amide group; and is optionally further substituted as defined in any of the other embodiments.

In an embodiment, the present invention relates to a subgroup of Formula (I) as defined in the general reaction schemes.

In an embodiment the compound of Formula (I) is selected from the group consisting of any of the exemplified compounds,
and the free bases, the pharmaceutically acceptable salts, and the solvates thereof.

All possible combinations of the above indicated embodiments are considered to be embraced within the scope of the invention.

METHODS FOR THE PREPARATION OF COMPOUNDS

In this section, as in all other sections unless the context indicates otherwise, references to Formula (I) also include all other sub-groups and examples thereof as defined herein.

The general preparation of some typical examples of the compounds of Formula (I) is described hereunder and in the specific examples, and are generally prepared from starting materials which are either commercially available or can be prepared by published methods. The following schemes are only meant to represent examples of the invention and are in no way meant to be a limit of the invention.

Alternatively, compounds of the present invention may also be prepared by analogous reaction protocols as described in the general schemes below, combined with standard synthetic processes commonly used by those skilled in the art including also analogous reaction protocols as described in WO2016033486, WO2017147410 and WO2018183418.

The skilled person will realize that in the reactions described in the Schemes, although this is not always explicitly shown, it may be necessary to protect reactive functional groups (for example hydroxy, amino, or carboxy groups) where these are desired in the final product, to avoid their unwanted participation in the reactions. In general, conventional protecting groups can be used in accordance with standard practice. The protecting groups may be removed at a convenient subsequent stage using methods known from the art.

The skilled person will realize that in the reactions described in the Schemes, it may be advisable or necessary to perform the reaction under an inert atmosphere, such as for example under $N_2$-gas atmosphere.

It will be apparent for the skilled person that it may be necessary to cool the reaction mixture before reaction work-up (refers to the series of manipulations required to isolate and purify the product(s) of a chemical reaction such as for example quenching, column chromatography, extraction).

The skilled person will realize that heating the reaction mixture under stirring may enhance the reaction outcome. In some reactions microwave heating may be used instead of conventional heating to shorten the overall reaction time.

The skilled person will realize that another sequence of the chemical reactions shown in the Schemes below, may also result in the desired compound of Formula (I).

The skilled person will realize that intermediates and final compounds shown in the Schemes below may be further functionalized according to methods well-known by the person skilled in the art. The intermediates and compounds described herein can be isolated in free form or as a salt, or a solvate thereof. The intermediates and compounds described herein may be synthesized in the form of mixtures of tautomers and stereoisomeric forms that can be separated from one another following art-known resolution procedures.

The meaning of the chemical abbreviations used in the schemes below are as defined in Table 1.

Compounds of Formula (I), wherein $R^{1a}$ is defined as H (hydrogen); hereby referred to as compounds of Formula (I-a), can be prepared according to Scheme 1,

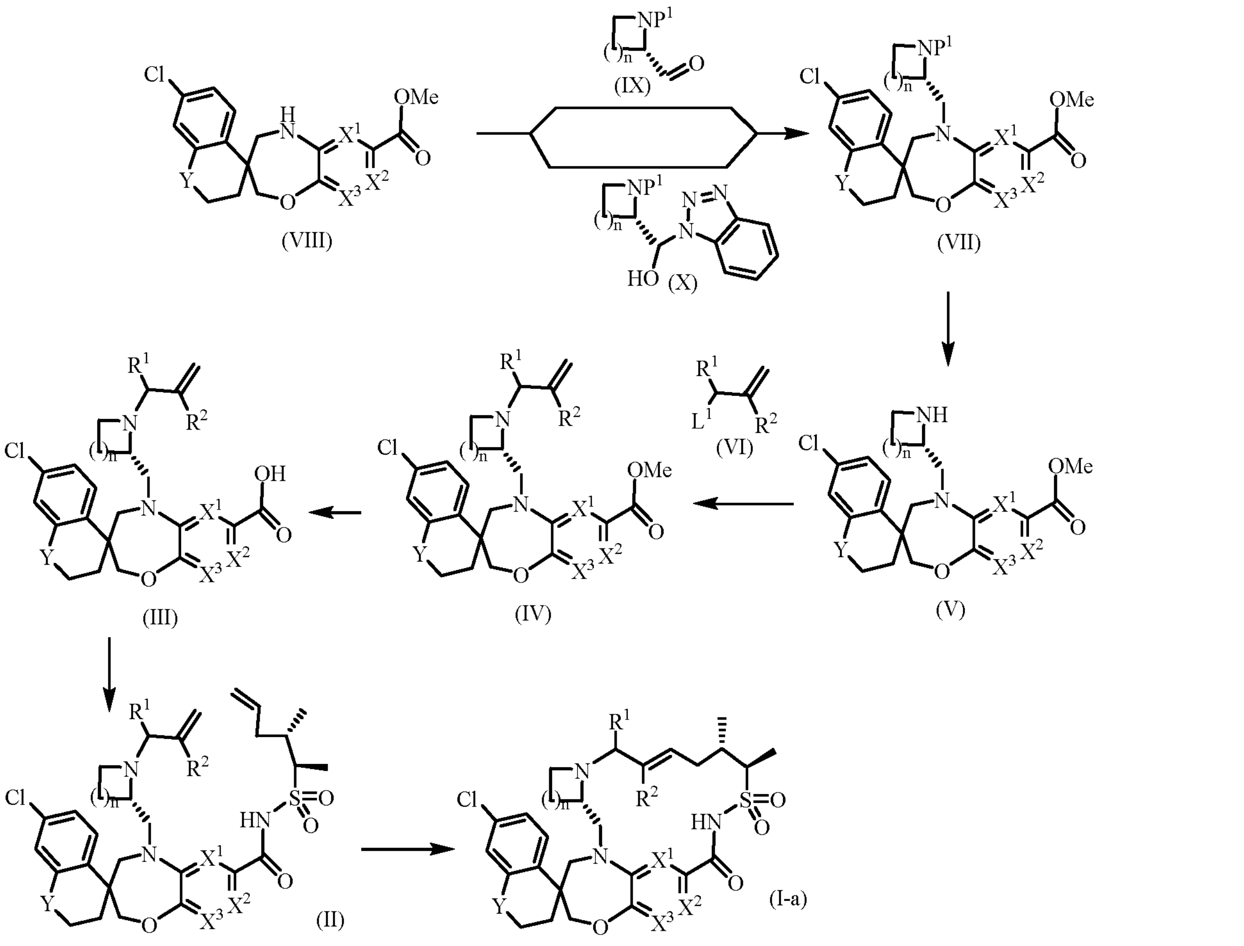

(VIII) (IX) (X) (VII) (VI) (V) (IV) (III) (II) (I-a)

Scheme 1

wherein $X^1$, $X^2$, $X^3$, Y, n, $R^1$ and $R^2$ are as defined in Formula (I),

- by reacting an intermediate of Formula (II), in the presence of a suitable ring closing metathesis catalyst such as, for example, Grubbs-Hoveyda 2nd generation catalyst, in a suitable solvent such as dichloroethane, at a suitable temperature such as room temperature or 60 °C.
- Intermediates of Formula (II) can be prepared by reacting an intermediate of Formula (III), wherein $X^1$, $X^2$, $X^3$, Y, n, $R^1$ and $R^2$ are as defined in Formula (I), with (2R,3S)-3-methylhex-5-ene-2-sulfonamide, in a suitable solvent such as, for example, DCM, in the presence of a suitable base such as, for example, DMAP, and in the presence of a suitable coupling agent such as, for example, EDC.HCl, at a suitable temperature such as, for example, room temperature.
- Intermediates of Formula (III) can be prepared by reacting an intermediate of Formula (IV), wherein $X^1$, $X^2$, $X^3$, Y, n, $R^1$ and $R^2$ are as defined in Formula (I), with a suitable base such as, for example, LiOH or NaOH, in a suitable solvent such as, for example, water, or a suitable mixture of solvents such as, for example, water and MeOH, or a mixture of water, MeOH, and THF, at a suitable temperature such as, for example, room temperature, or 60 °C.
- Intermediates of Formula (IV) can be prepared by reacting an intermediate of Formula (V), wherein $X^1$, $X^2$, $X^3$, Y, and n, are as defined in Formula (I), with an intermediate of Formula (VI), wherein $R^1$ and $R^2$ are as defined in Formula (I), and $L^1$ is a suitable leaving group such as, for example, Br or MsO, in the presence of a suitable base such as, for example, $Cs_2CO_3$, in a suitable solvent such as, for example, DCM, at a suitable temperature such as, for example, room temperature.
- Intermediates of Formula (V) can be prepared by reacting an intermediate of Formula (VII), wherein $X^1$, $X^2$, $X^3$, Y, and n, are as defined in Formula (I), and $P^1$ is a suitable protecting group such as, for example, Boc, with a suitable deprotecting agent such as, for example, TFA or a solution of HCl in dioxane, in a suitable solvent such as, for example, DCM or dioxane, at a suitable temperature such as, for example, room temperature.
- Intermediates of Formula (VII) can be prepared by reacting an intermediate of Formula (VIII), wherein $X^1$, $X^2$, $X^3$, Y, and n, are as defined in Formula (I), with a suitable intermediate of Formula (IX), or a suitable activated form of

this intermediate such as a compound of Formula (X), in the presence of a suitable acid such as, for example, AcOH, and in the presence of a suitable reducing agent such as, for example, $NaBH(OAc)_3$, in a suitable solvent such as, for example, DCM, at a suitable temperature such as, for example, 0 °C or room temperature.

- Intermediates of Formula (X) can be prepared by reacting an intermediate of Formula (IX) with benzotriazole, in a suitable solvent such as, for example, methyltertbutyl ether, at a suitable temperature such as, for example, room temperature.

Alternatively, compounds of Formula (I), wherein $R^{1a}$ is defined as H (hydrogen); hereby named compounds of Formula (I-a), can be prepared according to Scheme 2,

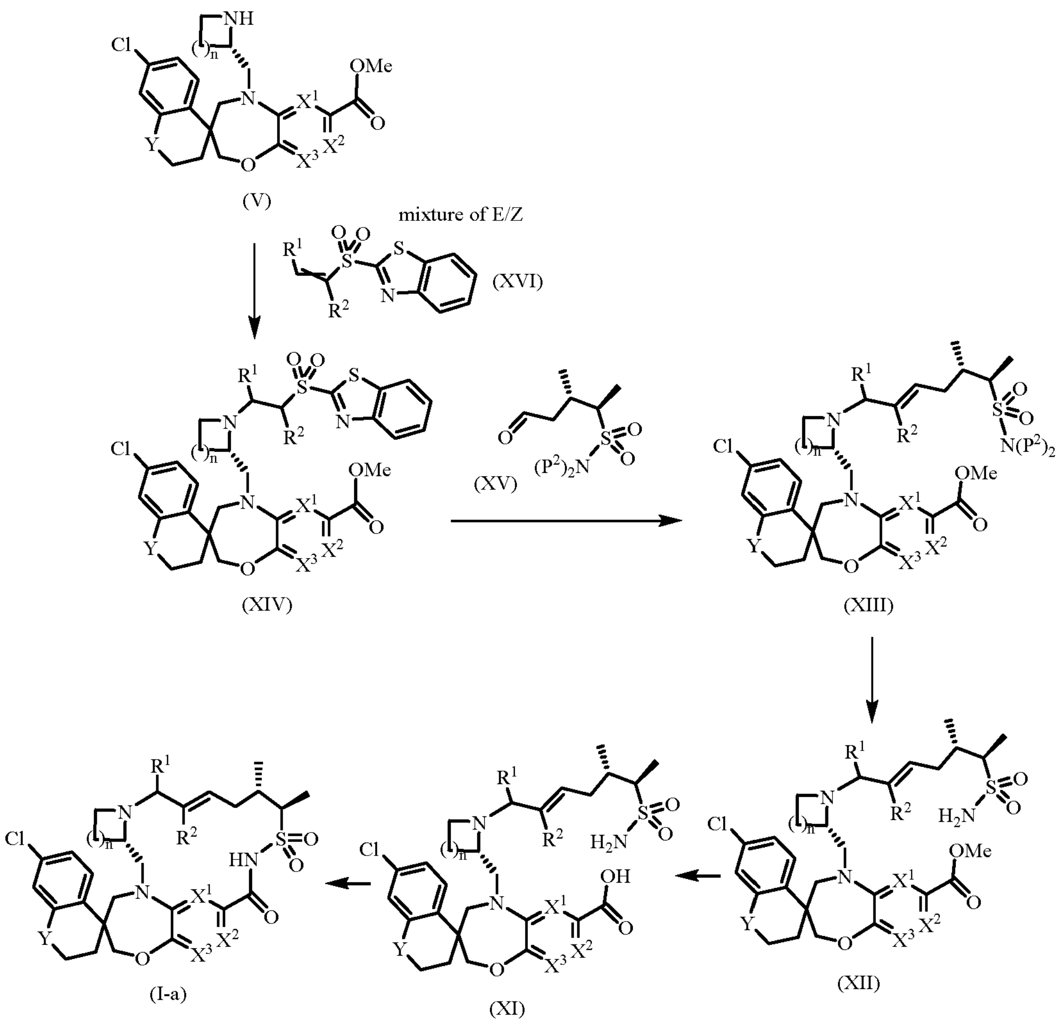


(V) (XVI) (XIV) (XV) (XIII) (XII) (XI) (I-a)

Scheme 2

- by reacting an intermediate of Formula (XI), wherein $X^1$, $X^2$, $X^3$, Y, n, $R^1$ and $R^2$ are as defined in Formula (I), with a suitable coupling agent such as, for example, DECP, in the presence of a suitable base such as, for example, DBU, in a suitable solvent such as, for example, DMF, at a suitable temperature such as room temperature.
- Intermediates of Formula (XI) can be prepared by reacting an intermediate of Formula (XII), wherein $X^1$, $X^2$, $X^3$, Y, n, $R^1$ and $R^2$ are as defined in Formula (I), with a suitable base such as, for example, LiOH or NaOH, in a suitable solvent such as, for example, water, or a suitable mixture of solvents such as, for example, water and THF, or a mixture of water, MeOH, and THF, at a suitable temperature such as, for example, room temperature, or 50 °C.
- Intermediates of Formula (XII) can be prepared by reacting an intermediate of Formula (XIII), wherein $X^1$, $X^2$, $X^3$, Y, n, $R^1$ and $R^2$ are as defined in Formula (I), and $P^2$ is a suitable protecting group such as, for example, p-methoxybenzyl, with a suitable deprotecting agent such as, for example, TFA, in a suitable solvent such as, for example, DCM, at a suitable temperature such as, for example, room temperature.
- Intermediates of Formula (XIII) can be prepared by reacting an intermediate of Formula (XIV), wherein $X^1$, $X^2$, $X^3$,

Y, n, $R^1$ and $R^2$ are as defined in Formula (I), with an intermediate of Formula (XV), wherein $P^2$ is a suitable protecting group such as, for example, p-methoxybenzyl, in the presence of a suitable base such as, for example, tBuOK, in a suitable solvent such as, for example, THF, at a suitable temperature such as, for example, -20 °C or room temperature.

- Intermediates of Formula (XIV) can be prepared by reacting an intermediate of Formula (V) with an intermediate of Formula (XVI), wherein $R^1$ and $R^2$ are as defined in Formula (I), in a suitable solvent such as, for example, DCM, at a suitable temperature such as, for example, room temperature.

Intermediates of Formula (VIII) have been described in literature but can also be prepared according to Scheme 3,

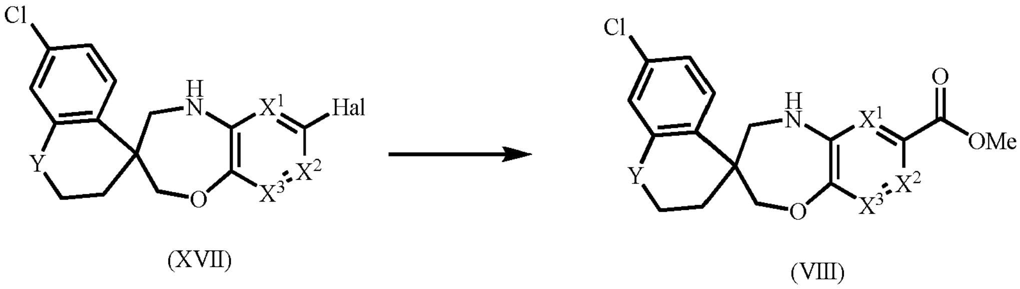

(XVII) (VIII)

Scheme 3

by reacting an intermediate of Formula (XVII), wherein $X^1$, $X^2$, $X^3$ and Y, are as defined in Formula (I), and Hal is defined as an halogen such as, for example, I or Br, in particular I, with MeOH, in the presence of a suitable pressure of CO such as, for example, 50 atm, in the presence of a suitable catalyst such as, for example, [1,1'-bis(diphenylphosphino)ferrocene]dichloropalladium(II) ($PdCl_2$(dppf)), at a suitable temperature such as, for example, 100 °C.

Alternatively, when $R^1$ is defined as $-CH_2NR^bR^c$, and $R^{1a}$ is defined as H (hydrogen), compounds of Formula (I), can be prepared according to Scheme 4,

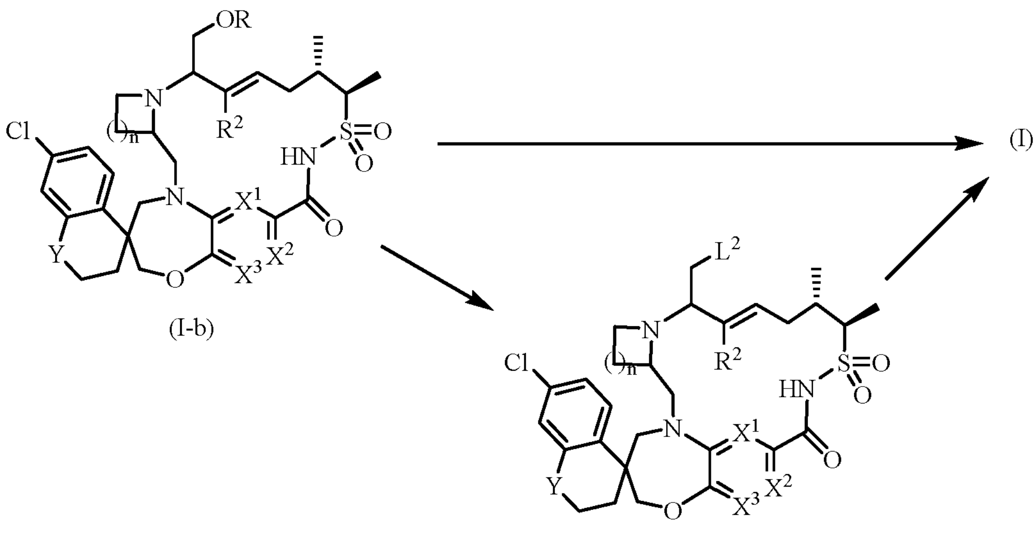

(I-b) (I) (XX)

Scheme 4

- by reacting an intermediate of Formula (XX), wherein $X^1$, $X^2$, $X^3$, Y, $R^2$, and n, are as defined in Formula (I), $R^{1a}$ is defined as H (hydrogen), and $L^2$ is a suitable leaving group such as, for example, mesylate, with an excess of a suitable amine of formula $HNR^bR^c$ such as, for example, 3 equivalents, in a suitable solvent such as, for example, ACN, at a suitable temperature such as, for example, between room temperature and 80 °C.
- alternatively by reacting an intermediate of Formula (I-b), wherein $X^1$, $X^2$, $X^3$, Y, $R^2$, and n, are as defined in Formula

(I), and $R^{1a}$ is defined as H (hydrogen), with a suitable activating agent such as, for example, triflic anhydride, in a suitable solvent such as, for example, DCM, at a suitable temperature such as, for example, between room temperature and -78 °C, and reacting the resulting triflate in situ with an excess of a suitable amine of formula $HNR^bR^c$ such as, for example, 4 equivalents.

- Intermediates of Formula (XX) can be prepared by reacting a compound of Formula (I-b), wherein $X^1$, $X^2$, $X^3$, Y, $R^2$, and n are defined as in Formula (I), by conversion of the alcohol group to a suitable leaving group ($L^2$) such as, for example, mesylate, in the presence of a suitable base such as, for example, $Et_3N$, in a suitable solvent such as, for example, DCM, at a suitable temperature such as, for example, room temperature.

Alternatively, when $R^1$ is defined as $-CH_2OR^a$ or both $R^1$ and $R^{1a}$ are defined as $-CH_2OR^a$, compounds of Formula (I) can also be prepared according to Scheme 4,

- by reacting a compound of Formula (I-b), wherein $X^1$, $X^2$, $X^3$, Y, n, $R^2$, are defined as in Formula (I), and $R^{1a}$ is defined as $CH_2OH$ or H (hydrogen), with a suitable alkyl halide such as, for example, 2-bromoethyl methyl ether, in a suitable solvent such as, for example, DMF, and in the presence of a suitable base such as, for example, NaH, at a suitable temperature such as, for example, between room temperature and 80 °C.
- alternatively by reacting a compound of Formula (I-b), wherein $X^1$, $X^2$, $X^3$, Y, n, $R^2$, are defined as in Formula (I), and $R^{1a}$ is defined as $CH_2OH$ or H (hydrogen), with a suitable aryl halide such as, for example, 2-fluoropyridine, in a suitable solvent such as, for example, THF, and in the presence of a suitable base such as, for example, NaH, at a suitable temperature such as, for example, between room temperature and 50 °C.

Alternatively, when $R^1$ and/or $R^{1a}$ are defined as $-CH_2F$, compounds of Formula (I) can be prepared according to Scheme 4, by reacting an intermediate of Formula (I-b), wherein $R^1$ and/or $R^{1a}$ are defined as $-CH_2OH$, with a suitable fluorinating agent such as, for example, diethylaminosulfur trioxide, in a suitable solvent such as, for example, THF, at a suitable temperature such as, for example, -10 °C.

A skilled person will understand that in case $R^{1a}$ is of Formula $CH_2OR$, it can undergo similar transformations as shown for $R^1$ having Formula $CH_2OR$ in Scheme 4. When $R^{1a}$ is of Formula $CH_2OR$, $R^1$ could be a hydrogen, or of Formula $CH_2OR$. A skilled person will also understand that in case both $R^1$ and $R^{1a}$ are of Formula $CH_2OR$, differential or concurrent transformation of both $CH_2OR$ groups is possible depending on stoichiometry of reagents and/or protective group strategies.

Compounds of Formula (I-b) wherein the R group is hydrogen and $X^1$, $X^2$, $X^3$, Y, $R^2$, and n, are as defined in Formula (I) can be prepared by reacting a compound/intermediate of Formula (I-b) (compound or intermediate dependent on definition of R group), wherein R is a suitable protecting group such as, for example, TBDMS, with a suitable deprotecting agent such as, for example, TBAF, in a suitable solvent such as, for example, THF, at a suitable temperature such as, for example, room temperature.

Compounds/intermediates of Formula (I-b), wherein $X^1$, $X^2$, $X^3$, Y, and n, are defined as in Formula (I), $R^2$ is H (hydrogen), $R^{1a}$ is H (hydrogen), and $R^1$ is defined as $CH_2OR$, can be prepared according to Scheme 5,

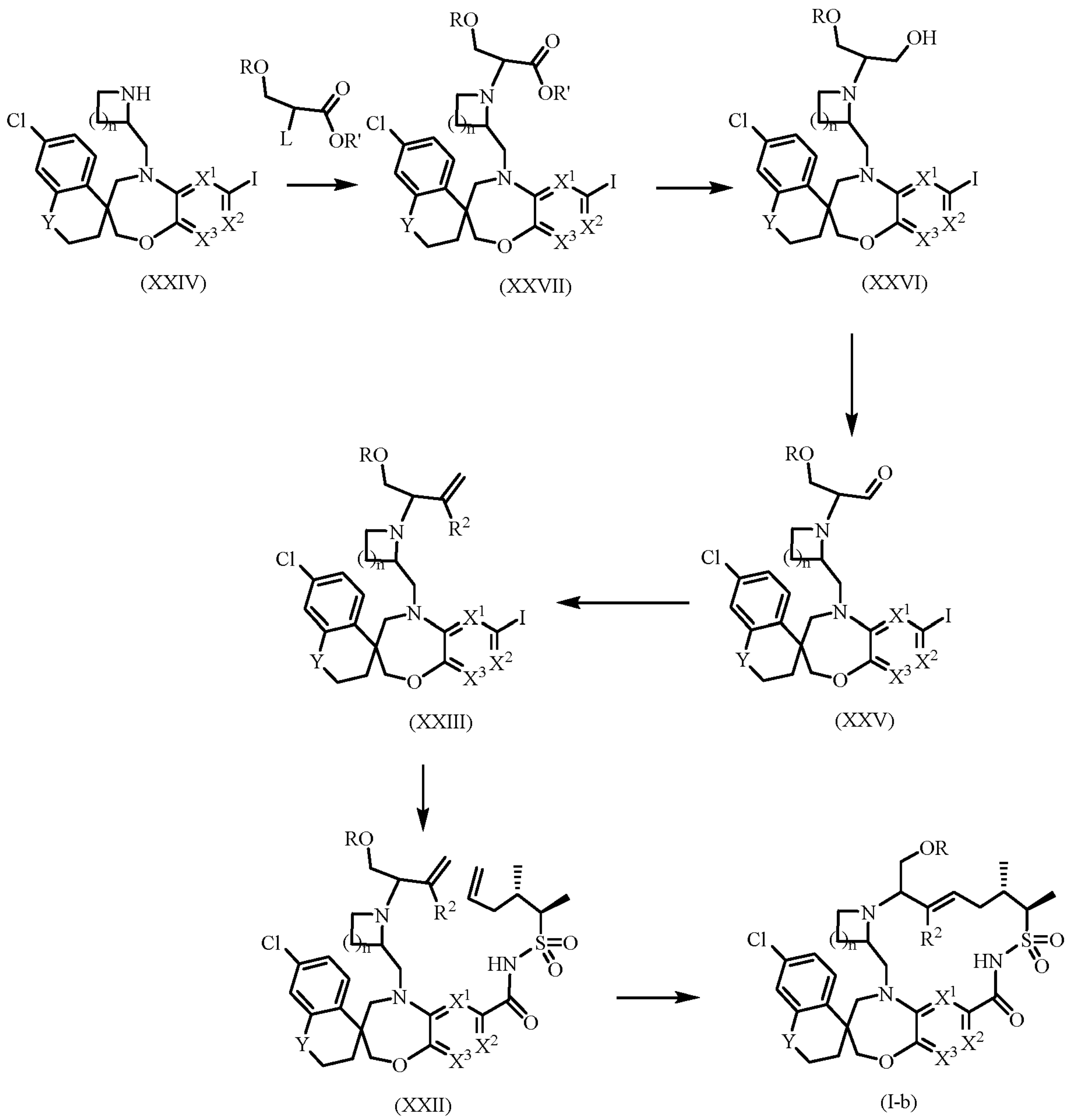

(XXIV) (XXVII) (XXVI)

(XXIII) (XXV)

(XXII) (I-b)

Scheme 5

- by reacting an intermediate of Formula (XXII), wherein $X^1$, $X^2$, $X^3$, Y, $R^2$, and n, are as defined in Formula (I), and R is defined as a suitable protecting group such as, for example, TBDMS, in the presence of a suitable catalyst such as, for example, the Grubbs-Hoveyda 2nd generation catalyst, in a suitable solvent such as, for example, DCE, at a suitable temperature such as, for example, 50 °C.
- Intermediates of Formula (XXII) can be prepared by reacting an intermediate of Formula (XXIII), wherein $X^1$, $X^2$, $X^3$, Y, $R^2$, and n, are as defined in Formula (I), and R is defined as a suitable protecting group such as, for example, TBDMS, with (2R,3S)-3-methylhex-5-ene-2-sulfonamide, in the presence of a suitable catalyst such as, for example, $PdCl_2$(dppf), in a suitable solvent such as, for example, THF, in the presence a suitable base such as, for example, DBU, and in the presence of CO gas, at a suitable pressure such as, for example, 50 bar, at a suitable temperature such as, for example, 100 °C.
- Intermediates of Formula (XXIII) can be prepared by reacting intermediates of Formula (XXV), wherein $X^1$, $X^2$, $X^3$, Y, and n, are as defined in Formula (I), and R is defined as a suitable protecting group such as, for example, TBDMS, or R is defined as an alkyl group such as, for example, Me, with a suitable alkyl triphenylphosphonium halide such as, for example, methyl triphenylphosphonium bromide, in a suitable solvent such as, for example, THF, in the presence of a suitable base such as, for example, KOtBu, at a suitable temperature such as, for example, between

0 °C and room temperature.

- Intermediates of Formula (XXV) can be prepared by reacting intermediates of Formula (XXVI), wherein $X^1$, $X^2$, $X^3$, Y, and n, are as defined in Formula (I), and R is defined as a suitable protecting group such as, for example, TBDMS, or R is defined as an alkyl group such as, for example, Me, with a suitable oxidizing agent such as, for example, a mixture of DMSO and oxalyl chloride, in a suitable solvent such as, for example, DCM, in the presence of a suitable base such as, for example, TEA, at a suitable temperature such as, for example, between -70 °C and room temperature.
- Intermediates of Formula (XXVI) can be prepared by reacting intermediates of Formula (XXVII), wherein $X^1$, $X^2$, $X^3$, Y, and n, are as defined in Formula (I), and R is defined as a suitable protecting group such as, for example, TBDMS, or R is defined as an alkyl group such as, for example, Me, and R' is defined as an alkyl group such as, for example, Me or Et, with a suitable reducing reagent such as, for example, DIBAH, in a suitable solvent such as, for example, THF, at a suitable temperature such as, for example, between -78 °C and room temperature.
- Intermediates of Formula (XXVII) can be prepared by reacting intermediates of Formula (XXIV), wherein $X^1$, $X^2$, $X^3$, Y, and n, are as defined in Formula (I), with a suitable halo alkyl propanoate such as, for example, 2-bromo-3-methoxypropanoate, in a suitable solvent such as, for example, DMF, in the presence of a suitable base such as, for example, DIPEA, at a suitable temperature such as, for example, room temperature.
- Alternatively, intermediates of Formula (XXVII) can be prepared in two steps, by reacting intermediates of Formula (XXIV), wherein $X^1$, $X^2$, $X^3$, Y, and n, are as defined in Formula (I), in the first step, in the presence of a suitable halo alkyl propanoate such as, for example, methyl 2-bromo-3-hydroxypropanoate, in a suitable solvent such as, for example, DMF, in the presence of a suitable base such as, for example, DIPEA, at a suitable temperature such as, for example, room temperature; and in the second step, in the presence of a suitable protecting group such as, for example, tert. butyl dimethyl silyl chloride, in the presence of a suitable base such as, for example, imidazole, in a suitable solvent such as, for example, DMF, at a suitable temperature such as, for example, room temperature.

Intermediates of Formula (XXIV), wherein $X^1$, $X^2$, $X^3$, Y, and n, are as defined in Formula (I), can be prepared according to Scheme 6,

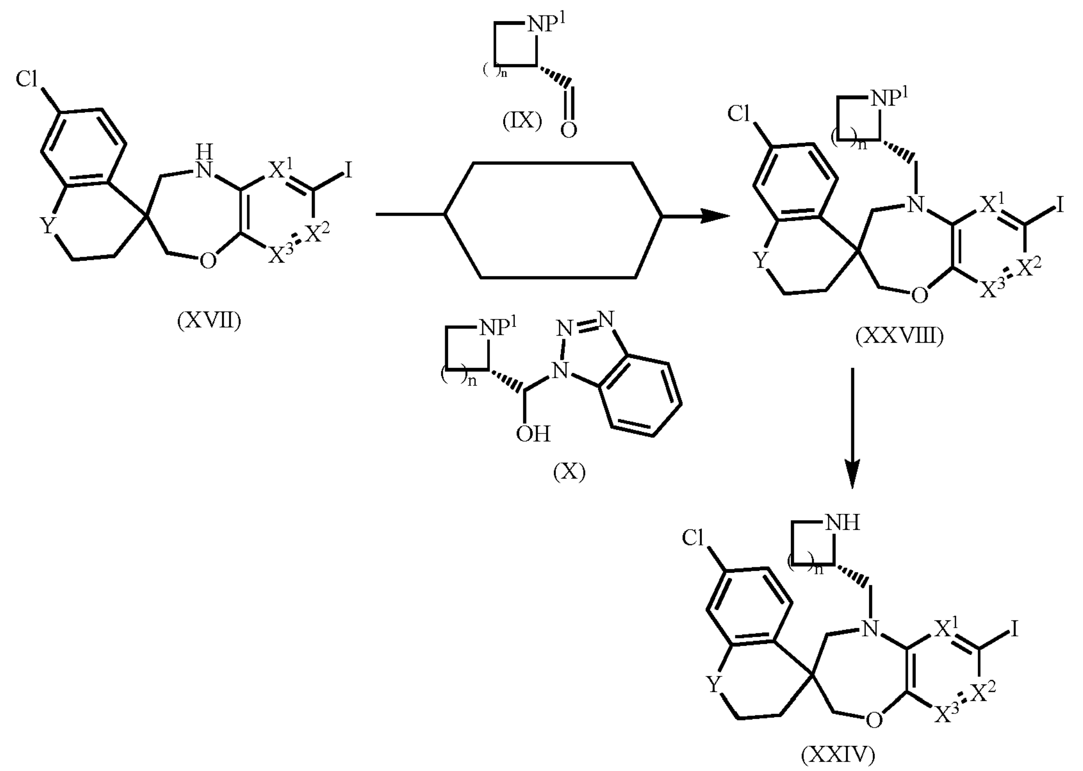

Scheme 6

- by reacting intermediates of Formula (XXVIII), wherein $P^1$ is defined as a suitable protecting group such as, for example, Boc, with a suitable deprotecting agent such as, for example, TFA, in a suitable solvent such as, for example, DCM, at a suitable temperature such as, for example, room temperature.

- Intermediates of Formula (XXVIII) can be prepared by reacting intermediates of Formula (XVII), wherein $X^1$, $X^2$, $X^3$, and Y are as defined in Formula (I), in a similar way as for the preparation of Intermediates of Formula (VII).

Alternatively, compounds of Formula (I) wherein $R^1$ and/or $R^{1a}$ are $CH_2OH$, can be prepared according to Scheme 7,

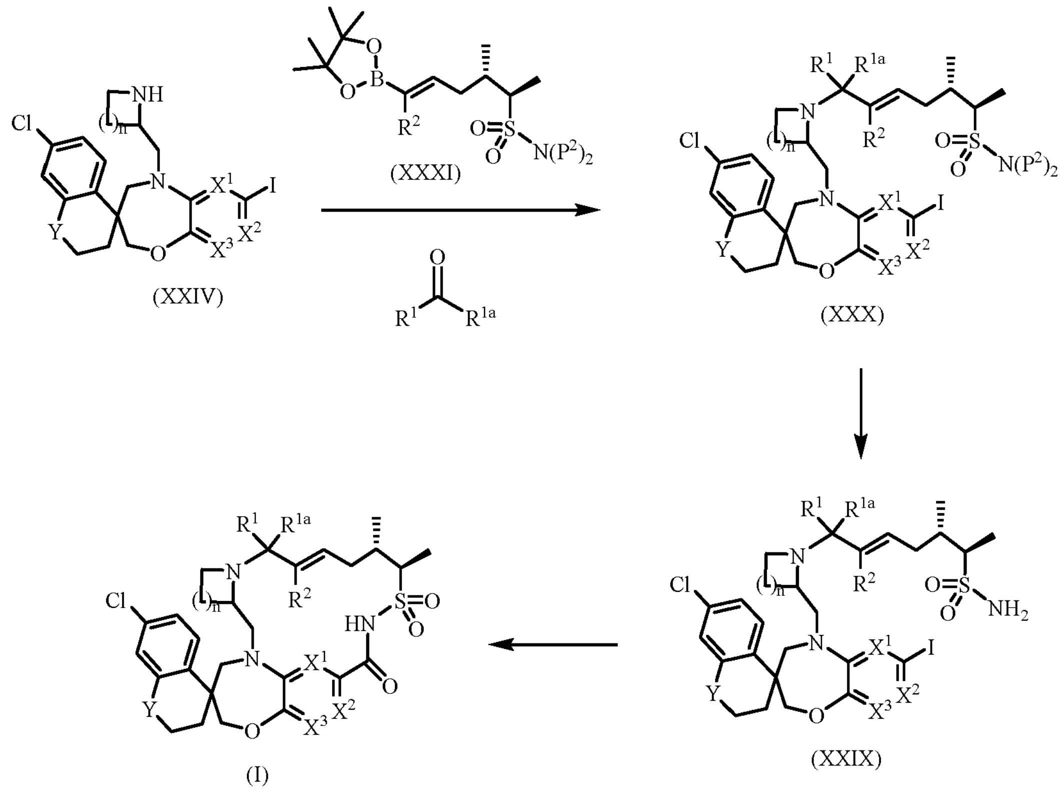

Scheme 7

- by reacting intermediates of Formula (XXIX), under a CO atmosphere, at a suitable pressure such as, for example, 50 bars, in the presence of a suitable base such as, for example, DBU, in the presence of a suitable catalyst such as, for example, $PdCl_2$(dppf), in a suitable solvent such as, for example, THF, at a suitable temperature such as, for example, 100 °C.
- Intermediates of Formula (XXIX) can be prepared by reacting an intermediate of Formula (XXX), wherein $X^1$, $X^2$, $X^3$, Y, n and $R^2$ are as defined in Formula (I), $R^1$ and/or $R^{1a}$ are $CH_2OH$, and $P^2$ is a suitable protecting group such as, for example, p-methoxybenzyl, with a suitable deprotecting agent such as, for example, TFA, in a suitable solvent such as, for example, DCM, at a suitable temperature such as, for example, room temperature.
- Intermediates of Formula (XXX) can be prepared by reacting an intermediate of Formula (XXIV) with an intermediate of Formula (XXXI) and a suitable aldehyde or a suitable aldehyde precursor such as, for example, 1,4-dioxane-2,5-diol (CAS [23147-58-2]), in a suitable solvent such as, for example, DCM, at a suitable temperature such as, for example, 50 °C.
- Alternatively, intermediates of Formula (XXX) can be prepared by reacting an intermediate of Formula (XXIV) with an intermediate of Formula (XXXI) and a suitable ketone such as, for example, 1,3-dihydroxyacetone, in the presence of a suitable base such as, for example, $Et_3N$, in a suitable solvent such as, for example, DCM, at a suitable temperature such as, for example, room temperature.

Intermediates of Formula (XXXI), wherein $R^2$ is defined as F (fluorine), can be prepared according to Scheme 8,

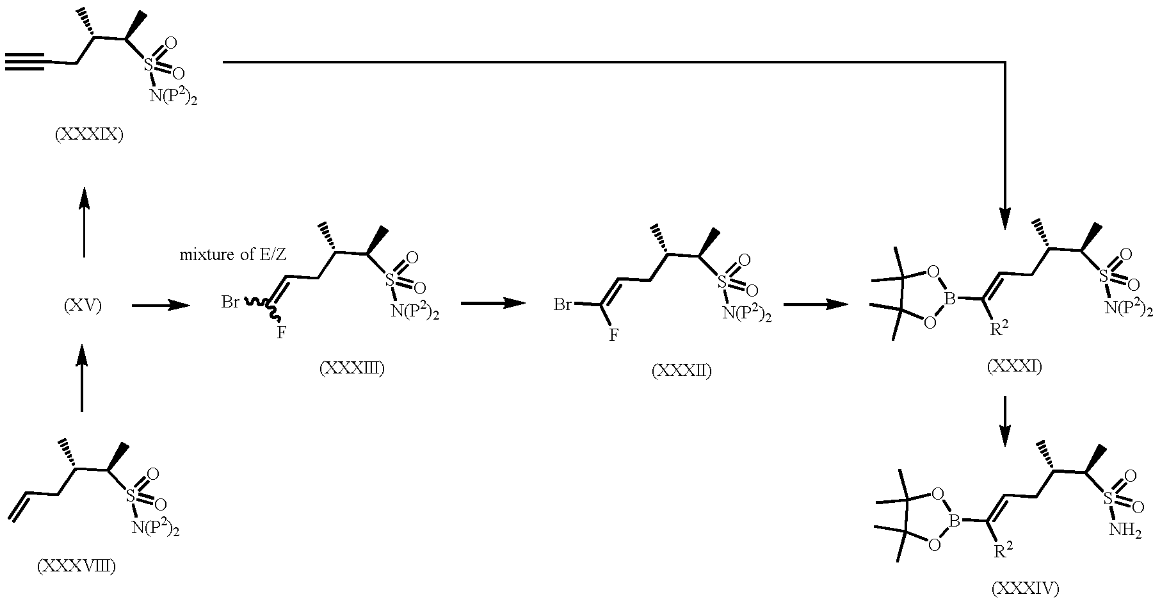

(XXXIX)
(XV)
(XXXIII)
(XXXII)
(XXXI)
(XXXVIII)
(XXXIV)

Scheme 8

- by reacting an intermediate of Formula (XXXII) with bis(pinacolato)diboron, in the presence of a suitable base such as, for example, potassium acetate, in the presence of a suitable catalyst such as, for example, $PdCl_2(dppf).CH_2Cl_2$, in a suitable solvent such as, for example, 1,4-dioxane, at a suitable temperature such as, for example, 95 °C.
- Intermediates of Formula (XXXII) can be prepared by reacting an intermediate of Formula (XXXIII) with a suitable base such as, for example, LiHMDS, in a suitable solvent such as, for example, THF, at a suitable temperature such as, for example 0 °C.
- Intermediates of Formula (XXXIII) can be prepared by reacting an intermediate of Formula (XV) with fluorotribromomethane and triphenylphosphine, in the presence of a suitable dialkylzinc derivative such as, for example, diethylzinc, in a suitable solvent such as, for example, THF, at a suitable temperature such as, for example, room temperature.
- Intermediates of Formula (XV) can be prepared by reacting an intermediate of Formula (XXXVIII) with ozone, in the presence of a suitable reductant such as, for example, triphenylphosphine, in a suitable solvent such as, for example, DCM or MeOH or a mixture thereof, at a suitable temperature such as, for example, -78 °C.
- Intermediates of Formula (XXXVIII) are described in literature or can be prepared in analogy with published procedures known to a skilled person.

Alternatively, intermediates of Formula (XXXI), wherein $R^2$ is defined as H (hydrogen), can be prepared according to Scheme 8,

- by reacting an intermediate of Formula (XXXIX) with a suitable borylation agent such as, for example, bis(pinacolato)diboron, in the presence of a suitable catalyst such as, for example, copper (I) oxide, in the presence of a suitable phosphine such as, for example, triphenylphosphine, in the presence of a suitable base such as, for example, $K_2HPO_4$, in a suitable solvent such as, for example, MeOH, at a suitable temperature such as, for example, room temperature.
- Intermediates of Formula (XXXIX) can be prepared by reacting an intermediate of Formula (XV) with alkynylation reagent such as, for example, dimethyl (1-diazo-2-oxopropyl)phosphonate, in the presence of a suitable base such as, for example, $K_2CO_3$, in a suitable solvent such as, for example, MeOH, at a suitable temperature such as, for example, room temperature.

Intermediates of Formula (XXXIV) can be prepared according to Scheme 8, by reacting an intermediate of Formula (XXXI) with a suitable deprotecting agent such as, for example, TFA, in a suitable solvent such as, for example, DCM, at a suitable temperature such as, for example, room temperature.
Alternatively, compounds of Formula (I), wherein $R^1$ and/or $R^{1a}$ is $CH_2OH$, can be prepared according to Scheme 9,

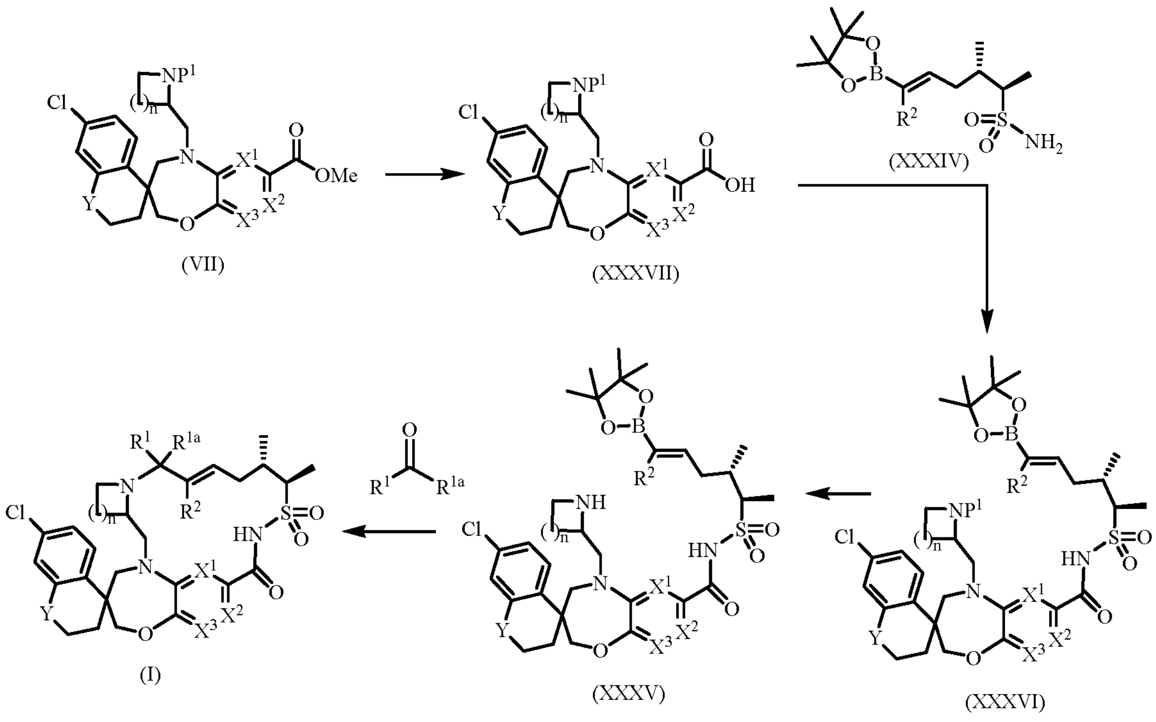

(VII) (XXXVII) (XXXIV) (XXXVI) (XXXV) (I)

Scheme 9

- by reacting an intermediate of Formula (XXXV) with a suitable aldehyde or a suitable aldehyde precursor, such as, for example, 1,4-dioxane-2,5-diol (CAS [23147-58-2]), in the presence of a suitable base such as, for example, $Et_3N$, in a suitable solvent such as, for example, DCM, at a suitable temperature such as, for example, room temperature.
- Alternatively by reacting an intermediate of Formula (XXXV) with a suitable ketone, such as, for example, 1,3-dihydroxyacetone, in the presence of a suitable base such as, for example, $Et_3N$, in a suitable solvent such as, for example, DCM, at a suitable temperature such as, for example, room temperature.
- Intermediates of Formula (XXXV) can be prepared by reacting an intermediate of Formula (XXXVI) with a suitable deprotecting agent such as, for example, TFA, in a suitable solvent such as, for example, DCM, at a suitable temperature such as, for example, room temperature.
- Intermediates of Formula (XXXVI) can be prepared by reacting an intermediate of Formula (XXXVII) with an intermediate of Formula (XXXIV), in the presence of a suitable coupling agent such as, for example, EDCI, in the presence of a suitable base such as, for example, $Et_3N$ or DMAP, or a mixture thereof, in a suitable solvent such as, for example, DCM, at a suitable temperature such as, for example, room temperature.
- Intermediates of Formula (XXXVII) can be prepared by reacting an intermediate of Formula (VII) with a suitable hydrolyzing agent such as, for example, LiOH, in a suitable solvent such as, for example, water, THF, or MeOH, or a mixture thereof, at a suitable temperature such as, for example, 50 °C.

It will be appreciated that where appropriate functional groups exist, compounds of various formulae or any intermediates used in their preparation may be further derivatized by one or more standard synthetic methods employing condensation, substitution, oxidation, reduction, or cleavage reactions. Particular substitution approaches include conventional alkylation, arylation, heteroarylation, acylation, sulfonylation, halogenation, nitration, formylation and coupling procedures.

The compounds of Formula (I) may be synthesized in the form of racemic mixtures of enantiomers which can be separated from one another following art-known resolution procedures. The racemic compounds of Formula (I) containing a basic nitrogen atom may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of Formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically.

In the preparation of compounds of the present invention, protection of remote functionality (e.g., primary or

secondary amine) of intermediates may be necessary. The need for such protection will vary depending on the nature of the remote functionality and the conditions of the preparation methods. Suitable amino-protecting groups (NH-Pg) include acetyl, trifluoroacetyl, t-butoxycarbonyl (Boc), benzyloxycarbonyl (CBz) and 9-fluorenylmethyleneoxycarbonyl (Fmoc). The need for such protection is readily determined by one skilled in the art. For a general description of protecting groups and their use, see T. W. Greene and P. G. M. Wuts, Protective Groups in Organic Synthesis, 4th ed., Wiley, Hoboken, New Jersey, 2007.

PHARMACOLOGY OF COMPOUNDS

It has been found that the compounds of the present invention inhibit one of more MCL-1 activities, such as MCL-1 antiapoptotic activity.

An MCL-1 inhibitor is a compound that blocks one or more MCL-1 functions, such as the ability to bind and repress proapoptotic effectors Bak and Bax or BH3 only sensitizers such as Bim, Noxa or Puma.

The compounds of the present invention can inhibit the MCL-1 pro-survival functions. Therefore, the compounds of the present invention may be useful in treating and / or preventing, in particular treating, diseases that are susceptible to the effects of the immune system such as cancer.

In another embodiment of the present invention, the compounds of the present invention exhibit anti-tumoral properties, for example, through immune modulation.

In an embodiment, the present invention is directed to a compound of formula (I) for use in methods for treating and / or preventing a cancer, wherein the cancer is selected from those described herein, comprising administering to a subject in need thereof (preferably a human), a therapeutically effective amount of a compound of Formula (I), or pharmaceutically acceptable salt, or a solvate thereof.

In an embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of acute lymphoblastic leukemia (ALL), acute myeloid leukemia (AML), B cells acute lymphoblastic leukemia, B-cell chronic lymphocytic leukemia (CLL), bladder cancer, breast cancer, chronic lymphocytic leukemia, chronic myeloid leukemia, colon adenocarcinoma, diffuse large B cell lymphoma, esophageal cancer, follicular lymphoma, gastric cancer, head and neck cancer (including, but not limited to head and neck squamous cell carcinoma), hematopoietic cancer, hepatocellular carcinoma, Hodgkin lymphoma, liver cancer, lung cancer (including but not limited to lung adenocarcinoma), lymphoma, medulloblastoma, melanoma, monoclonal gammopathy of undetermined significance, multiple myeloma, myelodysplastic syndromes, myelofibrosis, myeloproliferative neoplasms, ovarian cancer, ovarian clear cell carcinoma, ovarian serous cystadenoma, pancreatic cancer, polycythemia vera, prostate cancer, rectum adenocarcinoma, renal cell carcinoma, smoldering multiple myeloma, T cell acute lymphoblastic leukemia, T cell lymphoma, and Waldenstroms macroglobulinemia.

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is preferably selected from the group consisting of acute lymphoblastic leukemia (ALL), acute myeloid leukemia (AML), B cells acute lymphoblastic leukemia, B-cell chronic lymphocytic leukemia (CLL), breast cancer, chronic lymphocytic leukemia, chronic myeloid leukemia, diffuse large B cell lymphoma, follicular lymphoma, hematopoietic cancer, Hodgkin lymphoma, lung cancer (including, but not limited to lung adenocarcinoma) lymphoma, monoclonal gammopathy of undetermined significance, multiple myeloma, myelodysplastic syndromes, myelofibrosis, myeloproliferative neoplasms, smoldering multiple myeloma, T cell acute lymphoblastic leukemia, T cell lymphoma and Waldenstroms macroglobulinemia.

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of adenocarcinoma, benign monoclonal gammopathy, biliary cancer (including, but not limited to, cholangiocarcinoma), bladder cancer, breast cancer (including, but not limited to, adenocarcinoma of the breast, papillary carcinoma of the breast, mammary cancer, medullary carcinoma of the breast), brain cancer (including, but not limited to, meningioma), glioma (including, but not limited to, astrocytoma, oligodendroglioma; medulloblastoma), bronchus cancer, cervical cancer (including, but not limited to, cervical adenocarcinoma), chordoma, choriocarcinoma, colorectal cancer (including, but not limited to, colon cancer, rectal cancer, colorectal adenocarcinoma), epithelial carcinoma, endothelial sarcoma (including, but not limited to, Kaposi's sarcoma, multiple idiopathic hemorrhagic sarcoma), endometrial cancer (including, but not limited to, uterine cancer, uterine sarcoma), esophageal cancer (including, but not limited to, adenocarcinoma of the esophagus, Barrett's adenocarinoma), Ewing sarcoma, gastric cancer (including, but not limited to, stomach adenocarcinoma), gastrointestinal stromal tumor (GIST),

head and neck cancer (including, but not limited to, head and neck squamous cell carcinoma), hematopoietic cancers (including, but not limited to, leukemia such as acute lymphocytic leukemia (ALL) (including, but not limited to, B-cell ALL, T-cell ALL), acute myelocytic leukemia (AML) (e.g. B-cell AML, T-cell AML), chronic myelocytic leukemia (CML) (e.g. B-cell CML, T-cell CML), and chronic lymphocytic leukemia (CLL) (e.g. B-cell CLL, T- cell CLL), lymphoma such as Hodgkin lymphoma (HL) (including, but not limited to, B-cell HL, T-cell HL) and non-Hodgkin lymphoma (NHL) (e.g. B-cell NHL such as diffuse large cell lymphoma (DLCL) (e.g. diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (including, but not limited to, mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (including, but not limited to, Waldenstrom's macro globulinemia), immunoblastic large cell lymphoma, hairy cell leukemia (HCL), precursor B -lymphoblastic lymphoma and primary central nervous system (CNS) lymphoma, T-cell NHL such as precursor T-lymphoblastic lymphoma/leukemia, peripheral T-cell lymphoma (PTCL) (e.g. cutaneous T-cell lymphoma (CTCL) (including, but not limited to, mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma, a mixture of one or more leukemia/lymphoma as described above, multiple myeloma (MM), heavy chain disease (including, but not limited to, alpha chain disease, gamma chain disease, mu chain disease), immunocytic amyloidosis, kidney cancer (including, but not limited to, nephroblastoma a.k.a. Wilms' tumor, renal cell carcinoma), liver cancer (including, but not limited to, hepatocellular cancer (HCC), malignant hepatoma), lung cancer (including, but not limited to, bronchogenic carcinoma, non-small cell lung cancer (NSCLC), squamous lung cancer (SLC), adenocarcinoma of the lung, *Lewis lung carcinoma*, lung neuroendocrine tumors, typical carcinoid, atypical carcinoid, small cell lung cancer (SCLC), and large cell neuroendocrine carcinoma), myelodysplastic syndromes (MDS), myeloproliferative disorder (MPD), polycythemia vera (PV), essential thrombocytosis (ET), agnogenic myeloid metaplasia (AMM) a.k.a. myelofibrosis (MF), chronic idiopathic myelofibrosis, chronic myelocytic leukemia (CML), chronic neutrophilic leukemia (CNL), hypereosinophilic syndrome (HES), ovarian cancer (including, but not limited to, cystadenocarcinoma, ovarian embryonal carcinoma, ovarian adenocarcinoma), pancreatic cancer (including, but not limited to, pancreatic andenocarcinoma, intraductal papillary mucinous neoplasm (IPMN), Islet cell tumors), prostate cancer (including, but not limited to, prostate adenocarcinoma), skin cancer (including, but not limited to, squamous cell carcinoma (SCC), keratoacanthoma (KA), melanoma, basal cell carcinoma (BCC)) and soft tissue sarcoma (e.g. malignant fibrous histiocytoma (MFH), liposarcoma, malignant peripheral nerve sheath tumor (MPNST), chondrosarcoma, fibrosarcoma, myxosarcoma).

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of benign monoclonal gammopathy, breast cancer (including, but not limited to, adenocarcinoma of the breast, papillary carcinoma of the breast, mammary cancer, medullary carcinoma of the breast), hematopoietic cancers (including, but not limited to, leukemia such as acute lymphocytic leukemia (ALL) (including, but not limited to, B-cell ALL, T-cell ALL), acute myelocytic leukemia (AML) (e.g. B-cell AML, T-cell AML), chronic myelocytic leukemia (CML) (e.g. B-cell CML, T-cell CML), and chronic lymphocytic leukemia (CLL) (e.g. B-cell CLL, T- cell CLL), lymphoma such as Hodgkin lymphoma (HL) (including, but not limited to, B-cell HL, T-cell HL) and non-Hodgkin lymphoma (NHL) (e.g. B-cell NHL such as diffuse large cell lymphoma (DLCL) (e.g. diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (including, but not limited to, mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (including, but not limited to, Waldenstrom's macro globulinemia), immunoblastic large cell lymphoma, hairy cell leukemia (HCL), precursor B -lymphoblastic lymphoma and primary central nervous system (CNS) lymphoma, T-cell NHL such as precursor T-lymphoblastic lymphoma/leukemia, peripheral T-cell lymphoma (PTCL) (e.g. cutaneous T-cell lymphoma (CTCL) (including, but not limited to, mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma, a mixture of one or more leukemia/lymphoma as described above, multiple myeloma (MM), heavy chain disease (including, but not limited to, alpha chain disease, gamma chain disease, mu chain disease), immunocytic amyloidosis, liver cancer (including, but not limited to, hepatocellular cancer (HCC), malignant hepatoma), lung cancer (including, but not limited to, bronchogenic carcinoma, non-small cell lung cancer (NSCLC), squamous lung cancer (SLC), adenocarcinoma of the lung, Lewis lung carcinoma, lung neuroendocrine tumors, typical carcinoid, atypical carcinoid, small cell lung cancer (SCLC), and large cell neuroendocrine carcinoma), myelodysplastic syndromes (MDS), myeloproliferative disorder (MPD), and prostate cancer (including, but not limited to, prostate adenocarcinoma).

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is selected from the group consisting of prostate, lung, pancreatic, breast, ovarian, cervical, melanoma, B-cell chronic lymphocytic leukemia (CLL), acute myeloid leukemia (AML), and acute lymphoblastic leukemia (ALL).

In another embodiment, the present invention is directed to a compound of formula (I) for use in a method for treating and / or preventing cancer comprising administering to a subject in need thereof, preferably a human, a therapeutically effective amount of a compound of Formula (I), or a pharmaceutically acceptable salt, or a solvate thereof, wherein the cancer is multiple myeloma.

The compounds according to the present invention or pharmaceutical compositions comprising said compounds, may also have therapeutic applications in combination with immune modulatory agents, such as inhibitors of the PD1/PDL1 immune checkpoint axis, for example antibodies (or peptides) that bind to and/or inhibit the activity of PD-1 or the activity of PD-L1 and or CTLA-4 or engineered chimeric antigen receptor T cells (CART) targeting tumor associated antigens.

The compounds according to the present invention or pharmaceutical compositions comprising said compounds, may also be combined with radiotherapy or chemotherapeutic agents (including, but not limited to, anti-cancer agents) or any other pharmaceutical agent which is administered to a subject having cancer for the treatment of said subject's cancer or for the treatment or prevention of side effects associated with the treatment of said subject's cancer.

The compounds according to the present invention or pharmaceutical compositions comprising said compounds, may also be combined with other agents that stimulate or enhance the immune response, such as vaccines.

In an embodiment, the present invention is directed to a compound of formula (I) for use in methods for treating and / or preventing a cancer (wherein the cancer is selected from those described herein) comprising administering to a subject in need thereof (preferably a human), a therapeutically effective amount of co-therapy or combination therapy; wherein the co-therapy or combination therapy comprises a compound of Formula (I) of the present invention and one or more anti-cancer agent(s) selected from the group consisting of (a) immune modulatory agent (such as inhibitors of the PD1/PDL1 immune checkpoint axis, for example antibodies (or peptides) that bind to and/or inhibit the activity of PD-1 or the activity of PD-L1 and or CTLA-4); (b) engineered chimeric antigen receptor T cells (CART) targeting tumor associated antigens; (c) radiotherapy; (d) chemotherapy; and (e) agents that stimulate or enhance the immune response, such as vaccines.

The present invention is directed to compounds of Formula (I) and pharmaceutically acceptable salts, and solvates thereof, for use as a medicament.

The present invention is directed to compounds of Formula (I) and pharmaceutically acceptable salts, and solvates thereof, for use in the inhibition of MCL-1 activity.

As used herein, unless otherwise noted, the term "anti-cancer agents" shall encompass "anti-tumor cell growth agents" and "anti-neoplastic agents".

The present invention is directed to compounds of Formula (I) and pharmaceutically acceptable salts, and solvates thereof, for use in treating and / or preventing diseases (preferably cancers) mentioned above.

The present invention is directed to compounds of Formula (I) and pharmaceutically acceptable salts, and solvates thereof, for treating and / or preventing diseases (preferably cancers) mentioned above.

The present invention is directed to compounds of Formula (I) and pharmaceutically acceptable salts, and solvates thereof, for treating and / or preventing, in particular for treating, a disease, preferably a cancer, as described herein (for example, multiple myeloma).

The present invention is directed to compounds of Formula (I) and pharmaceutically acceptable salts, and solvates thereof, for use in treating and / or preventing, in particular for treating, a disease, preferably a cancer, as described herein (for example, multiple myeloma).

The present invention is directed to compounds of Formula (I) and pharmaceutically acceptable salts, and solvates thereof, for treating and / or preventing, in particular for treating, MCL-1 mediated diseases or conditions, preferably cancer, more preferably a cancer as herein described (for example, multiple myeloma).

The present invention is directed to compounds of Formula (I) and pharmaceutically acceptable salts, and solvates thereof, for use in treating and / or preventing, in particular for use in treating, MCL-1 mediated diseases or conditions, preferably cancer, more preferably a cancer as herein described (for example, multiple myeloma).

The present invention relates to compounds of Formula (I) and pharmaceutically acceptable salts, and solvates thereof, for the manufacture of a medicament.

The present invention relates to compounds of Formula (I) and pharmaceutically acceptable salts, and solvates thereof, for the manufacture of a medicament for the inhibition of MCL-1.

The present invention relates to compounds of Formula (I) and pharmaceutically acceptable salts, and solvates thereof, for the manufacture of a medicament for treating and / or preventing, in particular for treating, a cancer, preferably a cancer as herein described. More particularly, the cancer is a cancer which responds to inhibition of MCL-1 (for example, multiple myeloma).

The present invention is directed to compounds of Formula (I) and pharmaceutically acceptable salts, and

solvates thereof, for the manufacture of a medicament for treating and / or preventing, in particular for treating, any one of the disease conditions mentioned hereinbefore.

The present invention is directed to compounds of Formula (I) and pharmaceutically acceptable salts, and solvates thereof, for the manufacture of a medicament for treating and / or preventing any one of the disease conditions mentioned hereinbefore.

The compounds of Formula (I) and pharmaceutically acceptable salts, and solvates thereof, can be administered to subjects, preferably humans, for treating and / or preventing of any one of the diseases mentioned hereinbefore.

In view of the utility of the compounds of Formula (I) and pharmaceutically acceptable salts, and solvates thereof, there is provided a compound of Formula (I) for use in a method of treating subjects, preferably mammals such as humans, suffering from any of the diseases mentioned hereinbefore; or a method of slowing the progression of any of the diseases mentioned hereinbefore in subject, humans; or a compound of Formula (I) for use in a method of preventing subjects, preferably mammals such as humans, from suffering from any one of the diseases mentioned hereinbefore.

Said methods comprise the administration, i.e. the systemic or topical administration, preferably oral or intravenous administration, more preferably oral administration, of an effective amount of a compound of Formula (I) or a pharmaceutically acceptable salt, or a solvate thereof, to subjects such as humans.

One skilled in the art will recognize that a therapeutically effective amount of the compounds of the present invention is the amount sufficient to have therapeutic activity and that this amount varies *inter alias*, depending on the type of disease, the concentration of the compound in the therapeutic formulation, and the condition of the patient. In an embodiment, a therapeutically effective daily amount may be from about 0.005 mg/kg to 100 mg/kg.

The amount of a compound according to the present invention, also referred to herein as the active ingredient, which is required to achieve a therapeutic effect may vary on case-by-case basis, for example with the specific compound, the route of administration, the age and condition of the recipient, and the particular disorder or disease being treated. The methods of the present invention may also include administering the active ingredient on a regimen of between one and four intakes per day. In these methods of the present invention, the compounds according to the invention are preferably formulated prior to administration.

The present invention also provides compositions for treating and / or preventing the disorders (preferably a cancer as described herein) referred to herein. Said compositions comprise a therapeutically effective amount of a compound of Formula (I), or a pharmaceutically acceptable salt, or a solvate thereof, and a pharmaceutically acceptable carrier or diluent.

While it is possible for the active ingredient (e.g. a compound of the present invention) to be administered alone, it is preferable to administer it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of the present invention may be prepared by any methods well known in the art of pharmacy, for example, using methods such as those described in, for example, Gennaro et al. Remington's Pharmaceutical Sciences (18th ed., Mack Publishing Company, 1990, see especially Part 8 : Pharmaceutical preparations and their Manufacture).

The compounds of the present invention may be administered alone or in combination with one or more additional therapeutic agents. Combination therapy includes administration of a single pharmaceutical dosage formulation which contains a compound according to the present invention and one or more additional therapeutic agents, as well as administration of the compound according to the present invention and each additional therapeutic agent in its own separate pharmaceutical dosage formulation.

Therefore, in an embodiment, the present invention is directed to a product comprising, as a first active ingredient a compound according to the invention and as further, as an additional active ingredient one or more anti-cancer agent(s), as a combined preparation for simultaneous, separate or sequential use in the treatment of patients suffering from cancer.

The one or more other anti-cancer agents and the compound according to the present invention may be administered simultaneously (e.g. in separate or unitary compositions) or sequentially, in either order. In an embodiment, the two or more compounds are administered within a period and / or in an amount and / or a manner that is sufficient to ensure that an advantageous or synergistic effect is achieved. It will be appreciated that the preferred method and order of administration and the respective dosage amounts and regimes for each component of the combination will depend on the particular other anti-cancer agent and the compound of the present invention being administered, their route of administration, the particular condition, in particular tumor, being treated and the particular host being treated.

The following examples further illustrate the present invention.

EXAMPLES

Several methods for preparing the Compounds of this invention are illustrated in the following examples. Unless otherwise noted, all starting materials were obtained from commercial suppliers and used without further purification, or alternatively can be synthesized by a skilled person by using published methods.

Table 1: Abbreviations

| Abbreviation | Meaning |
|---|---|
| **AcOH** | acetic acid |
| **DCM** | dichloromethane |
| **min** | minute(s) |
| **DCE** | dichloroethane |
| **DMF** | *N*,*N*-dimethylformamide |
| **DIPE** | diisopropyl ether |
| **DIPEA** | *N*,*N*-diisopropylethylamine |
| **DBU** | 1,8-diazabicyclo[5.4.0]undec-7-ene |
| **DMSO** | dimethyl sulfoxide |
| **DMAP** | 4-dimethylaminopyridine |
| **Me** | methyl |
| **MsCl** | methanesulfonyl chloride |
| **EDC.HCl or EDCI** | N-(3-dimethylaminopropyl)-N'-ethylcarbodiimide hydrochloride |
| **EtOAc** | ethyl acetate |
| **$Et_3N$** | triethylamine |
| **eq.** | equivalent(s) |
| **Et** | ethyl |
| **EtOH** | ethanol |
| **Me** | methyl |
| **MeOH** | methanol |
| **h** | hour(s) |
| **min** | minute(s) |
| **DIBALH or DIBAH** | di-isobutylaluminiumhydride |
| **HPLC** | high performance liquid chromatography |
| **SFC** | supercritical fluid chromatography |
| **Prep** | preparative |
| **RP** | reversed phase |
| **MeOH** | methanol |
| **LiHMDS** | lithium bis(trimethylsilyl)amide |
| **$NaBH(OAc)_3$** | sodium triacetoxyborohydride |
| **SFC** | super critical fluid chromatography |
| **THF** | tetrahydrofuran |
| **Celite®** | diatomaceous earth |
| **Dicalite®** | diatomaceous earth |

(continued)

| Abbreviation | Meaning |
|---|---|
| **RP** | reversed phase |
| **iPrNH$_2$** | isopropylamine |
| **Boc** | tert-butyloxycarbonyl |
| **DEAD** | diethyl azodicarboxylate |
| **DECP** | diethyl cyanophosphonate |
| **DAST** | (diethylamino)sulfur trifluoride |
| **DIAD** | diisopropyl azodicarboxylate |
| **DPPA** | diphenylphosphoryl azide |
| **PBu$_3$** | tributylphosphine |
| **NaOAc** | sodium acetate |
| **PPh$_3$ or Ph$_3$P** | triphenylphosphine |
| **PMB** | p-methoxybenzyl |
| **TFA** | trifluoroacetic acid |
| **tBuOK or KOtBu** | potassium tert-butoxide |
| **PdCl$_2$(dppf)** | [1,1'-bis(diphenylphosphino)ferrocene]dichloropalladium(II) |
| **TBDMS** | tert-butyldimethylsilyl |

As understood by a person skilled in the art, Compounds synthesized using the protocols as indicated may contain residual solvent or minor impurities.

A skilled person will realize that, even where not mentioned explicitly in the experimental protocols below, typically after a column chromatography purification, the desired fractions were collected and the solvent was evaporated.

In case no stereochemistry is indicated, this means it is a mixture of stereoisomers, unless otherwise is indicated or is clear from the context.

**Preparation of intermediates**

For intermediates that were used in a next reaction step as a crude or as a partially purified intermediate, in some cases no mol amounts are mentioned for such intermediate in the next reaction step or alternatively estimated mol amounts or theoretical mol amounts for such intermediate in the next reaction step are indicated in the reaction protocols described below.

A1) tert-butyl (S)-2-formylazetidine-1-carboxylate (Intermediate 1)

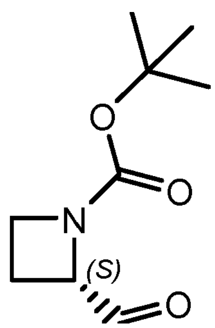

Dess-Martin periodinane [87413-09-0] (30 g, 1.3 eq.) was added to a stirred solution of (S)-1-Boc-2-azetidinemethanol [161511-85-9] (10 g, 53.4 mmol) in DCM (250 mL) at 0 °C. The reaction mixture was stirred at room temperature for 2 h. The reaction mixture was quenched by the addition of a solution of sodium thiosulfate in a saturated aqueous NaHCOs solution. The resulting mixture was stirred vigorously for 15 min. The resulting suspension was filtered over a pad of Celite®. The filter pad was washed with dichloromethane. The combined filtrate was separated, and the aqueous layer was extracted with DCM (2x). The combined organic layer was washed twice with saturated aqueous $NaHCO_3$, dried with $MgSO_4$, and filtered. The solvents of the filtrate were evaporated to give 10.17 g of Intermediate 1 (quantitative) as an oil, used without further purification.

tert-butyl (S)-2-((1H-benzo[d][1,2,3]triazol-1-yl)(hydroxy)methyl)azetidine-1-carboxylate Intermediate 2)

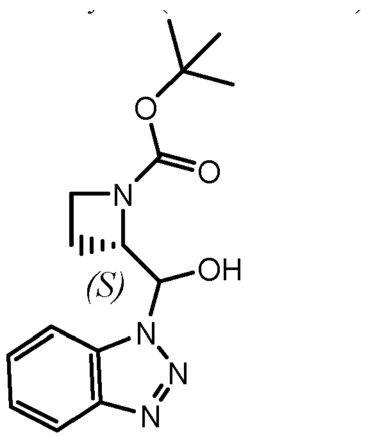

Tert-butyl methyl ether (40 mL) was added to Intermediate 1 (9 g, 48.59 mmol). The resulting suspension was stirred for 10 minutes at room temperature and then filtered. The solid was rinsed with tert-butyl methyl ether. The filtrate was transferred to a round bottom flask and then cooled down to 0 °C. 1H-benzotriazole (5.79 g, 1 eq.) was added to the solution and the reaction mixture was stirred at room temperature for 18 h. The solvents were evaporated to give Intermediate 2 (14.79 g, quantitative), used without further purification.

2-(((2R,3 S)-3-methylhex-5-en-2-yl)thio)pyrimidine (Intermediate 3)

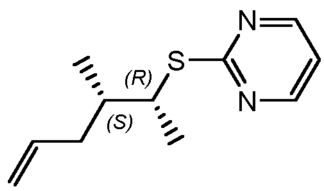

A solution of DEAD (25.79 mL, 1.7 eq.) in THF (100 mL) was added dropwise to $PBu_3$ (36.09 mL, 1.5 eq.) in degassed THF (300 mL) under nitrogen atmosphere at 0 °C. A solution of (2S,3S)-3-methylhex-5-en-2-ol [125225-80-1] (11 g, 1 eq.) in THF (200 mL) was added dropwise to the mixture at 0 °C. The mixture was stirred at 0 °C for 30 min (the solution turned light orange). Pyrimidine-2-thiol [131242-36-9] (30.79 g, 2.85 eq.) was added gradually to the mixture. The reaction mixture was stirred at 0 °C for 1 h and then at room temperature overnight. The reaction mixture was filtered. To the filtrate was added 500 mL EtOAc. The solution was washed twice with 1 N $K_2CO_3$ (300 mL) and then twice with brine (300 mL). The aqueous layer was back-extracted with 400 mL EtOAc. The combined organic layer was dried over $Na_2SO_4$ and concentrated in vacuo. The residue was purified by flash column chromatography over silica gel (eluent: petroleum ether/EtOAc from 100/0 to 0/100). The desired fractions were collected and the solvent was concentrated to dryness under vacuum to give Intermediate 3 (35.4 g, 68 % yield).

2-(((2R,3S)-3-methylhex-5-en-2-yl)sulfonyl)pyrimidine Intermediate 4

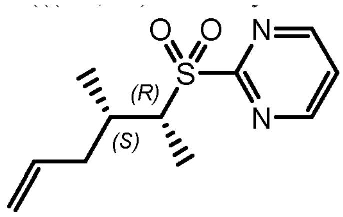

To a mixture of $Na_2WO_4$ [10213-10-2] (1.075 g, 0.1 eq.), phenylphosphonic acid [157171-33-1] (0.515 g, 0.1 eq.) and tetrabutylammonium sulfate (3.75 mL, 0.1 eq.) was added $H_2O_2$ (9.24 g, 2.5 eq.) all at once at room temperature. The mixture was aged at room temperature for 5 minutes before a solution of Intermediate 3 (10 g, 1 eq.) in toluene (150 mL) was added in one portion. The biphasic reaction mixture was heated to 50 °C with vigorous stirring. After 60 minutes the reaction was cooled to room temperature. The reaction mixture was poured into water (150 mL). The layers were separated and the aqueous layer was extracted with EtOAc (300 mL × 3). The combined organic layer was washed with a saturated aqueous solution of NazSzOs (200 mL × 2), dried over $MgSO_4$, filtered, and concentrated. The residue was purified by flash column chromatography over silica gel (eluent: petroleum ether/EtOAc from 100/0 to 0/100). The desired fractions were collected and the solvent was concentrated under vacuum to give Intermediate 4 (5.6 g, 68 %) as a yellow oil.

Sodium (2R,3S)-3-methylhex-5-ene-2-sulfonate (Intermediate 5)

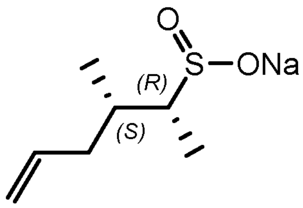

A solution of Intermediate 4 (5.6 g, 1 eq.) in MeOH (30 mL) was cooled to 0 °C and treated with NaOMe (4.794 g, 1 eq.). The mixture was allowed to warm to room temperature for 20 min. The solvent was removed under vacuum. Water (10 mL) was added to the mixture and it was extracted with EtOAc (10 mL × 3). The combined organic layer was concentrated under vacuum to give Intermediate 5 (6.3 g, quantitative) as a yellow solid. The product was used for the next step without further purification.

(2R,3S)-3-methylhex-5-ene-2-sulfonamide (Intermediate 6)

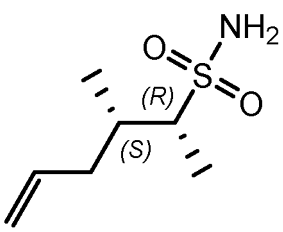

Intermediate 5 (33.2 g, 1 eq.) was dissolved in MeOH (200 mL) and treated with NaOAc (18.48 g, 1.25 eq.), followed by a solution of hydroxylamine O-sulfonic acid (25.48 g, 1.25 eq.) in water (15 mL). The reaction was stirred overnight at room temperature. The mixture was neutralized with solid NaHCOs and extracted with EtOAc (400 mL × 3). The combined organic layers was dried over $MgSO_4$, filtered and concentrated. The residue was purified by flash

column chromatography over silica gel (eluent: petroleum ether/EtOAc from 100/0 to 0/100). The desired fractions were collected, and the solvent was concentrated under vacuum to give Intermediate 6 (18.1 g, 56 %) as clear oil.

(2R,3S)-3-methylhex-5-ene-2-sulfonamide (Intermediate 7)

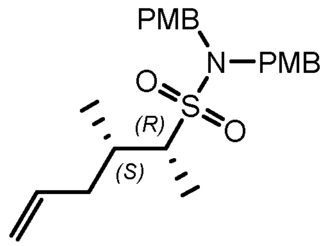

To a solution of Intermediate 6 (5 g, 28.2 mmol) in DMF (50 mL) was added $K_2CO_3$ (15.593 g, 4 eq.), followed by slow addition of 4-methoxybenzyl chloride (11.474 mL, 3 eq). Once the addition was complete, the reaction was heated to 70 °C and was stirred at this temperature overnight. The reaction was filtered through a pad of dicalite® to remove the inorganics and concentrated under reduced pressure to give a pale-yellow oil. The oil was dissolved in EtOAc (150 mL) and washed with brine (2 × 100 mL). The organic layer was dried over $MgSO_4$, filtered, and concentrated under reduced pressure to afford a pale-yellow oil. The crude product was purified by flash column chromatography on silica gel (heptane:EtOAc - 1:0 to 8:2). After evaporation of the fractions containing product, the residue was purified by preparative HPLC (Stationary phase: RP XBridge Prep C18 OBD-10 μm,50×150 mm, Mobile phase: 0.25 % $NH_4HCO_3$ solution in water, $CH_3CN$) to give Intermediate 7 (5.98 g, yield 48 %) as a white solid.

(2R,3S)-N,N-bis(4-methoxybenzyl)-3-methyl-5-oxopentane-2-sulfonamide

(Intermediate 8)

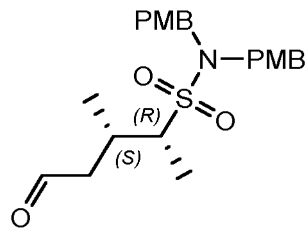

Intermediate 7 (1 g, 2.275 mmol) was dissolved in a mixture of DCM (12.5 mL) and MeOH (12.5 mL) and the resulting mixture was cooled to -78 °C. Ozone (109.199 mg, 1 eq.) was subsequently bubbled through the reaction mixture until a blue persistent color was observed (5 min). Nitrogen was then bubbled through the solution (still at -78 °C) to remove the blue color and this was followed by addition of $PPh_3$ (2.984 g, 5 eq). Once the addition was complete, the reaction was left stirring at -78 °C for 1 h. The reaction mixture was then slowly allowed to warm to room temperature and was stirred for 1 h. The heterogenous mixture was filtered through a pad of dicalite®. The pad was thoroughly washed with DCM. The filtrate was concentrated under reduced pressure to give a green oil that was purified by flash column chromatography on silica gel (heptane:EtOAc - 1:0 to 3:1) to give Intermediate 8 (920 mg, yield 87 %) as a colorless oil.

(2R,3S)-N,N-bis[(4-methoxyphenyl)methyl]-3-methyl-hex-5-yne-2-sulfonamide

(Intermediate 37)

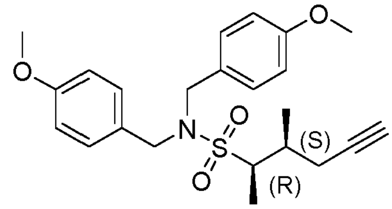

Dimethyl (1-diazo-2-oxopropyl)phosphonate (0.3 mL, 1 eq.) was added to a suspension of Intermediate 8 (800 mg, 1.9 mmol) and $K_2CO_3$ (527 mg, 2 eq.) in MeOH (5 mL) at 0 °C. After 30 min at 0 °C, the reaction mixture was allowed to reach room temperature and stirring was continued for 4 h. The reaction mixture was diluted with 10 mL DCM, filtered, and the filtrate was evaporated. The residue was purified via preparative HPLC (Stationary phase: RP XBridge Prep C18 OBD-10 μm,50 × 150mm, Mobile phase: 0.25 % $NH_4HCO_3$ solution in water, $CH_3CN$), yielding Intermediate 37 (500 mg, yield: 63 %).

(2R,3S,E)-N,N-bis(4-methoxybenzyl)-3-methyl-6-(4,4,5,5-tetramethyl-1,3,2-dioxaborolan-2-vl)hex-5-ene-2-sulfonamide Intermediate 38)

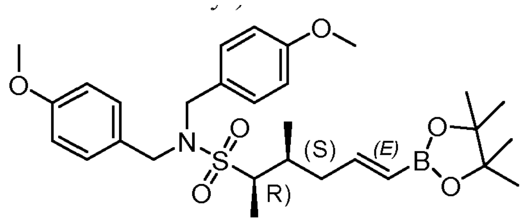

A mixture of bis(pinacolato)diboron (187 mg, 1.5 eq.), copper(I) oxide (19.4 mg, 0.27 eq.), $PPh_3$ (49 mg, 0.38 eq.), potassium phosphate dibasic (171 mg, 2 eq.) and MeOH ( 1.6 mL) was stirred in a tube. The tube was closed and purged with nitrogen for 10 min. Intermediate 37 (200 mg, 0.48 mmol) dissolved in MeOH (1.6 mL) was added. The reaction mixture was stirred at room temperature for 3 h. EtOAc (6 mL) was added. The reaction mixture was stirred for 5 min and was filtered. The filtrate was evaporated and the residue was stirred in DIPE, the solid was filtered off, and the filtrate was evaporated, yielding Intermediate 38 ( 243 mg , yield: 93 %), used without further purification.

Intermediate 51

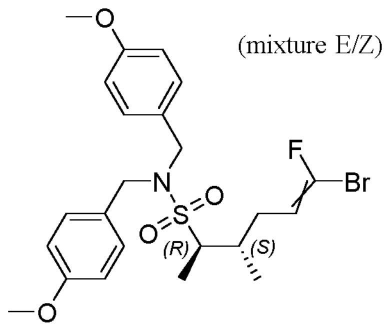

(mixture E/Z)

Diethyl zinc [557-20-0] (27 mL, 1 M in heptane, 1.4 eq.) was added over 4 h via a syringe pump at room temperature to a stirred solution of Intermediate 8 (8 g, 19.07 mmol), fluorotribromomethane [353-54-8] (2.8 mL, 1.5 eq.) and triphenylphosphine [603-35-0] (7.5 g, 1.5 eq.) in dry THF (150 mL). Right after addition, the yellow solution was quenched with MeOH (20 mL) and evaporated under reduced pressure. The residue was purified by column chromatography on silica gel with heptane/EtOAc (1:0 to 4:1) to afford Intermediate 51 (8.4 g, yield: 86 %) as a clear oil (48/52 mixture of Z/E isomers).

Intermediate 52

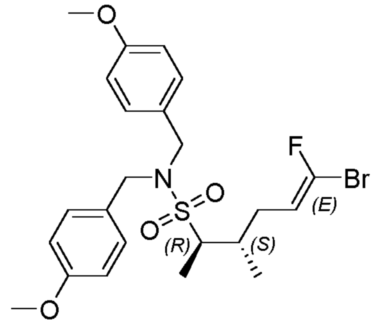

LiHMDS [4039-32-1] (60 mL, 1 M in THF, 1.1 eq.) was added over 14 h via a syringe pump to a cold (0 °C) stirred solution of Intermediate 51 (28.0 g, 54.42 mmol) in dry THF (350 mL). After the addition, the mixture was further stirred for 30 min at 0 °C before quenching with water (100 mL). EtOAc (150 mL) was added. The organic layer was extracted, washed with brine (100 mL), dried (MgSO4), filtered, and evaporated under reduced pressure. The residue was purified by column chromatography on silica gel using heptane/EtOAc (1:0 to 4:1) to afford Intermediate 52 (13.72 g, yield: 49 %) as a clear oil with >99:1 E/Z ratio.

Intermediate 53

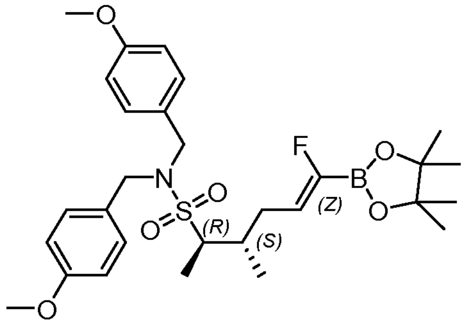

Intermediate 52 (1.55 g, 3.013 mmol) was dissolved in dry 1,4-dioxane (15 mL). This solution was added dropwise over 30min to a degassed solution of bis(pinacolato)diboron [73183-34-3] (3.1 g, 4 eq.), potassium acetate (900 mg, 3 eq.), and 1,1'-bis(diphenylphosphino)ferrocene-Palladium(II) dichloride dichloromethane complex [95464-05-4] (250 mg, 0.1 eq.) in dry 1,4-dioxane (45mL) at 95 °C in a closed vessel. After addition the reaction mixture was stirred at 95 °C for 5 h and then overnight at 25 °C. Dicalite was added to the reaction mixture and it was filtered. The filtrate was concentrated and the residue was taken up in heptane, stirred for 5 min, then filtered. The filter was washed with heptane. The combined filtrate was concentrated and purified by flash chromatography on silica gel using heptane/EtOAc (from 100:0 to 70:30) as eluent, affording Intermediate 53 (1.4 g, yield: 83 %).

methyl (S)-5-(((S)-1-(tert-butoxycarbonyl)azetidin-2-yl)methyl)-6'-chloro-3',4,4',5-tetrahydro-2H,2'H-spiro[benzo[b][1,4]oxazepine-3,1'-naphthalene]-7-carboxylate

(Intermediate 9)

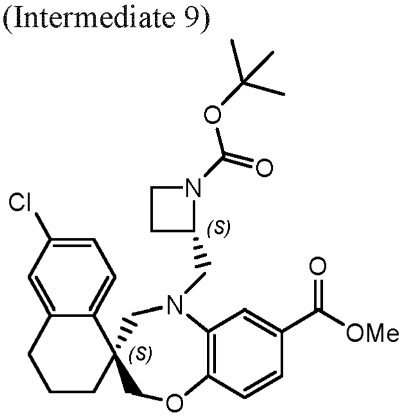

AcOH (12 mL, 25 eq.) was added to a solution of methyl (S)-6'-chloro-3',4,4',5-tetrahydro-2H,2'H-spiro[benzo[b][1,4]oxazepine-3,1'-naphthalene]-7-carboxylate [1883726-85-9] (3 g, 8.384 mmol) and Intermediate 2 (7.82 g, 3 eq.) in dry DCM (50 mL) at room temperature. After being stirred for 1 h at room temperature, the reaction mixture was cooled to 0 °C. Then, sodium triacetoxyborohydride (890 mg, 0.5 eq.) was added in 9 portions, every 30 min. The reaction mixture was quenched with a solution of saturated $NaHCO_3$, and diluted with EtOAc. Layers were separated, and the organic layer was washed with brine, dried with $MgSO_4$, filtered and solvents were removed under reduced pressure. The residue was purified by flash chromatography on silica gel (eluent: EtOAc/Heptane: 0/100 to 30/70) to afford Intermediate 9 (3.6 g, yield 73 %) as a white powder.

methyl (S)-5-(((S)-azetidin-2-yl)methyl)-6'-chloro-3',4,4',5-tetrahydro-2H,2'H-spiro[benzo[b][1,4]oxazepine-3,1'-naphthalene]-7-carboxylate (Intermediate 10)

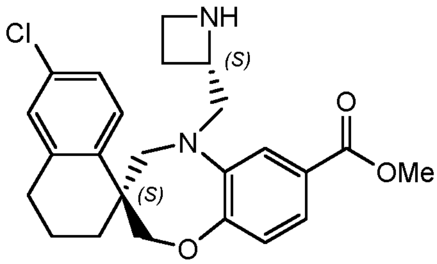

To a solution of Intermediate 9 (1.98 g, 3.494 mmol) in DCM (20 mL) was slowly added TFA (9.5 mL, 35.5 eq.). The reaction mixture was stirred at room temperature for 4 h. The reaction mixture was diluted with DCM and added dropwise to a saturated solution of $NaHCO_3$/ice/DCM. The layers were separated and the aqueous layer was extracted twice with DCM. The combined organic layer was washed with brine, dried with $MgSO_4$, filtered, and solvents were removed under reduced pressure to afford Intermediate 10 (1.23 g, yield 76 %) as a white powder.

methyl (3S)-5-(((2S)-1-(2-(benzo[d]thiazol-2-ylsulfonyl)-2-fluoroethyl)azetidin-2-yl)methyl)-6'-chloro-3',4,4',5-tetrahydro-2H,2'H-spiro[benzo[b][1,4]oxazepine-3,1'-naphthalene]-7-carboxylate (Intermediate 11)

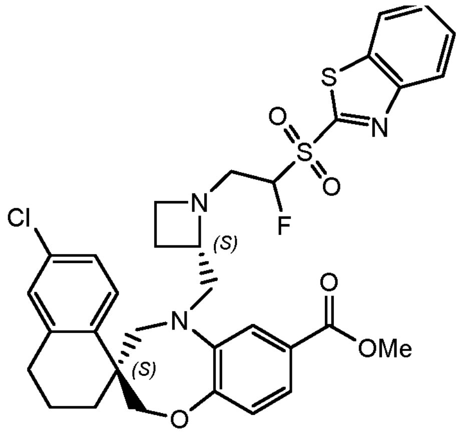

Intermediate 10 (500 mg, 1.113 mmol) and 2-((1-fluorovinyl)sulfonyl)benzo[d]thiazole [1200123-14-3] (324.8 mg, 1.2 eq.) were stirred in dry DCM (20 mL) overnight at room temperature. The reaction mixture was quenched with $NH_4Cl$, and extracted 3 times with DCM. The combined organic layer was washed with brine, dried with $MgSO_4$, and solvents were evaporated under reduced pressure. The residue was purified by flash chromatography on silica gel (eluent: EtOAc/Heptane : 0/100 to 40/60) to afford Intermediate 11 (491 mg, 66 %).

methyl (S)-5-(((S)-1-((SS,6R)-6-(N,N-bis(4-methoxybenzyl)sulfamoyl)-2-fluoro-5-methylhept-2-en-1-yl)azetidin-2-yl)methyl)-6'-chloro-3',4,4',5-tetrahydro-2H,2'H-spiro[benzo[b][1,4]oxazepine-3,1'-naphthalene]-7-carboxylate (mixture E/Z)

(Intermediate 12)

(mixture E/Z)

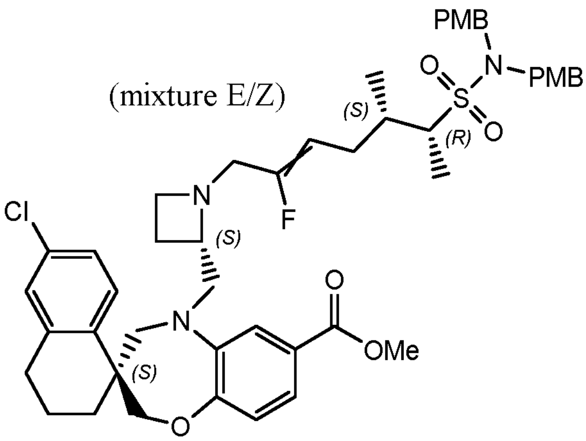

To a solution of Intermediate 11 (381 mg, 0.568 mmol) and Intermediate 8 (397.49 mg, 1.5 eq) in THF (10 mL) at -21 °C under nitrogen atmosphere was slowly added a solution of tBuOK (70.17 mg, 1.1 eq) in THF (5 mL) over 30 min. After being stirred 15 min at -21 °C, the reaction mixture was warmed to room temperature and stirred for 15 min. Additional Intermediate 8 (0.5 eq.) and tBuOK (0.5 eq.) were added and the reaction mixture was stirred for another 30 min. Then, the reaction mixture was cooled to -21 °C and additional tBuOK (1.1 eq.) was added over 30 min. The reaction mixture was quenched with $NH_4Cl$. DCM was added. The layers were separated, and the aqueous layer was extracted with DCM. The combined organic layer was washed with brine, dried with $MgSO_4$, filtered and solvents were removed under reduced pressure. The residue was purified by flash chromatography on silica gel (eluent: EtOAc/Heptane : 0/100 to 40/60) to afford Intermediate 12 (226.5 mg, 42 %) as a white powder.

methyl (S)-6'-chloro-5-(((S)-1-((SS,6R)-2-fluoro-5-methyl-6-sulfamoylhept-2-en-1-yl)azetidin-2-yl)methyl)-3',4,4',5-tetrahydro-2H,2'H-spiro[benzo[b][1,4]oxazepine-3,1'-naphthalene]-7-carboxylate (mixture E/Z) (Intermediate 13)

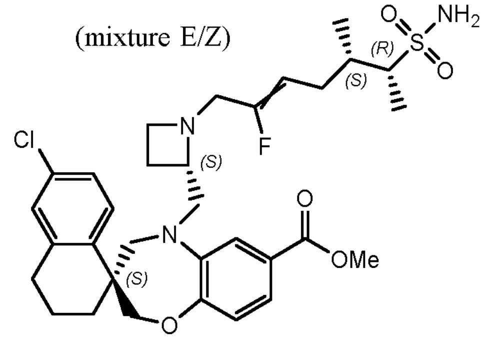

To a solution of Intermediate 12 (226.5 mg, 0.241 mmol) in DCM (7 mL) under nitrogen atmosphere was added TFA (1.84 mL, 100 eq.) dropwise. The reaction mixture was stirred at room temperature for 21 h. The reaction mixture was quenched with saturated aqueous $NaHCO_3$ and diluted with DCM. The aqueous layer was extracted three times with DCM. The combined organic layer was washed with brine, dried with $MgSO_4$, filtered, and solvents were removed under reduced pressure to afford Intermediate 13 (131.9 mg, 57%).

(S)-6'-chloro-5-(((S)-1-((5S,6R)-2-fluoro-5-methyl-6-sulfamoylhept-2-en-1-yl)azetidin-2-yl)methyl)-3',4,4',5-tetrahydro-2H,2'H-spiro[benzo[b][1,4]oxaaepine-3,1'-naphthalene]-7-carboxylic acid (mixture E/Z) (Intermediate 14)

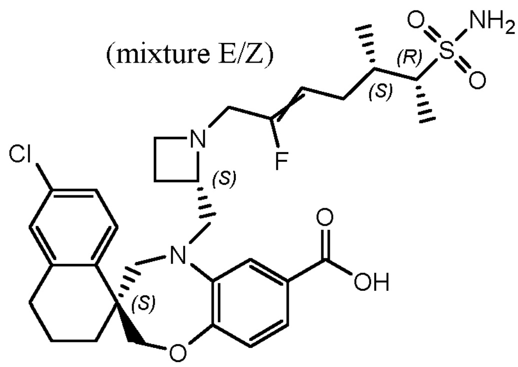

To a solution of Intermediate 13 (131.9 mg, 0.15 mmol) in THF (4 mL) and water (1 mL) was added LiOH (14.34 mg, 4 eq.). The reaction mixture was stirred at room temperature for 16 h followed by 2 h at 50 °C. The solvents were evaporated and the residue was purified by preparative HPLC (Stationary phase: RP XBridge Prep C18 OBD-10 μm, 30x150 mm, Mobile phase: 0.25 % $NH_4HCO_3$ solution in water, $CH_3CN$) to give Intermediate 14 (16 mg, 17 %).

A2) Methyl (S)-5-(((S)-1-allylazetidin-2-yl)methyl)-6'-chloro-3',4,4',5-tetrahydro-2H,2'H-spiro[benzo[b][1,4]oxazepine-3,1'-naphthalene]-7-carboxylate (Intermediate 15)

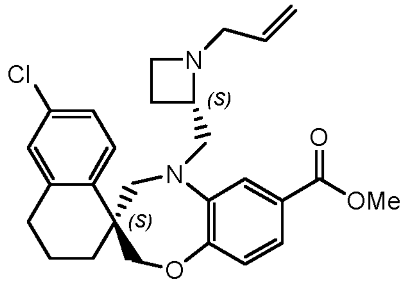

Allyl bromide (65.3 µL, 1.1 eq.) was added to a suspension of Intermediate 10 (333 mg, 0.686 mmol) and $Cs_2CO_3$ (1.12 g, 5 eq.) in dry $CH_3CN$ (40 mL) at room temperature. The reaction mixture was stirred at room temperature for 24 h. The solvent was evaporated. The residue was partitioned between water and EtOAc and the layers were separated. The aqueous layer was extracted again with EtOAc. The combined organic layer was dried on $MgSO_4$, filtered, and evaporated. The residue was purified by column chromatography (SNAP Ultra 25 g; eluent: AcOEt/EtOH 3/1: heptane 0:100 -> 60:40) to give Intermediate 15 (212 mg, 51 %) as a colorless solid, dried under vacuum at 45 °C.

(S)-5-(((S)-1-allylazetidin-2-yl)methyl)-6'-chloro-3',4,4',5-tetrahydro-2H,2'H-spiro[benzo[b][1,4]oxazepine-3,1'-naphthalene]-7-carboxylic acid (Intermediate 16)

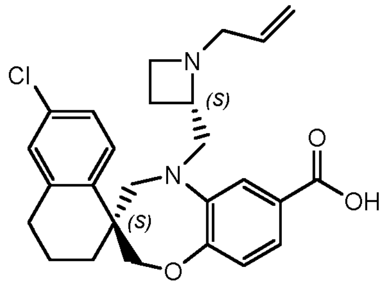

LiOH (25.11 mg, 3 eq.) was added to a solution of Intermediate 15 (212 mg, 0.35 mmol) in water, distilled (5 mL) and THF (15 mL). The mixture was stirred at room temperature overnight and then at 60 °C for 24 h. The solvents were evaporated under reduced pressure. A purification was performed via Prep HPLC (Stationary phase: RP XBridge Prep C18 OBD-10µm, 50x150mm, Mobile phase: 0.25 % $NH_4HCO_3$ solution in water, $CH_3CN$) to afford Intermediate 16 (104 mg, 66 %) as a white solid after evaporation of the solvents.

(S)-5-(((S)-1-allylazetidin-2-yl)methyl)-6'-chloro-N-(((2R,3S)-3-methylhex-5-en-2-yl)sulfonyl)-3',4,4',5-tetrahydro-2H,2'H-spiro[benzo[b][1,4]oxazepine-3,1'-naphthalene]-7-carboxamide (Intermediate 17)

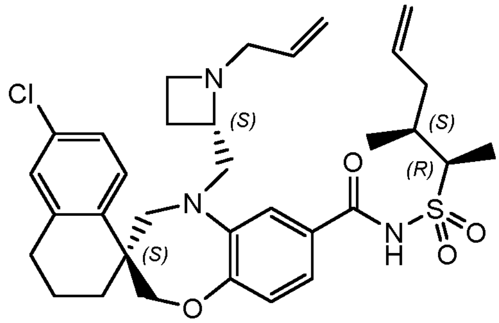

DMAP (47.68 mg, 1.7 eq.) followed by EDC.HCl (88.03 mg, 2 eq.) were added to a solution of Intermediate 16

(104 mg, 0.23 mmol) and Intermediate 6 (101.75 mg, 2.5 eq.) in dry DCM (5 mL) at room temperature. The reaction mixture was stirred at room temperature overnight. 3 mL saturated aqueous $NH_4Cl$ was added to the reaction mixture and the mixture was stirred for a few minutes. The layers were separated and the aqueous layer was extracted again with DCM. The combined organic layer was dried by filtration on Extrelut NT3 and was evaporated. A purification was performed via Prep HPLC (Stationary phase: RP XBridge Prep C18 OBD-10μm, 50x150mm, Mobile phase: 0.25 % $NH_4HCO_3$ solution in water, $CH_3CN$) to afford Intermediate 17 (88 mg, 54 %) as an off-white solid after evaporation of the solvents at 60 °C.

A3) (6-chloro-1-((4-iodo-2-nitrophenoxy)methyl)-1,2,3,4-tetrahydronaphthalen-1-yl)methanol (Intermediate 18)

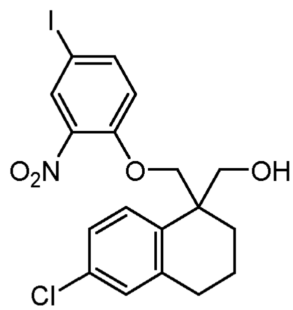

This reaction was performed in two batches. For each batch, (6-chloro-1,2,3,4-tetrahydronaphthalene-1,1-diyl)dimethanol [1883726-74-6] (300 g, 1.32 mol) and 1-fluoro-4-iodo-2-nitrobenzene (353 g, 1.32 mol) were dissolved in acetonitrile (1.4 L). $K_2CO_3$ (549 g, 3.97 mol) was added to the reaction mixture and it was stirred at 50 °C for 16 h. The reaction mixture was filtered and the filtrate was evaporated. The two batches were then combined and the residue was purified by column chromatography ($SiO_2$, Petroleum ether: Dichloromethane=3/1 to Petroleum ether/EtOAc=1:1). Intermediate 18 was obtained as a yellow oil (600 g, 48 % yield).

6-chloro-1-((4-iodo-2-nitrophenoxy)methyl)-1,2,3,4-tetrahydronaphthalene-1-carbaldehyde Intermediate 19

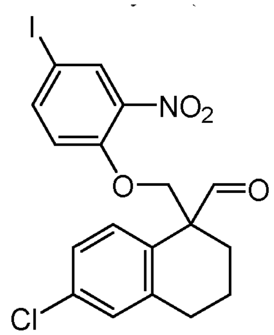

This reaction was performed in three batches. For each batch, DMSO (99.0 g, 1.27 mol) was added to a solution of $(COCl)_2$ (161 g, 1.27 mol) in DCM (2.4 L) at -78 °C. The reaction mixture was stirred at -78 °C for 15 min. Intermediate 18 (200 g, 422 mmol) in DCM (0.90 L) was then added at -78 °C and stirring was continued for 30 min. at -78 °C. $Et_3N$ (214 g, 2.11 mol) was added at -78°C and the reaction mixture was allowed to warm to room temperature. Stirring was continued at room temperature for 1.5 h. Aqueous $NaHCO_3$ (1 L) was added and the mixture was extracted with DCM (0.5 L × 2). The three batches were then combined and evaporated to yield Intermediate 19 (560 g) as a yellow solid, used without further purification.

(S)-6'-chloro-7-iodo-3',4,4',5-tetrahydro-2H,2'H-spiro[benzo[b][1,4]oxazepine-3,1'-naphthalene] (Intermediate 20) (and its enantiomer)

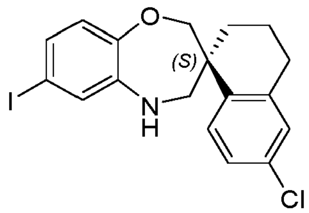

Intermediate 20

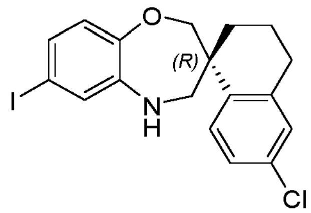

Enantiomer of Intermediate 20

This reaction was performed in three batches. Iron (153 g, 2.75 mol) was added to a solution of Intermediate 19 (185 g, 392 mmol) in AcOH (2.5 L) at 70 °C and the reaction mixture was stirred at 70 °C for 3 h. The solvent was evaporated and DCE (1.9 L) was added to the residue. $NaBH(OAc)_3$ (333 g, 1.57 mol) was then added portionwise at 0°C. Stirring was continued at room temperature for 1 h. The three batches were combined. Citric acid (10 % solution in water, 5. L) was added and the mixture was extracted with DCM (2 L × 2). The combined organic layer was evaporated. The residue was purified by SFC (column: DAICEL CHIRALPAK AD (250 mm*50 mm, 10 um); mobile phase: [0.1 % $NH_3H_2O$ in EtOH]; B %: 50 %-50 %, 8.5 min) to afford Intermediate 20 (95.2 g, 40 % yield) and its enantiomer (105.1 g, 44 % yield), both as yellow solids.

tert-butyl (S)-2-(((S)-6'-chloro-7-iodo-3',4'-dihydro-2H,2'H-spiro[benzo[b][1,4]oxazepine-3,1'-naphthalen]-5(4H)-yl)methyl)azetidine-1-carboxylate (Intermediate 21)

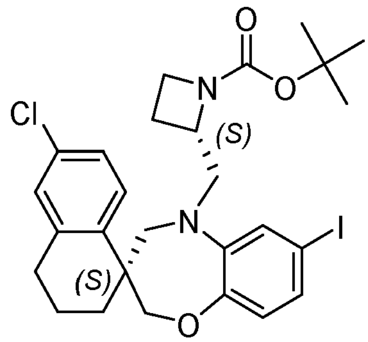

A mixture of Intermediate 20 (7.58 g, 17.8 mmol) and Intermediate 2 (16.25 g, 53.4 mmol) was dissolved in DCM (75 mL) and AcOH (25 mL) was added. The mixture was stirred for 30 minutes at room temperature and then cooled to 0 °C. Sodium triacetoxyborohydride (11.3 g, 53.4 mmol) was added portionwise. After addition, the reaction mixture was stirred at room temperature for 16 hours. The reaction mixture was poured portionwise into a cooled (0 °C) solution of NaOH (21.3 g, 53.4 mmol) in 300 mL water. After addition the mixture was diluted with DCM and water. The organic layer was separated, washed with water, dried with $MgSO_4$, filtered, and the solvents of the filtrate were evaporated under reduced pressure. The residue was dissolved in DCM and purified by flash chromatography over silica (eluent: dichloromethane). The fractions containing product were combined and the solvents were evaporated yielding Intermediate 21 (9.8 g, yield 92 %)

tert-butyl (2S)-2-[[(3S)-6'-chloro-7-iodo-spiro[2,4-dihydro-1,5-benzoxazepine-3,1'-tetralin]-5-yl]methyl]pyrrolidine-1-carboxylate (Intermediate 39)

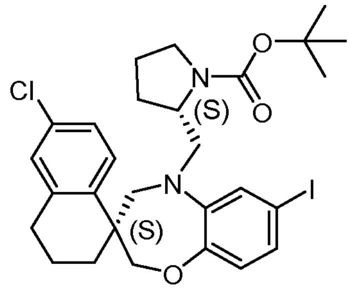

Intermediate 20 (13.197 g, 31 mmol) and N-Boc-L-prolinal (18.53 g, 3 eq.) were dissolved in $CH_2Cl_2$ (150 mL) and then AcOH (35.5 mL, 20 eq.) was added. The mixture was stirred for 30 min at room temperature and then cooled down to 0 °C. Then sodium triacetoxyborohydride (19.711 g, 3 eq.) was added portionwise. After addition the reaction mixture was stirred at room temperature for 18 hours. The reaction mixture was poured out portionwise into a cooled (0 °C) solution of NaOH (31 g) in 620 mL water. After addition the mixture was diluted with $CH_2Cl_2$ and water. The organic layer was separated, washed with water, dried with $MgSO_4$, filtered and the solvents of the filtrate were evaporated. The residue was purified by flash chromatography on silica gel (eluent: $CH_2Cl_2$) The fractions containing product were combined and the solvents were evaporated. This residue was purified again by HPLC (gradient ethylacetate/hexane) to give Intermediate 39 (12.2 g, yield: 64 %).

(S)-5-(((S)-azetidin-2-yl)methyl)-6'-chloro-7-iodo-3',4,4',5-tetrahydro-2H,2'H-spiro[benzo[b][1,4]oxazepine-3,1'-naphthalene] (Intermediate 22)

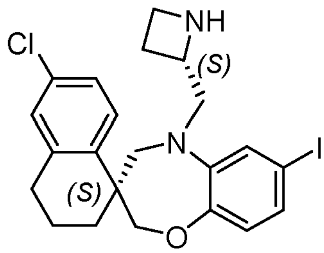

A mixture of Intermediate 21 (9.8 g, 16.5 mmol) and DCM (125 mL) was stirred at room temperature. TFA (125 ml) was added dropwise. The reaction mixture was stirred at room temperature for 3 hours and was then evaporated to dryness keeping the temperature below 30 °C. The residue was taken up into DCM and was poured into an aqueous NaHCOs solution. The layers were separated and the organic layer was extracted with DCM. The combined organic fractions were washed with water, dried with $MgSO_4$, and evaporated. The residue was purified by flash chromatography on silica (eluent: DCM-MeOH/$NH_3$ gradient 100 %/0 % to 95 % /5 %) The pure fractions were collected and evaporated. The residual oil was stirred in $CH_3CN$ until precipitation occurred. The precipitate was filtered off and dried yielding Intermediate 22 (5.1 g, yield 63 %).

Intermediate 40 was prepared by an analogous reaction protocol as Intermediate 22 using the appropriate starting materials:

| Intermediate | Structure | *Starting material* |
|---|---|---|
| Intermediate 40 | 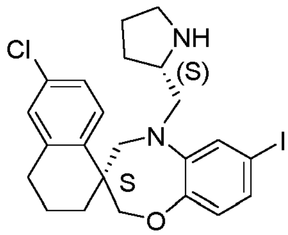 | Intermediate 39 |

methyl 2-((S)-2-(((S)-6'-chloro-7-iodo-3',4'-dihydro-2H,2'H-spiro[benzo[b][1,4]oxazepine-3,1'-naphthalen]-5(4H)-yl)methyl)azetidin-1-yl)-3-methoxypropanoate Intermediate 23

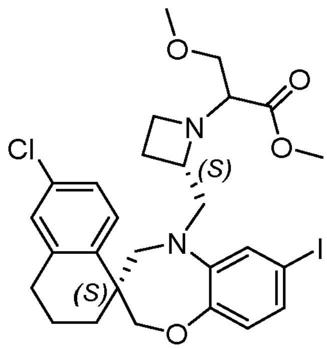

A mixture of Intermediate 22 (2.47 g, 5 mmol), methyl 2-bromo-3-methoxypropanoate (1.08 g, 5.5 mmol), DIPEA (1.29 g, 10 mmol) and DMF (29 mL) were stirred at room temperature for 72 hours. Again, 1.25 mmol of methyl 2-bromo-3-methoxypropanoate was added. The reaction mixture was stirred at room temperature for another 48 hours. The reaction mixture was poured into a solution of $Na_2CO_3$ (1.1 g, 10 mmol) in water (100 mL) and the mixture was extracted with EtOAc. The organic layer was washed with water, dried with $MgSO_4$, and evaporated. The residue was purified by flash chromatography on silica (eluent: 99 % DCM/1 % MeOH), yielding Intermediate 23 (3.1 g, yield 100 %).

Intermediate 41 was prepared by an analogous reaction protocol as Intermediate 23 using the appropriate starting materials:

| **Intermediate** | **Structure** | ***Starting material*** |
|---|---|---|
| Intermediate 41 | 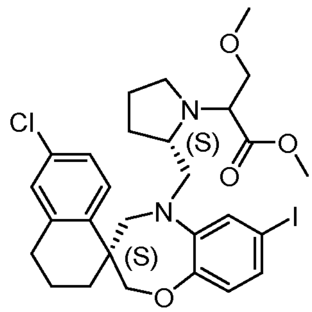 | Intermediate 40 |

2-((S)-2-(((S)-6'-chloro-7-iodo-3',4'-dihydro-2H,2'H-spiro[benzo[b][1,4]oxazepine-3,1'-naphthalen]-5(4H)-yl)methyl)azetidin-1-yl)-3-methoxypropan-1-ol (Intermediate 24)

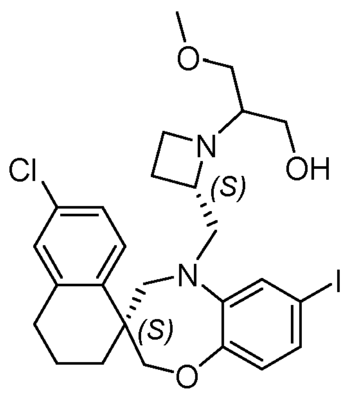

Intermediate 23 (3.05 g, 5 mmol) was dissolved in dry THF (50 mL) and cooled to -78 °C. The solution was stirred under nitrogen flow. Then DIBAH 1M in THF (20 mL, 20 mmol) was added dropwise at -78 °C. The reaction mixture was then stirred at room temperature for 3 hours. The reaction mixture was cooled on an ice bath and 25 mL MeOH were added dropwise; keeping the temperature below 10 °C. The reaction mixture was stirred for 30 min. Then water was added and the reaction mixture was extracted 3 times with DCM. The organic layer was dried with $MgSO_4$ and evaporated, yielding Intermediate 24 (2.6 g, yield 89 %), used without further purification.

Intermediate 42 was prepared by an analogous reaction protocol as Intermediate 24 using the appropriate starting materials:

| **Intermediate** | **Structure** | ***Starting material*** |
|---|---|---|
| Intermediate 42 | 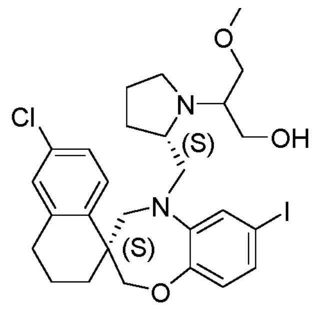 | Intermediate 41 |

2-((S)-2-(((S)-6'-chloro-7-iodo-3',4'-dihydro-2H,2'H-spiro[benzo[b][1,4]oxazepine-3,1'-naphthalen]-5(4H)-yl)methyl)azetidin-1-yl)-3-methoxypropanal Intermediate 25

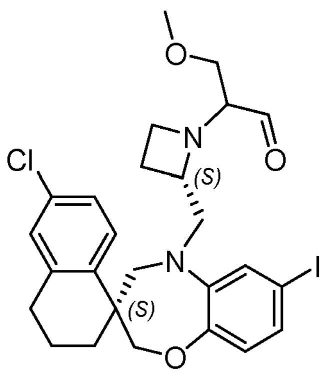

Dry DMSO (0.479 mL, 6.69 mmol) in dry DCM (15 mL) was stirred at -70 °C under nitrogen flow.

Oxalyl chloride (1.67 mL, 3.34 mmol) was added dropwise at -70 °C. The reaction mixture was stirred for 30 min at -70 °C. Intermediate 24 (1.3 g, 2.23 mmol) in 10 mL DCM was then added dropwise at -70 °C. The reaction mixture was stirred for 2 h at -70 °C. Then $Et_3N$ (1.86 mL, 13.38 mmol) in DCM was added dropwise at -70 °C. The reaction mixture was stirred at -70 °C for 1 h. Water was then added dropwise at -20 °C. The reaction mixture was warmed to room temperature and NaHCOs (562 mg, 6.69 mmol) in water (20 mL) was added. The reaction mixture was extracted with DCM. The organic layer was washed with water, dried with $MgSO_4$, and evaporated yielding Intermediate 25 (1.29 g, quantitative) which was used without further purification.

Intermediate 43 was prepared by an analogous reaction protocol as Intermediate 25 using the appropriate starting materials:

| Intermediate | Structure | *Starting material* |
|---|---|---|
| Intermediate 43 | 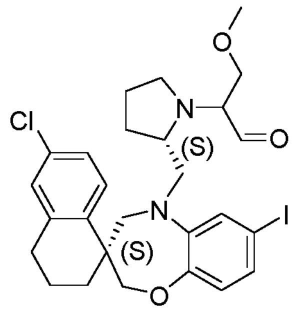 | Intermediate 42 |

Intermediate 26 and Intermediate 27

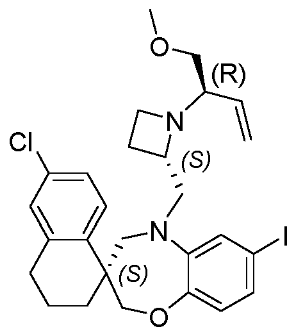

Intermediate 26

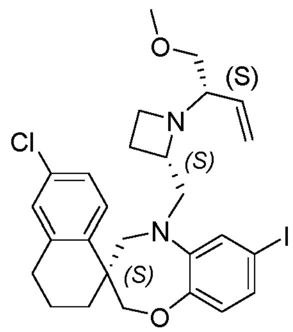

Intermediate 27

To a mixture of $Ph_3PCH_3Br$ (2.63 g, 7.36 mmol) in dry THF (50 mL) was added dropwise KOtBu 1 M in THF (6.69 mL, 6.69 mmol) at 0 °C under nitrogen atmosphere. The mixture was stirred at 0 °C for 1 h.

Intermediate 25 (1.29 g, 2.23 mmol) dissolved in 25 mL dry THF was then added dropwise. Another 25 mL of dry THF was added and the reaction mixture was stirred at room temperature for 16 h.

A saturated aqueous $NH_4Cl$ solution was added and the reaction mixture was stirred for 10 min before extraction with EtOAc. The organic layer was dried with $MgSO_4$, and evaporated to dryness. The residue was purified by flash chromatography on silica (eluent: DCM 99 %/MeOH 1 %) yielding a racemic mixture of Intermediates 26 and 27. This mixture was purified via preparative SFC (Stationary phase: Chiralpak Daicel IG 20 × 250 mm, Mobile phase: $CO_2$, EtOH + 0.4 % $iPrNH_2$), yielding Intermediate 26 (383 mg, yield 30 %) and Intermediate 27 (230 mg, yield 18 %).

Intermediate 44 was prepared by an analogous reaction protocol as Intermediates 26 and 27 using the appropriate starting materials:

| Intermediate | Structure | *Starting material* |
|---|---|---|
| Intermediate 44 | 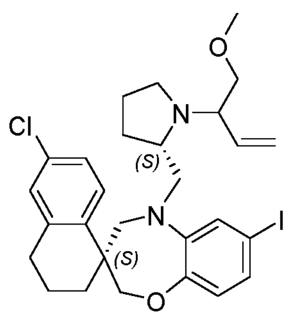 | Intermediate 43 |

Intermediate 29

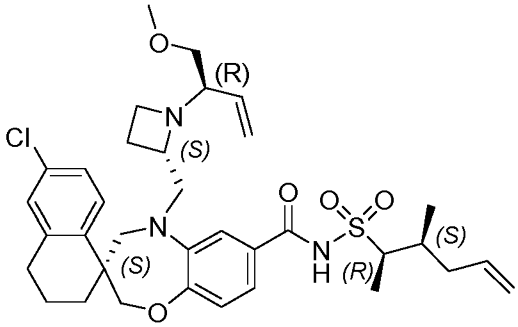

A stainless steel reactor was filled with Intermediate 26 (326 mg, 0.563 mmol), Intermediate 6 (150 mg, 1.5 eq.), $PdCl_2$(dppf) (41 mg, 0.1 eq.), DBU (171 mg, 2 eq.) in dry THF (20 mL). The reactor was sealed and pressurized with 50 bar of CO gas. The mixture was stirred at 100 °C for 16 h. The reaction mixture was cooled and the solvent was evaporated. The residue was poured into water and extracted with DCM. The organic layer was dried with $MgSO_4$ and evaporated. The residue was purified by preparative HPLC (Stationary phase: RP XBridge Prep C18 OBD-10 μm, 50x150 mm, Mobile phase: 0.25 % $NH_4HCO_3$ solution in water, $CH_3CN$) yielding Intermediate 29 (110 mg, yield 30 %).

Intermediate 45 and 46 were prepared by an analogous reaction protocol as Intermediate 29 using the appropriate starting materials:

| Intermediate | Structure | *Starting material* |
|---|---|---|
| Intermediate 45 | 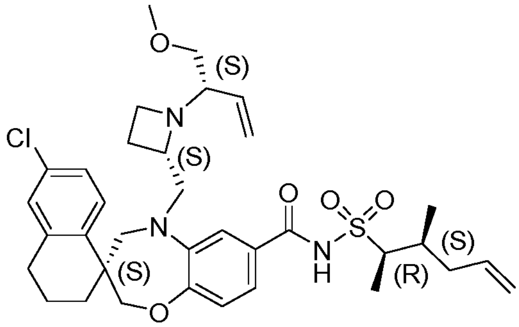 | Intermediate 27 |
| Intermediate 46 | 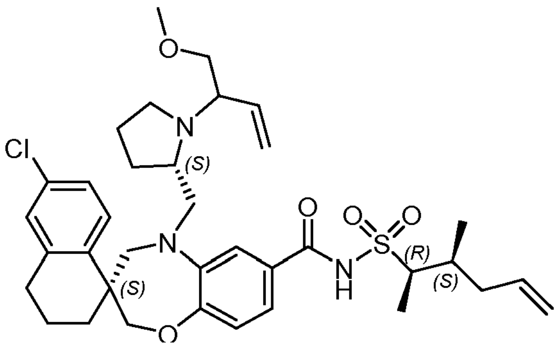 | Intermediate 44 |

A4) Tert-butyl 4-((7-chloro-4-(hydroxymethyl)chroman-4-yl)methoxy)-3-nitrobenzoate

(Intermediate 30)

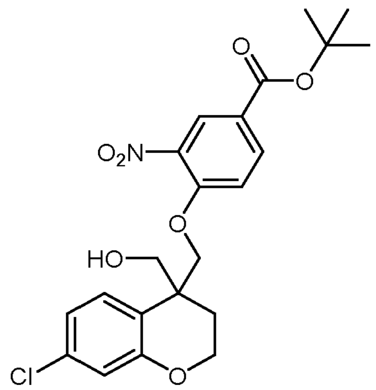

To a stirred solution of (7-chlorochromane-4,4-diyl)dimethanol [2130069-84-8] (600mg, 2.62 mmol) in dry THF (60 mL) was added tert-butyl 4-fluoro-3-nitrobenzoate (470 mg, 0.9 eq). After 5 min, LiHMDS (1.06 M in THF) (2.228 mL, 0.9 eq) was added dropwise at room temperature over 2 h and then left to stir further for 30 min. The reaction mixture was cooled to 0 °C, quenched with aqueous $NH_4Cl$ and extracted with EtOAc. The combined organic layer was washed with aqueous $NH_4Cl$ and brine, dried over $Na_2SO_4$, and concentrated under vacuum. The residue was purified by flash chromatography on silica (0 to 40 % EtOAc in heptane) to afford Intermediate 30 (630 mg, yield 54 %).

tert-butyl 4-((7-chloro-4-formylchroman-4-yl)methoxy)-3-nitrobenzoate (Intermediate 31

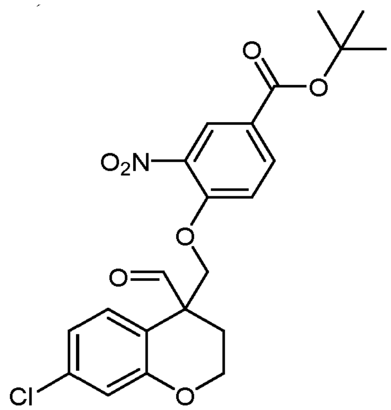

Intermediate 30 (680 mg, 1.667 mmol) and Dess-Martin periodinane [87413-09-0] (1 g, 1.41 eq) were stirred in dry DCM (20 mL) for 30 min at room temperature. The reaction was quenched by addition of aqueous saturated $NaHCO_3$. The layers were separated and the aqueous layer was extracted with EtOAc. The combined organic layer was washed with aqueous NaHCOs and brine, dried over $Na_2SO_4$, and concentrated under vacuum to give Intermediate 31 (676 mg, quantitative), used without further purification.

methyl 7'-chloro-4,5-dihydro-2H-spiro[benzo[b][1,4]oxazepine-3,4'-chromane]-7-carboxylate (Intermediate 32)

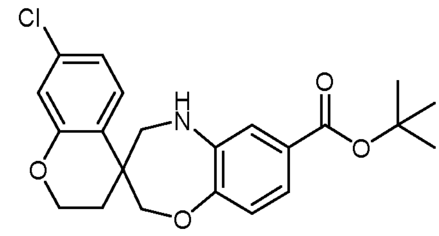

A solution of Intermediate 31 (676 mg, 1.66 mmol) in AcOH (20 mL) was heated to 70 °C. Iron powder (605 mg, 6.5 eq) was added and the mixture was stirred for 6 h at 70 °C. The solvent was then removed under vacuum. The residue was dissolved in DCE (15 mL) and $NaBH_4$ (25 mg, 0.4 eq) was added in 0.1 eq portions at 0 °C. The reaction mixture was stirred at room temperature for 1 h. The reaction mixture was quenched with water and extracted with DCM. The combined organic layer was washed with brine, dried over $Na_2SO_4$, and concentrated under reduced pressure. The residue was purified by flash chromatography on silica (eluent: 0 to 30 % EtOAc in heptane) to yield Intermediate 32 (420 mg, yield 64 %).

methyl 5-(((S)-azetidin-2-yl)methyl)-7'-chloro-4,5-dihydro-2H-spiro[benzo[b][1,4]oxazepine-3,4'-chromane]-7-carboxylate (Intermediate 33)

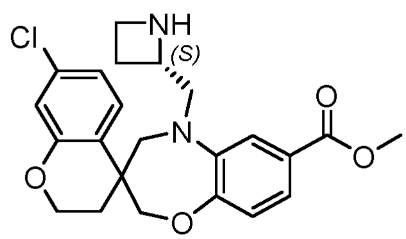

Intermediate 32 (700 mg, 1.79 mmol) and Intermediate 1 (1.658 g, 5 eq) were stirred in DCE (23mL) and AcOH (11 mL) at room temperature for 1 h. The reaction mixture was cooled to 0 °C and stirred at this temperature for 1 h. Sodium cyanoborohydride (49 mg, 0.4 eq) was added and the reaction mixture was stirred at 0 °C for 15 min. Additional sodium cyanoborohydride was added portionwise until completion of the reaction. The reaction was quenched by addition of saturated aqueous $NaHCO_3$ and DCM was added. The layers were separated and the aqueous layer was extracted twice with DCM. The combined organic layer was washed with $NaHCO_3$, dried on $MgSO_4$, filtered, and evaporated. The residue was dissolved in TFA (5 mL) and DCM (5 mL) and left to stir at room temperature until full deprotection. The reaction mixture was then slowly poured into a cold aqueous $NaHCO_3$/DCM mixture, and was stirred overnight. The layers were separated and the aqueous layer was extracted with DCM.

The combined organic layer was washed with brine, dried on $Na_2SO_4$, and concentrated. The residue was purified by flash chromatography on silica (eluent: 0 to 10 % MeOH in DCM) to yield Intermediate 33 (540 mg, yield 70 %).

methyl 5-(((S)-1-allylazetidin-2-yl)methyl)-7'-chloro-4,5-dihydro-2H-spiro[benzo[b][1,4]oxazepine-3,4'-chromane]-7-carboxylate (Intermediate 34)

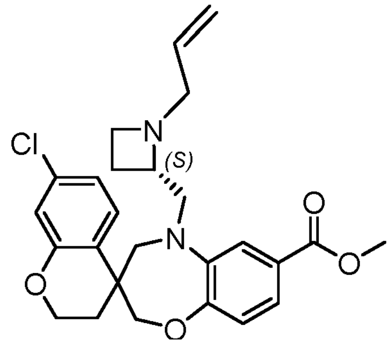

Allyl bromide (101 μL, 2 eq) was added to a suspension of Intermediate 33 (250 mg, 0.58 mmol) and $Cs_2CO_3$ (949 mg, 5 eq) in dry acetonitrile (3.75 mL) at room temperature.

The reaction mixture was stirred at room temperature for 1h. The reaction mixture was filtered and the residue was rinsed with EtOAc. The solvents of the filtrate were evaporated to afford Intermediate 34 (303 mg, quantitative), used without further purification.

5-(((S)-1-allylazetidin-2-yl)methyl)-7'-chloro-4,5-dihydro-2H-spiro[benzo[b][1,4]oxazepine-3,4'-chromane]-7-carboxylic acid (Intermediate 35)

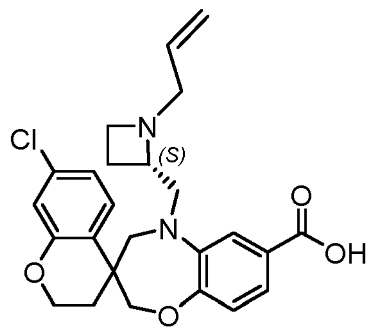

LiOH (50 mg, 3.2 eq) was added to a solution of Intermediate 34 (300 mg, 0.64 mmol) in water (1.5 mL) and THF (5 mL). The reaction mixture was stirred at 70 °C for 18 h. The solvent was evaporated under vacuum to yield Intermediate 35 (330 mg, yield 79 %, 70 % pure), used without further purification.

5-(((S)-1-allylazetidin-2-yl)methyl)-7'-chloro-N-(((2R,3S)-3-methylhex-5-en-2-yl)sulfonyl)-4,5-dihydro-2H-spiro[benzo[b][1,4]oxazepine-3,4'-chromane]-7-carboxamide (Intermediate 36)

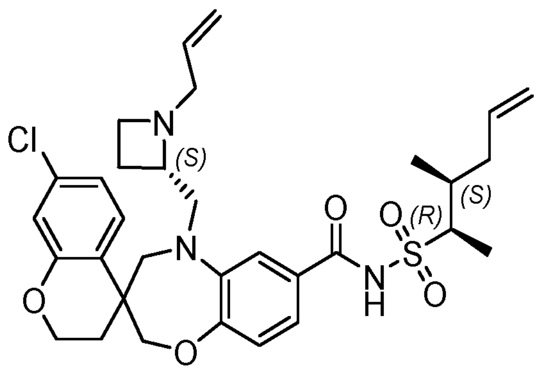

DMAP (105 mg, 1.7 eq) and 1-(3-dimethylaminopropyl)-3-ethylcarbodiimide hydrochloride (194 mg, 2 eq) were

added to a solution of Intermediate 35 (330 mg, 508 mmol, 70 % pure) and Intermediate 6 (225 mg, 2.5 eq) in dry DCM (5 mL) at room temperature. The reaction mixture was stirred at room temperature overnight. Saturated aqueous $NH_4Cl$ (3 mL) was added to the reaction mixture and it was stirred for a few minutes. The layers were separated and the aqueous layer was extracted again with DCM. The combined organic layer was dried with $Na_2SO_4$, filtered, and evaporated. The residue was purified by flash chromatography on silica (0 to 4 % MeOH in DCM) to afford Intermediate 36 (110 mg, yield 35 %).

Intermediate 47 and its diastereoisomer intermediate 47b

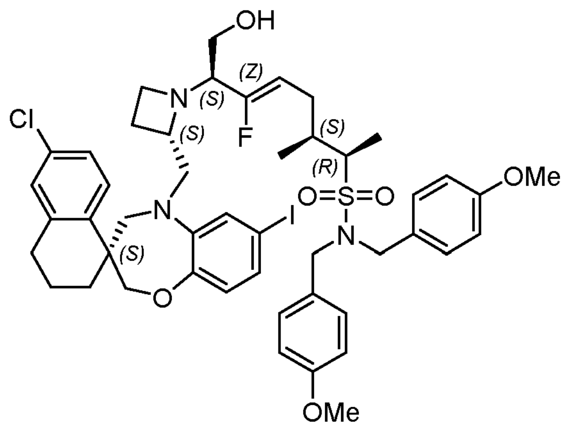

Intermediate 47

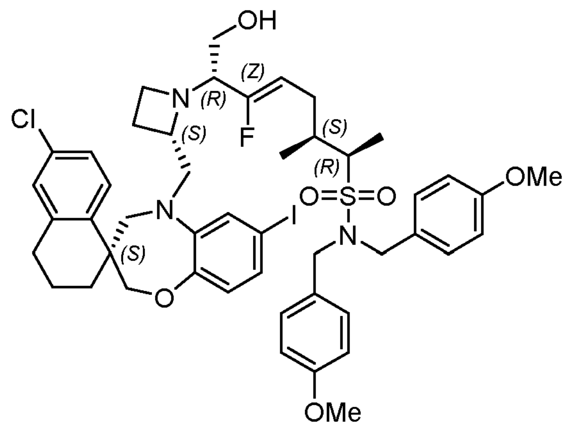

Intermediate 47b

Intermediate 22 (1.14 g, 2.31 mmol), Intermediate 53 (1.3 g, 1 eq.) and 1,4-dioxane-2,5-diol [23147-58-2] (595 mg, 2.1 eq.) were dissolved in MeOH (50 mL) and DCM (5 mL). The resulting mixture was heated at 50 °C for 3 h. After cooling to room temperature, solvents were removed under reduced pressure. The crude was purified by column chromatography on silica gel using heptane/EtOAc (1:0 to 9:1) to afford Intermediate 47 (1.69 g, yield: 75 %) and its diastereoisomer Intermediate 47b (150 mg, yield: 7 %) as off-white foams.

Intermediate 48 and intermediate 54 were prepared by an analogous reaction protocol as Intermediate 47 using the appropriate starting materials:

| Intermediate | Structure | *Starting material* |
|---|---|---|
| Intermediate 48 | 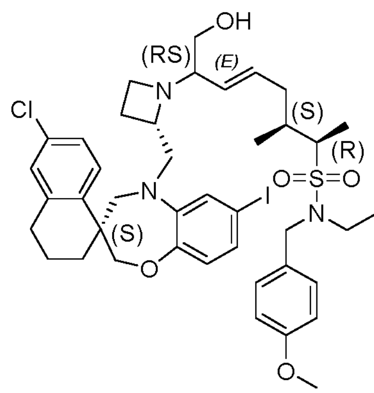 | Intermediate 22 and Intermediate 38 |

(continued)

| Intermediate | Structure | *Starting material* |
|---|---|---|
| Intermediate 54 | 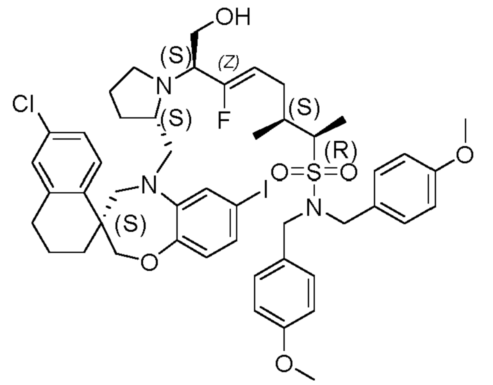 | Intermediate 40 and Intermediate 53 |

Intermediate 49

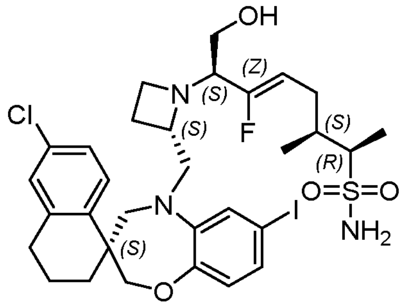

TFA (10 mL, 326 eq.) was added dropwise at 0 °C to a stirred solution of Intermediate 47 (389 mg, 0.4 mmol) and crushed 3 Å molecular sieves in dry DCM (90 mL). The mixture was allowed to warm to room temperature and was stirred for 10 h. Most of the TFA was removed under reduced pressure at room temperature. DCM was added. The mixture was filtered over celite. The filter pad was washed with DCM and the combined filtrate was poured into a saturated aqueous NaHCOs solution (50 mL). The organic layer was extracted and the aqueous layer was back-extracted with DCM. The combined organic layer was dried ($MgSO_4$), filtered, and evaporated under reduced pressure. The residue was purified by column chromatography on silica gel using heptane/EtOAc (1:4) to afford Intermediate 49 (241 mg, yield: 82 %) as an off-white foam.

The following intermediates were prepared by an analogous reaction protocol as Intermediate 49 using the appropriate starting materials:

| Intermediate | Structure | *Starting material* |
|---|---|---|
| Intermediate 50 | 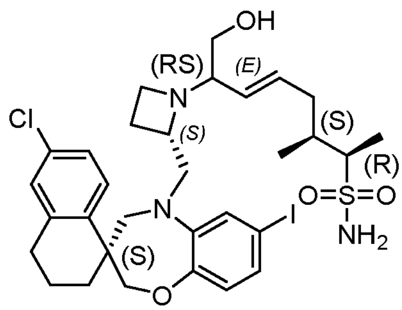 | Intermediate 48 |

(continued)

| Intermediate | Structure | *Starting material* |
|---|---|---|
| Intermediate 55 | 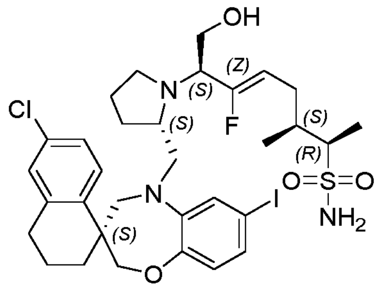 | Intermediate 54 |

Intermediate 56

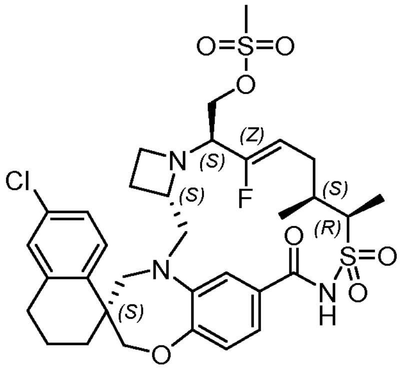

MsCl (13 μL, 0.167 mmol, 1.85 eq.) was added to a solution of Compound 9 (76 mg, 0.090 mmol) and $Et_3N$ (39 μL, 0.279 mmol) in dry DCM (10 mL) cooled to 0 °C. The resulting mixture was stirred at room temperature for 2 h. The reaction mixture was diluted with DCM (10 mL) and saturated aqueous NaHCOs (20 mL). The organic layer was separated, washed with brine (10 mL), dried ($MgSO_4$), filtered, and evaporated to afford Intermediate 56 (74 mg, yield: quantitative) as a yellow oil, used without further purification.

Intermediate 57

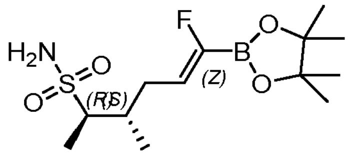

TFA (60 mL, 784 mmol, 28.4 eq.) was added dropwise to a stirred solution of Intermediate 53 (15.5 g, 27.6 mmol) in dry DCM (140 mL) and molecular sieves (15 g). The resulting mixture was stirred at room temperature overnight. The reaction mixture was filtered over celite and the filter pad was washed with DCM. The filtrate was concentrated under reduced pressure and coevaporated 5 times with toluene (10 mL) to afford Intermediate 57 (9.1 g, yield: 82 %) as a yellow solid, used without further purification.

Intermediate 58

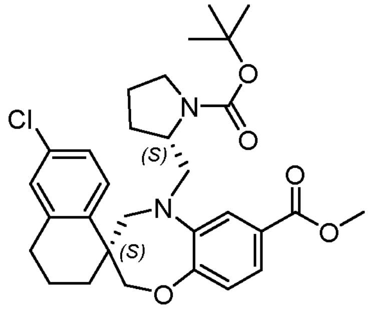

An autoclave was charged with Intermediate 39 (6.7 g, 0.011 mol), MeOH (940 mL), $Et_3N$ (4.87 mL, 0.0351 mol, 3.2 eq.), and THF (174 mL). Then [1,1'-bis(diphenylphosphino)ferrocene]dichloropalladium(II) (CAS [72287-26-4]) (1.183 g, 0.00162 mol, 0.15 eq.) was added and the setup was closed. This was flushed once with carbon monoxide and then pressurised with carbon monoxide (308 mg, 0.011 mol, 1 eq., 30 bar). The reaction mixture was heated at 100 °C for 20 h. The reaction mixture was concentrated and the residue was purified twice by chromatography on silicagel (eluent: DCM/MeOH 100/0 to 98/2) to give Intermediate 58 (5.25 g, yield: 88 %).

Intermediate 59

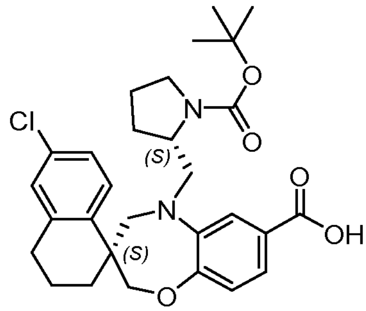

LiOH (1.125 g, 46.98 mmol, 4.8 eq.) was added to a solution of Intermediate 58 (5.25 g, 9.703 mmol) in THF (158 mL), water (158 mL), and MeOH (30 mL). The reaction mixture was stirred at 50 °C for 16 h. The reaction mixture was cooled to 10 °C, acidified to pH = 3-4 with 1 N aqueous HCl, and extracted with DCM. The organic layer was dried over $MgSO_4$, filtered, and evaporated to give Intermediate 59 (5.34 g, yield: quantitative) as an off-white foam.

Intermediate 60

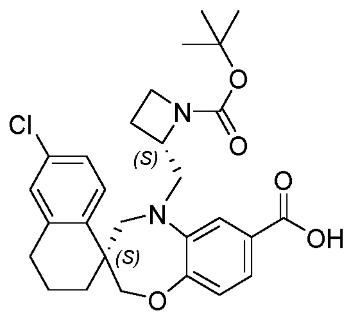

Intermediate 60 was prepared by an analogous reaction protocol as Intermediate 59, starting from Intermediate 9 instead of Intermediate 58.

Intermediate 61

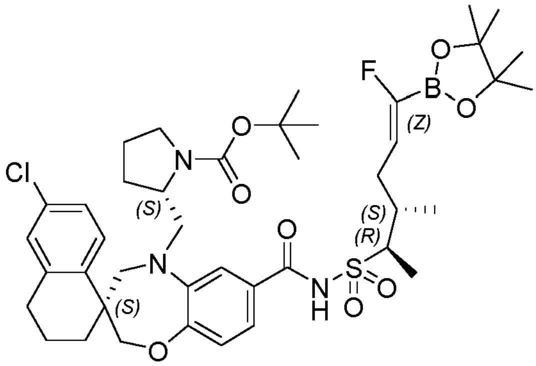

EDCI (600 mg, 3.13 mmol, 2.46 eq.) was added to a mixture of Intermediate 59 (670 mg, 1.27 mmol), Intermediate 57 (480 mg, 1.49 mmol, 1.17 eq.), DMAP (350 mg, 2.87 mmol, 2.25 eq.), and $Et_3N$ (1.25 mL, 8.99 mmol, 7.1 eq.) in DCM (15 mL) at room temperature. The reaction mixture was stirred at room temperature for 24 h. AcOH (1 mL, 17.468 mmol, 13.74 eq.) was added. The solvent was removed under reduced pressure and the residue was purified by flash chromatography on silica gel (heptane/EtOAc 100/0 to 0/100) to give Intermediate 61 (820 mg, 67 % purity), used without further purification.

Intermediate 62

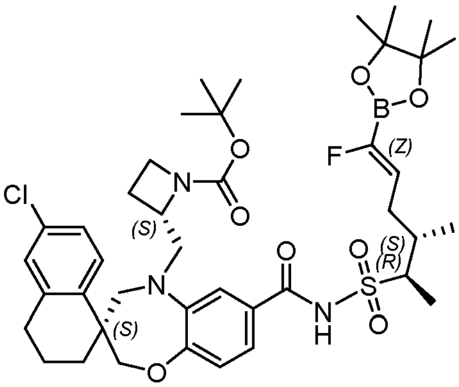

Intermediate 62 was prepared by an analogous reaction protocol as Intermediate 61, starting from Intermediate 60 instead of Intermediate 59.

Intermediate 63

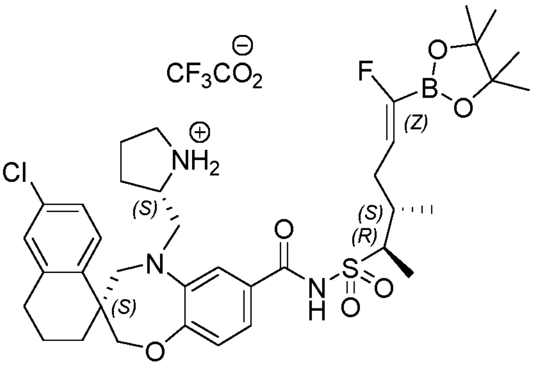

TFA (15 mL, 196 mmol, 81 eq.) was added to a solution of Intermediate 61 (2000 mg, 2.409 mmol) in DCM (30 mL) at room temperature. The reaction mixture was stirred at room temperature for 20 h. The reaction mixture was concentrated under reduced pressure and coevaporated with toluene. The residue was triturated in DIPE. The solid was filtered off, washed, and dried under vacuum at 35 °C to give Intermediate 63 (TFA salt, 1.9 g, yield: 93 %), used without further purification.

Intermediate 64

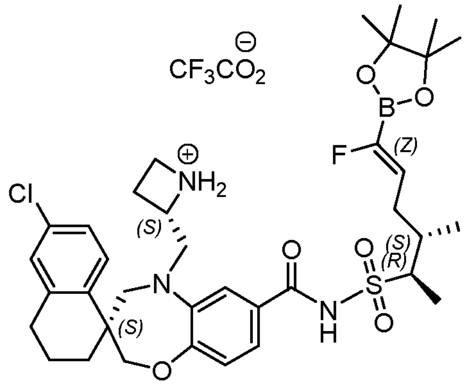

Intermediate 64 was prepared by an analogous reaction protocol as Intermediate 63, starting from Intermediate 62 instead of Intermediate 61.

Preparation of Compounds

Compound 1 and Compound 2

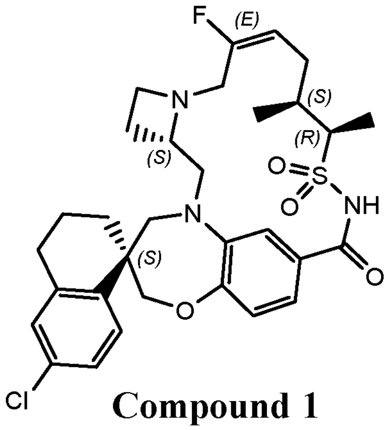

**Compound 1**
(illustrative example)

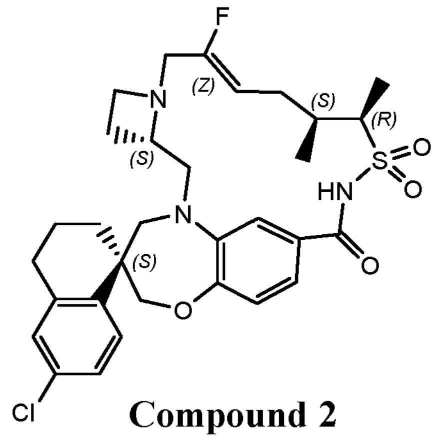

**Compound 2**

Diethyl cyanophosphonate (17.16 μL, 4 eq.) was added to a stirring mixture of Intermediate 14 (16 mg, 0.026 mmol) and DBU (15.4 μL, 4 eq.) in DMF (2 mL). The reaction mixture was stirred for 30 minutes. The reaction mixture was diluted with water and extracted 3 times with DCM. The combined organic layer was washed with brine, dried with $MgSO_4$, filtered, and the solvents were removed under reduced pressure. The residue was purified by preparative HPLC (Stationary phase: RP XBridge Prep C18 OBD-10μm, 30x150 mm, Mobile phase: 0.25 % $NH_4HCO_3$ solution in water, $CH_3CN$) to afford Compound 1 (2.4 mg, yield 15 %, illustrative example) and Compound 2 (5.2 mg, yield 33 %).

$^1H$ NMR Compound 1 (400 MHz, METHANOL-d4) δ ppm 1.05 (d, J=6.6 Hz, 3 H), 1.37 - 1.43 (m, 3 H), 1.43 - 1.53 (m, 1 H), 1.87 - 2.31 (m, 8 H), 2.57 - 2.68 (m, 1 H), 2.72 - 2.87 (m, 2 H), 3.28 (br s, 1 H), 3.35 (br d, J=9.2 Hz, 2 H), 3.48 - 3.60 (m, 2 H), 3.71 - 4.04 (m, 5 H), 4.07 - 4.13 (m, 1 H), 5.45 (d, J=21.7 Hz, 1 H), 6.87 (d, J=8.1 Hz, 1 H), 7.08 - 7.20 (m, 3 H), 7.26 (s, 1 H), 7.74 (d, J=8.4 Hz, 1 H)

$^1H$ NMR Compound 2 (400 MHz, METHANOL-d4) δ ppm 1.05 (d, J=6.8 Hz, 3 H), 1.34 (d, J=7.3 Hz, 3 H), 1.57 - 1.77 (m, 1 H), 1.80 - 2.27 (m, 8 H), 2.68 - 2.95 (m, 5 H), 3.07 - 3.15 (m, 1 H), 3.36 - 3.77 (m, 5 H), 3.91 - 4.13 (m, 4 H), 4.79 (d, J=37.6 Hz, 1 H), 6.84 (d, J=8.1 Hz, 1 H), 7.03 - 7.21 (m, 3 H), 7.72 - 7.79 (m, 1 H), 8.10 (br s, 1 H)

Compound 3

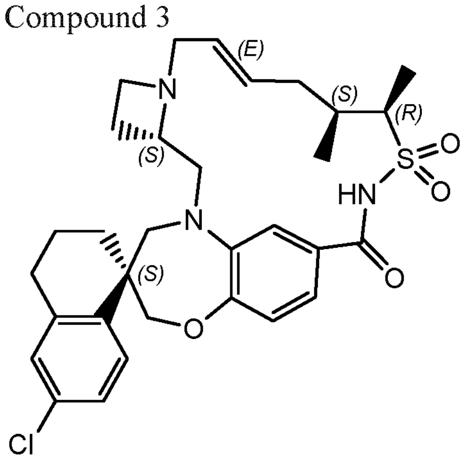

Intermediate 17 (30 mg, 0.049 mmol) was dissolved in DCE (4.2 mL) (Solution A). This was sonificated and flushed with nitrogen. [1,3-Bis(2,4,6-trimethylphenyl)imidazolidin-2-ylidene](([2-(propan-2-yloxy)phenyl]methylidene))rutheniumbis(ylium) dichloride [301224-40-8] (6.14 mg, 0.2 eq) was dissolved in DCE (30 mL), sonificated and flushed with nitrogen (Solution B). Solution A was added to Solution B with a syringe pump (0.175 mL/h). The reaction vessel was kept at 55°C under a nitrogen flow. After cooling to room temperature, activated carbon was added to the reaction mixture and it was vigorously stirred overnight. The reaction mixture was filtered through a plug of dicalite® and the filter pad was rinsed with DCM followed by MeOH. The filtrate was evaporated under vacuum at room temperature. The residue was purified by preparative HPLC (Stationary phase: RP XBridge Prep C18 OBD-10μm, 30x150 mm, Mobile phase: 0.25 % $NH_4HCO_3$ solution in water, $CH_3CN$) to afford compound 3 (4 mg, yield: 14 %)

$^1H$ NMR (400 MHz, METHANOL-d4) δ ppm 1.02 (d, J=6.8 Hz, 3 H), 1.29 - 1.35 (m, 3 H), 1.39 - 1.50 (m, 1 H), 1.84 - 2.25 (m, 9 H), 2.70 - 2.84 (m, 2 H), 3.03 - 3.13 (m, 1 H), 3.17 - 3.30 (m, 4 H), 3.47 - 3.57 (m, 1 H), 3.69 (d, J=14.1 Hz, 1 H), 3.88 - 3.98 (m, 2 H), 3.98 - 4.10 (m, 3 H), 5.53 (d, J=15.2 Hz, 1 H), 5.74 (d, J=15.6 Hz, 1 H), 6.83 (d, J=8.1 Hz, 1 H), 7.09 (d, J=2.2 Hz, 1 H), 7.15 - 7.22 (m, 2 H), 7.58 (br s, 1 H), 7.74 (d, J=8.4 Hz, 1 H)

Compound 4

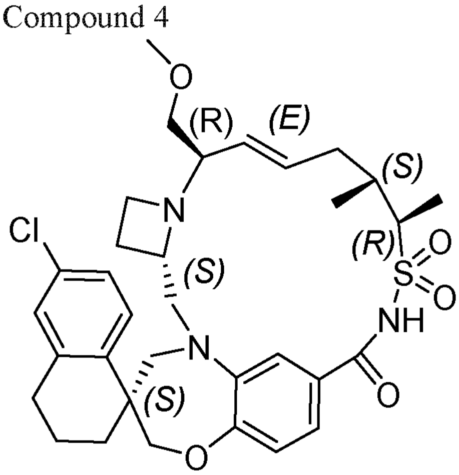

isolated as $iPrNH_2$ salt

Into an Easymax™ reaction vessel was added dry DCE (120 mL) which was degassed on an ultrasonic bath for 5 minutes. Then [1,3-bis(2,4,6-trimethylphenyl)imidazolidin-2-ylidene](([2-(propan-2-yloxy)phenyl]methylidene))rutheniumbis(ylium) dichloride [301224-40-8] (87 mg, 0.14 mmol) was added and the resulting solution was flushed through with nitrogen gas for 5 minutes. A degassed solution (ultrasonic bath) of Intermediate 29 (302 mg, 0.46 mmol) in dry DCE (20 mL) was then added dropwise over a period of 40 hours (0.5 mL/h), using a syringe pump, at 50 °C, under nitrogen atmosphere. After addition, the reaction mixture was stirred at 50 °C for 2 days. To push the reaction to completion, an additional amount of [1,3-bis(2,4,6-trimethylphenyl)imidazolidin-2-ylidene](([2-(propan-2-yloxy)phenyl]methylidene))rutheniumbis(ylium) dichloride (33 mg, 0.053 mmol) was added and the reaction mixture was stirred at 50 °C for 18 hours. The reaction was quenched at 50 °C by the addition of ethyl vinyl ether (0.44 mL, 4.6 mmol). The reaction mixture was stirred at 50 °C for 1 hour. Then, 0.62 g of metal scavenger Silicycle SiliaMetS® (0.62 g, loading

0.61 mmol/g) was added to scavenge the ruthenium catalyst. The resulting suspension was stirred at room temperature for 1 hour. The mixture was filtered over Dicalite®. The filter was washed intensively with EtOAc and with methanol. The solvents of the filtrate were evaporated under reduced pressure at 45 °C. The residue was dissolved in DCM and was purified by preparative HPLC (Stationary phase: RP XBridge Prep C18 OBD-10 μm, 50x150 mm, Mobile phase: 0.25 % $NH_4HCO_3$ solution in water, $CH_3CN$). The solvents were evaporated and co-evaporated with methanol under reduced pressure at 45°C. The residue was purified by preparative SFC (Stationary phase: Chiralpak Diacel AS 20 × 250 mm, Mobile phase: $CO_2$, EtOH + 0.4 % $iPrNH_2$) to afford Compound 4 (18.5 mg, 6 % yield).

1H NMR (400 MHz, CHLOROFORM-d) δ ppm 1.01 (d, J=5.85 Hz, 3 H) 1.29 - 1.35 (m, 1 H) 1.41 (d, J=7.21 Hz, 3 H) 1.70 - 1.91 (m, 2 H) 2.03 - 2.11 (m, 6 H) 2.66 - 2.81 (m, 2 H) 3.15 - 3.29 (m, 4 H) 3.32 (s, 3 H) 3.32 - 3.38 (m, 2 H) 3.40 - 3.49 (m, 1 H) 3.66 - 3.72 (m, 2 H) 3.86 (br d, J=15.05 Hz, 1 H) 4.02 (d, J=12.12 Hz, 1 H) 4.06 (d, J=12.12 Hz, 1 H) 4.19 (q, J=7.04 Hz, 1 H) 5.70 (dd, J=15.26, 8.78 Hz, 1 H) 5.75 - 5.85 (m, 1 H) 6.87 (d, J=8.05 Hz, 1 H) 6.98 (dd, J=8.05, 1.46 Hz, 1 H) 7.01 (s, 1 H) 7.06 (d, J=2.19 Hz, 1 H) 7.15 (dd, J=8.47, 2.30 Hz, 1 H) 7.67 (d, J=8.57 Hz, 1 H)

Alternative method for the synthesis of Compound 4

Reaction performed under nitrogen atmosphere. Into an Easymax reaction vessel was added dry toluene (56 mL) which was degassed for 5 min. The reaction mixture was stirred at 80 °C. Then [1,3-bis(2,4,6-trimethylphenyl)imidazolidin-2-ylidene](([2-(propan-2-yloxy)phenyl]methylidene))rutheniumbis(ylium) dichloride [301224-40-8] (25.8 mg, 0.3 eq.) was added and the resulting solution was flushed through with nitrogen gas for 5 min. A degassed solution of Intermediate 29 (90 mg, 0.137 mmol) dissolved in toluene (8 mL) was then added dropwise over a period of 4 h (2 mL/h), using a syringe pump, at 80 °C under nitrogen atmosphere. After the addition, the reaction mixture was stirred at 80 °C for 16 h. The reaction mixture was cooled to room temperature, treated with ethyl vinyl ether (0.132 mL, 10 eq.) and stirred for 1 h. The solvent was evaporated and the residue was purified by flash chromatography on silica gel (eluent: gradient DCM 90 $MeOH/NH_3$ 10 to MeOH 100 %). The product fractions were purified again by preparative SFC (Stationary phase: Chiralpak Diacel AS 20 × 250 mm, Mobile phase: $CO_2$, EtOH + 0.4 % $iPrNH_2$) yielding Compound 4 (36 mg, yield: 38 %)

Compounds 7 and 8 were prepared by an analogous reaction protocol as Compound 4 using the appropriate starting materials:

| Compound | Structure | *Starting material* |
|---|---|---|
| Compound 7 | 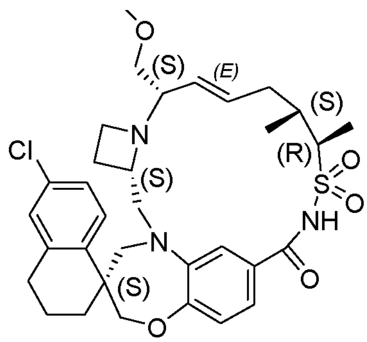 isolated as $iPrNH_2$ salt | Intermediate 45 |
| Compound 8 | 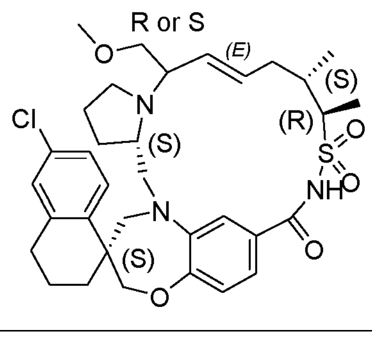 isolated as $iPrNH_2$ salt | Intermediate 46 |

Analytical data

Compound 8

1H NMR (400 MHz, CHLOROFORM-d) δ ppm 0.79 - 0.95 (m, 2 H) 1.03 (br d, J=5.6 Hz, 3 H) 1.45 (d, J=7.2

Hz, 4 H) 1.52 - 1.62 (m, 1 H) 1.65 - 1.73 (m, 1 H) 1.74 - 1.84 (m, 2 H) 1.85 - 1.94 (m, 2 H) 2.02 (br s, 3 H) 2.68 - 2.80 (m, 2 H) 2.84 - 3.04 (m, 2 H) 3.21 - 3.37 (m, 3 H) 3.40 (s, 3 H) 3.51 (brt, J=9.2 Hz, 1 H) 3.81 (br s, 1 H) 3.89 -4.04 (m, 3 H) 4.14 (d, J=12.2 Hz, 1 H) 4.22 - 4.34 (m, 1 H) 5.45 - 5.59 (m, 1 H) 5.75 - 5.90 (m, 1 H) 6.90 (s, 2 H) 6.98 - 7.10 (m, 2 H) 7.19 (dd, J=8.5, 2.2 Hz, 1 H) 7.69 (d, J=8.5 Hz, 1 H)

Compound 5 and Compound 6

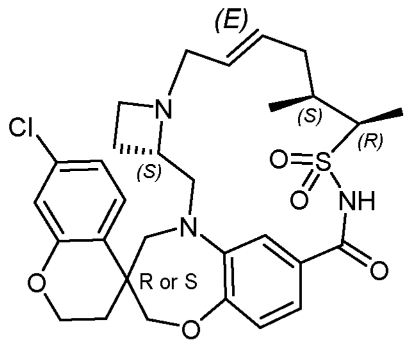

Compound 5

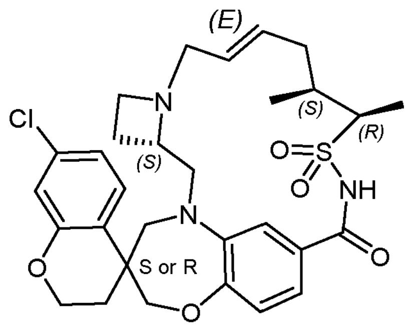

Compound 6

[1,3-Bis(2,4,6-trimethylphenyl)imidazolidin-2-ylidene](([2-(propan-2-yloxy)phenyl]methylidene))rutheniumbis(ylium) dichloride [301224-40-8] (45 mg, 0.4 eq) was dissolved in DCE (180 mL) and the solution was flushed with nitrogen (Solution A). Intermediate 36 (110 mg, 0.18 mmol) was dissolved in DCE (20 mL) and flushed with nitrogen (Solution B). Solution B was added via syringe pump into Solution A at 55 °C over 16 h. After the addition, stirring was continued at 55 °C for 5 h. Ethyl vinyl ether (200 μL) was added and after cooling to room temperature, metal scavenger Silicycle SiliaMetS® was added to scavenge the ruthenium catalyst. After 1 h of vigorous stirring, the mixture was filtered over Celite®. The solid was rinsed thoroughly with DCM and MeOH. The solvents of the filtrate were evaporated under reduced pressure. The residue was purified by flash chromatography on silica (0 to 40 % EtOAc in heptane). The fractions containing the racemic mixture of products were combined and evaporated. The residue was purified by preparative SFC (Stationary phase: Chiralpak Diacel AD 20 × 250 mm, Mobile phase: $CO_2$, EtOH + 0.4 % $iPrNH_2$) to yield Compound 5 (13 mg, 13 % yield) and Compound 6 (16 mg, 15 % yield).

Compound 5

1H NMR (400 MHz, METHANOL-d4) δ ppm 1.03 (d, J=6.9 Hz, 3 H), 1.33 (d, J=7.3 Hz, 3 H), 1.75 - 1.86 (m, 1 H), 1.93 - 2.01 (m, 2 H), 2.07 (dt, J=14.3, 2.9 Hz, 1 H), 2.15 - 2.27 (m, 3 H), 3.04 (br dd, J=14.5, 6.3 Hz, 1 H), 3.22 (br dd, J=15.7, 4.7 Hz, 1 H), 3.43 (d, J=14.3 Hz, 1 H), 3.61 - 3.69 (m, 1 H), 3.78 (d, J=14.3 Hz, 1 H), 3.86 - 3.92 (m, 1 H), 3.94 - 4.05 (m, 1 H), 4.13 (q, J=1.0 Hz, 3 H), 4.20 - 4.32 (m, 2 H), 5.43 - 5.51 (m, 1 H), 5.63 (br dt, J=15.1, 6.9 Hz, 1 H), 6.80 (d, J=2.2 Hz, 1 H), 6.89 (d, J=8.2 Hz, 1 H), 6.92 (dd, J=8.6, 2.0 Hz, 1 H), 7.24 (dd, J=8.2, 1.9 Hz, 1 H), 7.66 (d, J=8.5 Hz, 1 H), 7.69 - 7.73 (m, 1 H)

Compound 6

1H NMR (400 MHz, METHANOL-d4) δ ppm 1.03 (d, J=6.8 Hz, 3 H), 1.33 (d, J=7.3 Hz, 3 H), 1.99 - 2.09 (m, 4 H), 2.16 - 2.31 (m, 3 H), 3.06 (br dd, J=14.6, 6.5 Hz, 1 H), 3.18 (br dd, J=15.2, 6.2 Hz, 1 H), 3.37 - 3.46 (m, 2 H), 3.49 (s, 2 H), 3.51 - 3.57 (m, 1 H), 3.69 - 3.78 (m, 1 H), 3.86 (br d, J=7.5 Hz, 1 H), 4.11 - 4.17 (m, 1 H), 4.21 - 4.29 (m, 3 H), 5.48 - 5.56 (m, 1 H), 5.67 - 5.75 (m, 1 H), 6.82 (d, J=2.2 Hz, 1 H), 6.88 - 6.91 (m, 1 H), 6.92 (d, J=8.1 Hz, 1 H), 7.37 (d, J=8.4 Hz, 1 H), 7.53 - 7.57 (m, 1 H), 7.58 - 7.63 (m, 1 H)

Compound 9

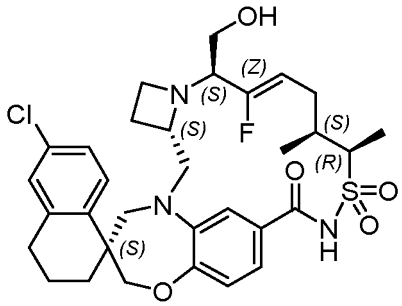

Intermediate 49 (241 mg, 0.329 mmol) was dissolved in dry THF (30 mL) in a stainless steel vessel. DBU [6674-22-2] (250 μL, 5 eq.) was added and the mixture was purged with nitrogen. $PdCl_2$(dppf) [72287-26-4] (25 mg, 0.1 eq.) was then added. The reactor was purged with CO and the vessel was sealed. The reaction mixture was heated at 100 °C under CO pressure (50 bar) for 20 h. After cooling to room temperature, the mixture was diluted with EtOAc, filtered through celite, and the filter was washed with EtOAc. The filtrate was evaporated under reduced pressure and the residue was purified by column chromatography on silica gel using heptane/EtOAc (1:0 to 0:1) to afford Compound 9 (58 mg, yield: 28 %) as a pale yellow solid and an impure fraction that was purified by reverse phase chromatography (Stationary phase: RP XBridge Prep C18 OBD- 5μm,50 × 250mm, Mobile phase: 0.25 % $NH_4HCO_3$ solution in water, $CH_3CN$) to afford a second batch of Compound 9 (90 mg, yield: 43 %) as a white solid.

$^1$H NMR (400 MHz, CHLOROFORM-*d*) δ ppm 1.06 (d, *J*=6.6 Hz, 3 H) 1.36 - 1.49 (m, 5 H) 1.77 - 2.17 (m, 8 H) 2.32 (br dd, *J*=16.3, 7.3 Hz, 1 H) 2.70 - 2.86 (m, 3 H) 3.09 - 3.45 (m, 6 H) 3.58 (d, *J*=7.5 Hz, 2 H) 3.71 - 3.90 (m, 3 H) 4.02 - 4.16 (m, 3 H) 4.79 - 5.07 (m, 1 H) 5.47 (br s, 1 H) 6.91 (d, *J*=7.9 Hz, 1 H) 6.97 - 7.08 (m, 2 H) 7.10 (d, *J*=2.0 Hz, 1 H) 7.18 (dd, *J*=8.6, 2.2 Hz, 1 H) 7.70 (d, *J*=8.6 Hz, 1 H)

The compounds in the table below were prepared by an analogous reaction protocol as Compound 9 using the appropriate starting materials:

| Compound | Structure | *Starting material* |
|---|---|---|
| Compound 10 | 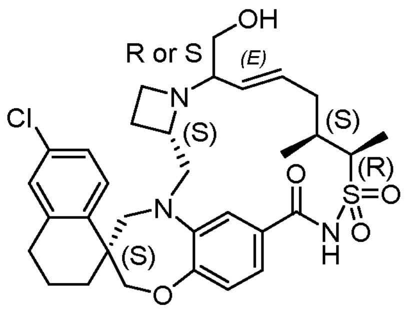 | Intermediate 50 |
| Compound 27 | 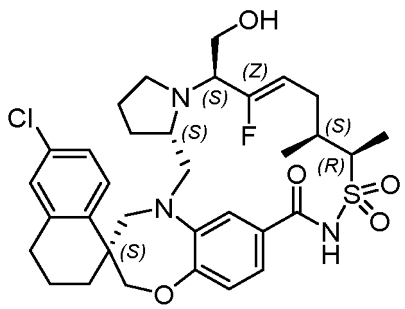 | Intermediate 55 |

Compound 11

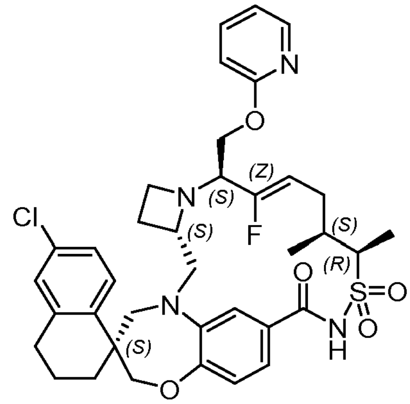

NaH (60 % dispersion in mineral oil) (7.9 mg, 5 eq.) was added cautiously to a stirred solution of Compound 9 (25 mg, 0.0395 mmol) in 1 mL of anhydrous THF at room temperature under nitrogen atmosphere. The resulting mixture was stirred for 30 min at room temperature, followed by dropwise addition of 2-fluoropyridine (19.2 mg, 5 eq.). The reaction mixture was stirred at 50 °C for 3 h. The reaction mixture was cooled to room temperature, and the reaction was quenched with a saturated aqueous $NH_4Cl$ solution. The mixture was extracted with AcOEt. The organic layer was dried with $MgSO_4$, filtered, and evaporated. The residue was purified by preparative HPLC (Stationary phase: RP XBridge Prep C18 OBD - 10 μm,50 × 150 mm, Mobile phase: 0.25 % $NH_4HCO_3$ solution in water, MeOH) yielding Compound 11 (13 mg, yield: 46 %).

$^1$H NMR (400 MHz, CHLOROFORM-*d*) δ ppm 1.04 - 1.16 (m, 3 H) 1.39 (br d, *J*=7.0 Hz, 6 H) 1.91 - 2.17 (m, 6 H) 2.22 (br s, 1 H) 2.28 - 2.43 (m, 1 H) 2.56 - 2.70 (m, 5 H) 2.76 - 2.93 (m, 2 H) 3.22 (br s, 1 H) 3.32 (br d, *J*=14.3 Hz, 1 H) 3.42 (s, 3 H) 3.75 (br d, *J*=14.1 Hz, 1 H) 3.94 - 4.29 (m, 6 H) 4.81 - 5.13 (m, 1 H) 6.84 (br d, *J*=0.9 Hz, 1 H) 6.98 (br d, *J*=7.9 Hz, 1 H) 7.04 - 7.19 (m, 2 H) 7.35 (br d, *J*=8.4 Hz, 2 H) 7.64 - 7.84 (m, 2 H) 8.24 (br d, *J*=3.7 Hz, 1 H) 11.90 - 12.14 (m, 1 H)

Compound 13

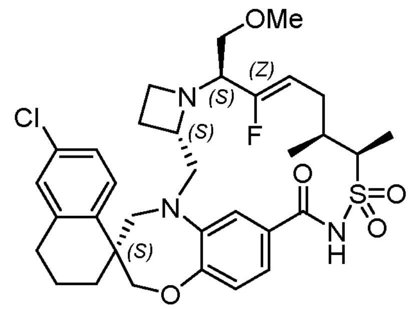

NaH (60 % dispersion in mineral oil, 11 mg, 3 eq.) was added to a stirred solution of Compound 9 (58 mg, 0.091 mmol) dissolved in anhydrous THF (10 mL). After 30 min, MeI (50 μL, 8.75 eq.) was added in one portion and the pink mixture was stirred at room temperature for 2 h. Water (10 mL) and EtOAc (20 mL) were added. The organic layer was extracted, washed with brine (2 × 10 mL), dried ($Na_2SO_4$), filtered, and evaporated. The residue was purified by preparative HPLC (Stationary phase: RP XBridge Prep C18 OBD- 5 μm, 50 × 250 mm, Mobile phase: 0.25 % $NH_4HCO_3$ solution in water, $CH_3CN$) to give Compound 13 (35.8 mg, yield: 60 %) as a pale brown powder.

$^1$H NMR (400 MHz, CHLOROFORM-*d*) δ ppm 1.06 (br d, J=6.2 Hz, 3 H) 1.39 - 1.51 (m, 3 H) 1.70 - 2.18 (m, 8 H) 2.36 (br dd, J=14.6, 7.8 Hz, 1 H) 2.67 - 2.85 (m, 2 H) 3.13 - 3.57 (m, 10 H) 3.69 - 3.82 (m, 2 H) 3.88 (br d, J=15.2 Hz, 1 H) 4.01 - 4.15 (m, 2 H) 4.15 - 4.25 (m, 1 H) 4.76 - 4.97 (m, 1 H) 6.95 (q, J=8.1 Hz, 2 H) 7.09 (br d, J=1.8 Hz, 2 H) 7.18 (dd, J=8.5, 1.9 Hz, 1 H) 7.69 (d, J=8.6 Hz, 1 H)

Compound 14

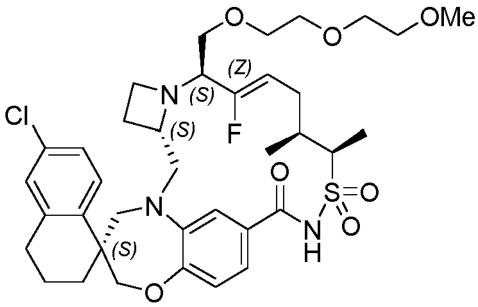

NaH (60 % dispersion in mineral oil, 8 mg, 4.2 eq.) was added cautiously to a stirred solution of Compound 9 (30 mg, 0.047 mmol) in anhydrous THF (2 mL) at room temperature. The resulting mixture was stirred for 20 min at room temperature, followed by dropwise addition of 1-bromo-2-(2-methoxyethoxy)ethane (CAS [54149-17-6]) (32 μL, 5 eq.). The mixture was then heated at 50 °C and stirred for 1.5 h. The reaction mixture was cooled to room temperature, diluted with EtOAc (10 mL) and washed with water. The organic layer was washed with brine (10 mL), dried on $MgSO_4$, filtered, and evaporated. The residue was stirred in DIPE and the precipitate was filtered off, washed with DIPE and dried in vacuo to afford Compound 14 (27 mg, yield: 77 %) as a white solid.

$^1H$ NMR (400 MHz, DMSO-$d_6$) δ ppm 0.88 (d, J=6.8 Hz, 3 H) 1.13 (br d, J=7.0 Hz, 3 H) 1.31 - 1.43 (m, 1 H) 1.76 - 2.05 (m, 7 H) 2.13 (br dd, J=16.5, 7.0 Hz, 1 H) 2.63 - 2.83 (m, 2 H) 3.00 (br dd, J=15.1, 9.4 Hz, 1 H) 3.06 - 3.19 (m, 3 H) 3.25 (s, 3 H) 3.34 - 3.40 (m, 3 H) 3.41 - 3.45 (m, 2 H) 3.46 - 3.56 (m, 6 H) 3.57 - 3.69 (m, 2 H) 3.80 (br d, J=14.1 Hz, 2 H) 3.91 - 3.96 (m, 1 H) 3.99 - 4.05 (m, 1 H) 4.93 - 5.30 (m, 1 H) 6.78 (d, J=8.1 Hz, 1 H) 6.99 - 7.05 (m, 1 H) 7.05 - 7.13 (m, 1 H) 7.16 (d, J=2.2 Hz, 1 H) 7.26 (dd, J=8.5, 2.3 Hz, 1 H) 7.67 (d, J=8.6 Hz, 1 H).

Compound 15

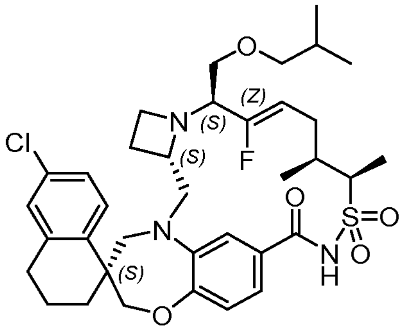

Compound 15 was prepared following an analogous procedure as Compound 14, using 1-bromo-2-methylpropane instead of 1-bromo-2-(2-methoxyethoxy)ethane.

$^1H$ NMR (400 MHz, CHLOROFORM-*d*) δ ppm 0.89 (dd, J=6.6, 3.5 Hz, 6 H) 1.07 (d, J=6.6 Hz, 3 H) 1.33 - 1.48 (m, 5 H) 1.85 (dt, J=13.4, 6.7 Hz, 2 H) 1.89 - 2.16 (m, 7 H) 2.37 (br dd, J=15.7, 8.0 Hz, 1 H) 2.69 - 2.86 (m, 2 H) 3.10 - 3.34 (m, 5 H) 3.37 (br d, J=6.8 Hz, 1 H) 3.45 (s, 2 H) 3.71 - 3.81 (m, 2 H) 3.88 (br d, J=15.2 Hz, 1 H) 4.04 - 4.15 (m, 2 H) 4.21 (q, J=7.2 Hz, 1 H) 4.76 - 4.92 (m, 1 H) 6.94 (s, 2 H) 7.09 (d, J=2.2 Hz, 2 H) 7.19 (dd, J=8.6, 2.2 Hz, 1 H) 7.70 (d, J=8.6 Hz, 1 H).

Compound 16

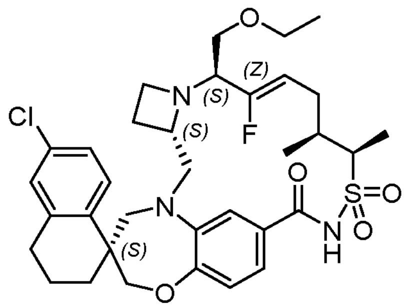

Compound 16 was prepared following an analogous procedure as Compound 14, using ethyl iodide instead of 1-bromo-2-(2-methoxyethoxy)ethane.

$^1$H NMR (400 MHz, DMSO-$d_6$) δ ppm 0.88 (d, J=6.8 Hz, 3 H) 1.05 - 1.16 (m, 6 H) 1.35 - 1.45 (m, 1 H) 1.76 - 1.94 (m, 4 H) 1.95 - 2.06 (m, 3 H) 2.14 (br dd, J=16.5, 6.8 Hz, 1 H) 2.65 - 2.84 (m, 2 H) 3.02 (dd, J=15.2, 9.5 Hz, 1 H) 3.13 - 3.30 (m, 7 H) 3.39 - 3.49 (m, 2 H) 3.58 - 3.70 (m, 2 H) 3.72 - 3.84 (m, 2 H) 3.90 - 3.97 (m, 1 H) 4.00 - 4.06 (m, 1 H) 5.10 - 5.32 (m, 1 H) 6.75 (d, J=8.1 Hz, 1 H) 7.05 (dd, J=8.1, 1.5 Hz, 1 H) 7.10 - 7.17 (m, 2 H) 7.25 (dd, J=8.5, 2.3 Hz, 1 H) 7.68 (d, J=8.4 Hz, 1 H).

Compound 17

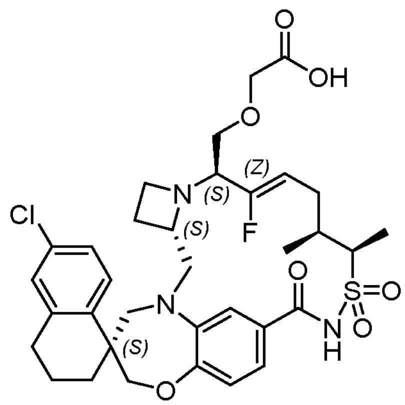

NaH (60 % dispersion in mineral oil) (40 mg, 10 eq.) was added portionwise to a stirred solution of Compound 9 (60 mg, 0.095 mmol) in anhydrous THF (3 mL). The resulting mixture was stirred for 15 min at room temperature. After cooling to 0 °C, bromoacetic acid (77 mg, 5.8 eq.) was added portionwise and the resulting mixture was stirred at 50 °C for 5 days. Upon cooling to room temperature, the mixture was carefully quenched with water and acifidied with a 1 M HCl aqueous solution until pH reached 5-6. DCM (10 mL) was added and the layers were separated. The aqueous layer was extracted again with DCM (3 × 5 mL). The combined organic layer was washed with brine (2 × 20 mL), dried on $MgSO_4$, filtered, and evaporated under reduced pressure. The residue was dissolved in a minimum amount of DCM followed by addition of DIPE. The precipitate was filtered off, washed with DIPE and dried in vacuo to yield Compound 17 (57 mg, yield 87 %) as a white solid.

$^1$H NMR (400 MHz, CHLOROFORM-*d*) δ ppm 1.09 (d, J=6.4 Hz, 3 H) 1.47 (d, J=7.3 Hz, 4 H) 1.76 - 1.90 (m, 1 H) 1.93 - 2.10 (m, 4 H) 2.13 - 2.25 (m, 2 H) 2.29 - 2.38 (m, 1 H) 2.70 - 2.86 (m, 2 H) 3.21 (d, J=14.3 Hz, 1 H) 3.36 (dd, J=15.6, 10.3 Hz, 1 H) 3.44 - 3.56 (m, 3 H) 3.62 - 3.70 (m, 1 H) 3.77 (d, J=14.1 Hz, 1 H) 3.91 - 4.03 (m, 3 H) 4.06 - 4.21 (m, 4 H) 4.23 - 4.31 (m, 1 H) 4.94 - 5.09 (m, 1 H) 6.93 - 7.07 (m, 3 H) 7.11 (d, J=2.2 Hz, 1 H) 7.18 (dd, J=8.5, 2.3 Hz, 1 H) 7.67 (d, J=8.6 Hz, 1 H).

Compound 18

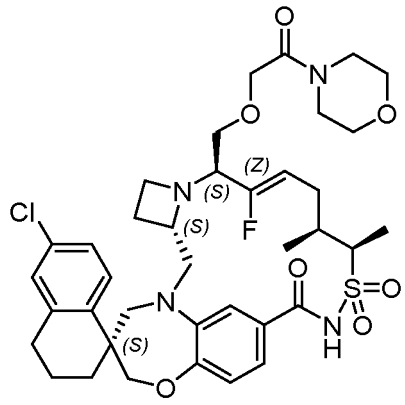

1-Propanephosphonic anhydride (CAS [68957-94-8]) (100 μL, 2.3 eq.) was added dropwise to a stirred solution of Compound 17 (25 mg, 0.036 mmol), morpholine (15 mg, 4.75 eq.), and $Et_3N$ (50 μL, 10 eq.) in DCM (1 mL) at room temperature. After the addition, the reaction mixture was stirred at room temperature for 2 h. The reaction mixture was diluted with DCM and washed with a saturated aqueous NaHCOs solution. The organic layer was dried over an isolute filter and the solvents of the filtrate evaporated under reduced pressure. The residue was purified by flash column chromatography on silica gel using DCM/MeOH (going from 100:0 to 95:5) as eluent. The product fractions were collected, evaporated, and coevaporated with DIPE until Compound 18 was obtained as a white solid (16 mg, yield: 58 %).

$^1$H NMR (400 MHz, CHLOROFORM-*d*) δ ppm 1.08 (d, J=6.6 Hz, 3 H) 1.46 (d, J=7.2 Hz, 3 H) 1.56 - 1.74 (m, 3 H) 1.77 - 1.83 (m, 1 H) 1.90 - 1.99 (m, 3 H) 2.01 - 2.06 (m, 1 H) 2.08 - 2.15 (m, 1 H) 2.36 (br dd, J=15.6, 8.1 Hz, 1 H) 2.78 (br d, J=4.4 Hz, 2 H) 3.19 (s, 1 H) 3.24 (br d, J=14.3 Hz, 1 H) 3.29 - 3.40 (m, 2 H) 3.53 (br s, 4 H) 3.60 - 3.63 (m, 2 H) 3.69 (br d, J=3.9 Hz, 7 H) 3.83 - 3.93 (m, 1 H) 4.03 - 4.15 (m, 3 H) 4.15 - 4.24 (m, 2 H) 4.70 - 4.97 (m, 1 H) 6.82 - 7.07 (m, 3 H) 7.09 (d, J=2.1 Hz, 1 H) 7.19 (dd, J=8.5, 2.1 Hz, 1 H) 7.69 (d, J=8.6 Hz, 1 H).

Compound 19

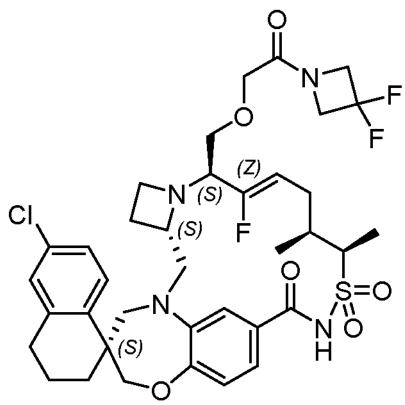

Compound 19 was prepared following an analogous procedure as Compound 18, using 3,3-difluoroazetidine hydrochloride instead of morpholine.

$^1$H NMR (400 MHz, CHLOROFORM-*d*) δ ppm 1.09 (br d, J=6.5 Hz, 3 H) 1.46 (br d, J=7.1 Hz, 4 H) 1.75 - 1.86 (m, 1 H) 1.88 - 2.18 (m, 6 H) 2.37 (br dd, J=16.5, 7.8 Hz, 1 H) 2.79 (br d, J=3.3 Hz, 2 H) 3.23 (br d, J=14.2 Hz, 5 H) 3.49 (br s, 2 H) 3.75 (br d, J=14.7 Hz, 2 H) 3.87 (br d, J=14.8 Hz, 1 H) 4.04 - 4.24 (m, 5 H) 4.38 (br t, J=12.0 Hz, 2 H) 4.47 - 4.72 (m, 2 H) 4.77 - 4.97 (m, 1 H) 6.94 (br d, J=4.8 Hz, 2 H) 7.00 - 7.07 (m, 1 H) 7.10 (s, 1 H) 7.17 - 7.22 (m, 1 H) 7.69 (d, J=8.6 Hz, 1 H) 7.98 - 8.22 (m, 1 H).

Compound 20

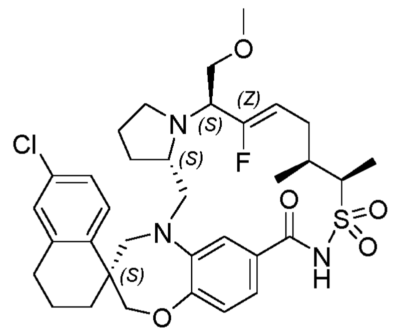

Compound 20 was prepared following an analogous procedure as for Compound 13, starting from Intermediate 40 instead of Intermediate 22.

Compound 22

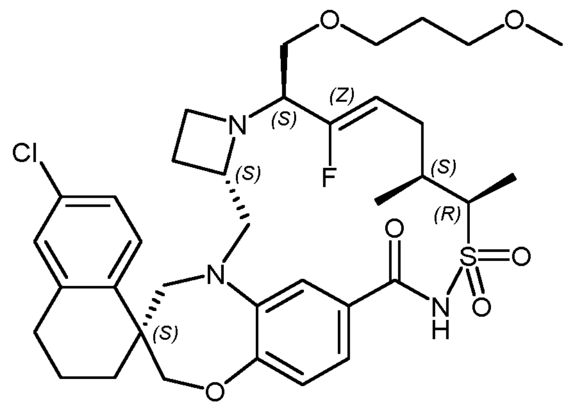

NaH (60 % dispersion in mineral oil) (14 mg, 8.8 eq.) was added cautiously to a stirred solution of Compound 9 (25 mg, 0.0395 mmol) in THF (1.5 mL) at room temperature. The resulting mixture was stirred for 30 min at room temperature. 1-iodo-3-methoxypropane [61542-10-7] (100 mg, 12.6 eq.) was then added dropwise and the reaction mixture was stirred at 50 °C for 24 h. The reaction mixture was cooled to room temperature, diluted with DCM (10 mL) and water and AcOH were added (until pH reached 5). The layers were separated and the organic layer was dried by filtration over an isolute filter. The filtrate was concentrated and the residue was purified by preparative HPLC (Stationary phase: RP XBridge Prep C18 OBD- 5 μm,50 × 250 mm, Mobile phase: 0.25 % $NH_4HCO_3$ solution in water, $CH_3CN$) to afford Compound 22 (6 mg, yield: 21 %).

The following compounds were prepared from Compound 9 using an analogous procedure as for Compound 22, using the indicated reagent instead of 1-iodo-3-methoxypropane

| | Structure | Reagent used |
|---|---|---|
| Compound 23 | 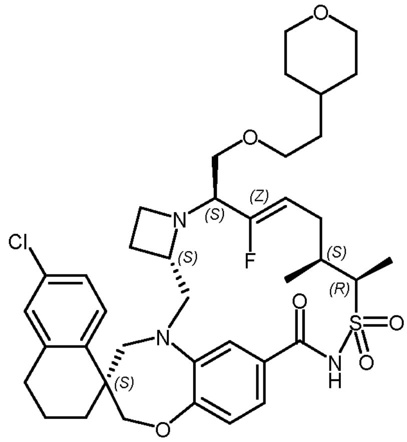 | 4-(2-bromoethyl)tetrahydropyran [4677-20-7] |
| Compound 24 | 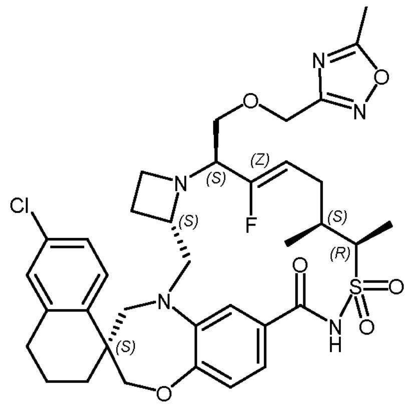 | 3-(bromomethyl)-5-methyl-1,2,4-oxadiazole [959406-40-7] |
| Compound 25 | 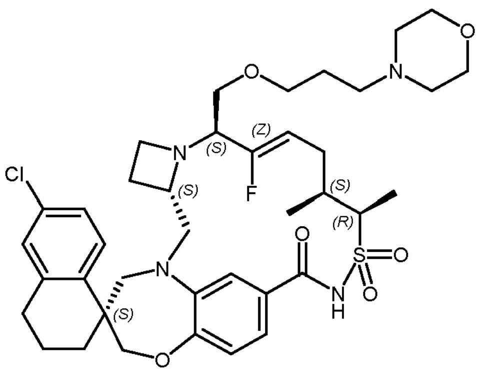 | 4-(3-bromopropyl)morpholine HBr salt [125422-83-5] |
| Compound 26 | 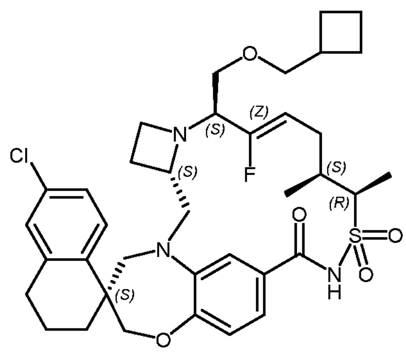 | (bromomethyl)cyclobutane [17247-58-4] |

Compound 28

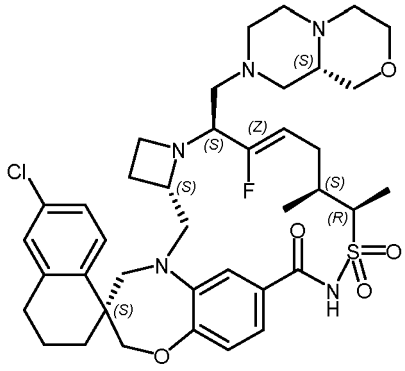

Compound 9 (47 mg, 0.074 mmol) and dry DCM (2.4 mL) were stirred in a 10 mL predried capped tube. Nitrogen was bubbled through the reaction mixture for 10 min before $Et_3N$ (23 μL, 0.169 mmol, 2.3 eq.) was added. The reaction mixture was cooled to - 78 °C and triflic anhydride (18 μL, 0.107 mmol, 1.4 eq.) was added dropwise at -78 °C. The resulting mixture was stirred at -78 °C for 4 h. (S)-octahydropyrazino[2,1-c][1,4]oxazine [1089759-42-1] (42 mg, 0.297 mmol, 4 eq.) in dry DCM (0.5 mL) was added dropwise at -78 °C. The reaction mixture was stirred at at -78 °C for 30 min and then at room temperature for 1 h. The reaction mixture was poured into water and extracted with DCM. The organic layer was dried on $MgSO_4$, filtered, and evaporated. The residue was purified by preparative SFC (Stationary phase: Chiralpak Daicel IG 20 × 250 mm, Mobile phase: $CO_2$, EtOH + 0.4 % $iPrNH_2$) to afford Compound 28 as its $iPrNH_2$ salt (4 mg, yield: 6 %).

$^1$H NMR (400 MHz, CHLOROFORM-*d*) δ ppm 0.80 - 0.94 (m, 2 H) 1.04 (br d, *J*=6.6 Hz, 3 H) 1.15 - 1.30 (m, 3 H) 1.32 - 1.49 (m, 5 H) 1.74 - 2.13 (m, 8 H) 2.14 - 2.24 (m, 1 H) 2.30 - 2.61 (m, 6 H) 2.65 (br d, *J*=11.4 Hz, 2 H) 2.75 (br d, *J*=10.1 Hz, 4 H) 3.08 (br dd, *J*=8.7, 3.4 Hz, 2 H) 3.18 - 3.34 (m, 4 H) 3.59 - 3.94 (m, 8 H) 4.02 (br s, 2 H) 4.81 - 5.23 (m, 1 H) 6.78 - 6.91 (m, 1 H) 6.98 (br s, 1 H) 7.03 - 7.03 (m, 1 H) 7.07 (br d, *J*=1.8 Hz, 2 H) 7.16 (br dd, *J*=8.6, 1.8 Hz, 1 H) 7.56 - 7.56 (m, 1 H) 7.69 (d, *J*=8.6 Hz, 1 H)

Compound 29

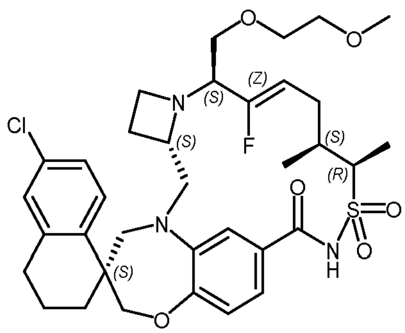

Compound 29 was prepared following an analogous procedure as for Compound 14, using 2-bromoethyl methyl ether instead of 1-bromo-2-(2-methoxyethoxy)ethane.

$^1$H NMR (400 MHz, CHLOROFORM-*d*) δ ppm 0.91 - 1.01 (m, 3 H) 1.22 - 1.32 (m, 4 H) 1.76 - 2.37 (m, 12 H) 2.66 - 2.83 (m, 2 H) 3.11 - 3.27 (m, 4 H) 3.27 -3.34 (m, 3 H) 3.40 - 3.66 (m, 6 H) 3.66 - 3.88 (m, 3 H) 3.99 (s, 2 H) 5.03 - 5.26 (m, 1 H) 6.52 - 6.76 (m, 1 H) 7.02 - 7.24 (m, 3 H) 7.70 (br d, J=8.5 Hz, 1 H).

Compound 30

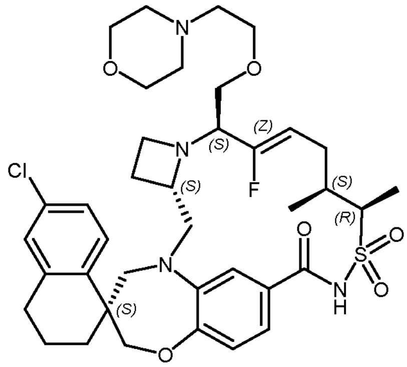

NaH (60 % dispersion in mineral oil, 13 mg, 0.325 mmol, 2.6 eq.) was added to a stirred solution of Compound 9 (80 mg, 0.127 mmol) in dry THF (10 mL). After 30 min, 4-(2-chloroethyl)morpholine (CAS [3240-94-6]) (50 μL, 0.364 mmol, 2.9 eq.) was added in one portion and the resulting mixture was stirred at room temperature for 48 h. Water (10 mL) and EtOAc (10 mL) were added. The organic layer was separated, washed with brine (3 × 5 mL), dried ($MgSO_4$), filtered, and evaporated. The residue was purified by reverse phase chromatography (eluent: $CH_3CN/NH_4HCO_3$) followed by preparative SFC (Stationary phase: Chiralpak Diacel AD 20 × 250 mm, Mobile phase: $CO_2$, EtOH) to afford Compound 30 (2 mg, yield: 2 %) as an off-white solid.

$^1$H NMR (400 MHz, CHLOROFORM-*d*) δ ppm 1.06 - 1.10 (m, 3 H) 1.43 - 1.47 (m, 3 H) 1.96 - 2.10 (m, 10 H) 2.34 - 2.42 (m, 1 H) 2.50 - 2.55 (m, 3 H) 2.55 - 2.64 (m, 2 H) 2.73 - 2.83 (m, 2 H) 3.14 - 3.40 (m, 5 H) 3.43 - 3.51 (m, 2 H) 3.54 - 3.79 (m, 8 H) 3.82 - 3.92 (m, 1 H) 4.02 - 4.18 (m, 3 H) 4.82 - 4.97 (m, 1H) 6.85 - 7.01 (m, 2 H) 7.02 - 7.14 (m, 2 H) 7.16 - 7.23 (m, 1 H) 7.66 - 7.73 (m, 1 H).

Compound 31

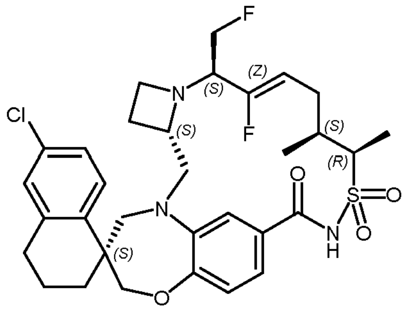

Diethylaminosulfur trifluoride (CAS [38078-09-0]) (15 μL, 0.119 mmol, 2 eq.) was added to a stirred solution of Compound 9 (50 mg, 0.059 mmol) in dry THF (3 mL) at - 10 °C. The reaction mixture was slowly allowed to warm to room temperature and was stirred for 3 days. Upon full conversion of the starting material, the mixture was cooled to 0 °C and the reaction was carefully quenched with saturated aqueous $NaHCO_3$. The reaction mixture was diluted with EtOAc (10 mL) and saturated aqueous NaHCOs (10 mL). The organic layer was separated, washed with brine (10 mL), dried ($MgSO_4$), filtered, and evaporated under reduced pressure. The residue was purified by preparative HPLC (Stationary phase: RP XBridge Prep C18 OBD-10 μm, 30 × 150 mm, Mobile phase: 0.5 % $NH_4Ac$ solution in water + 10 % $CH_3CN$, $CH_3CN$) to yield Compound 31 (5 mg, yield: 12 %) as an off-white solid.

$^1$H NMR (400 MHz, CHLOROFORM-*d*) δ ppm 1.07 - 1.14 (m, 3 H) 1.47 (d, J=7.3 Hz, 3 H) 1.75 - 1.86 (m, 2 H) 1.87 - 2.09 (m, 7 H) 2.13 - 2.20 (m, 1 H) 2.33 - 2.43 (m, 1 H) 2.73 - 2.82 (m, 2 H) 3.14 - 3.26 (m, 2 H) 3.27 - 3.41 (m, 2 H) 3.44 - 3.61 (m, 1 H) 3.72 - 3.87 (m, 2 H) 3.88 - 3.98 (m, 1 H) 4.05 - 4.17 (m, 2 H) 4.21 (q, J=7.0 Hz, 2 H) 4.81 - 5.01 (m, 1 H) 6.84 - 7.01 (m, 2 H) 7.06 - 7.25 (m, 3 H) 7.66 - 7.73 (m, 1 H).

Compound 32

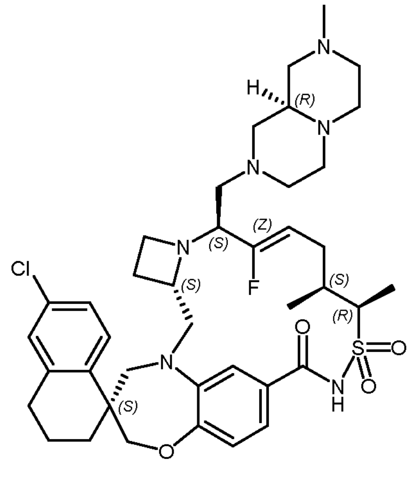

A solution of Intermediate 56 (69 mg, 0.0971 mmol) and 2-methyloctahydro-2H-pyrazino[1,2-a]pyrazine (CAS [63285-62-1]) (50 mg, 0.322 mmol, 3.3 eq.) in ACN (5 mL) was stirred at 80 °C for 18 h. Upon full conversion of the starting material, the reaction mixture was directly purified by preparative HPLC (Stationary phase: RP XBridge Prep C18 OBD - 10 μm, 30 × 150 mm, Mobile phase: 0.5 % $NH_4Ac$ solution in water + 10 % $CH_3CN$, $CH_3CN$) to afford Compound 32 (4 mg, yield: 5 %) as a yellow solid.

$^1H$ NMR (400 MHz, CHLOROFORM-*d*) δ ppm 1.05 - 1.08 (m, 3 H) 1.42 (s, 3 H) 1.55 - 1.64 (m, 2 H) 1.79 - 1.87 (m, 2 H) 1.91 - 2.11 (m, 12 H) 2.16 - 2.54 (m, 24 H) 2.69 - 2.86 (m, 9 H) 2.90 - 2.95 (m, 1 H) 2.98 - 3.07 (m, 1 H) 3.12 - 3.18 (m, 1 H) 3.21 - 3.27 (m, 2 H) 3.32 - 3.38 (m, 1 H) 3.42 - 3.47 (m, 1 H) 3.59 - 3.75 (m, 4 H) 3.75 - 3.90 (m, 2 H) 3.97 - 4.17 (m, 4 H) 4.75 - 4.96 (m, 1 H) 5.26 - 5.43 (m, 1 H) 6.91 (d, J=8.1 Hz, 1 H) 6.95 - 7.05 (m, 2 H) 7.09 (d, J=2.0 Hz, 1 H) 7.18 (dd, J=8.5, 2.3 Hz, 1 H) 7.65 - 7.75 (m, 1 H).

Compound 33

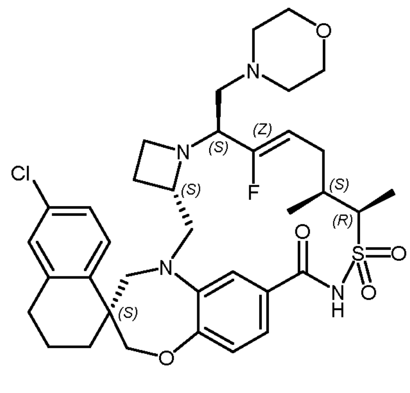

Compound 33 was prepared following an analogous procedure as for Compound 32, using morpholine instead of 2-methyloctahydro-2H-pyrazino[1,2-a]pyrazine.

$^1H$ NMR (400 MHz, CHLOROFORM-*d*) δ ppm 1.06 - 1.11 (m, 3 H) 1.45 (br d, J=6.4 Hz, 3 H) 1.90 - 2.12 (m, 8 H) 2.26 - 2.55 (m, 6 H) 2.57 - 2.63 (m, 1 H) 2.67 - 2.73 (m, 1 H) 2.75 - 2.83 (m, 2 H) 3.04 - 3.52 (m, 6 H) 3.61 - 3.85 (m, 8 H) 4.01 - 4.22 (m, 3 H) 4.72 - 4.95 (m, 1 H) 5.04 - 5.47 (m, 1 H) 6.84 - 6.99 (m, 2 H) 7.07 (br d, J=2.4 Hz, 1 H) 7.09 (d, J=2.0 Hz, 1 H) 7.15 - 7.22 (m, 1 H) 7.69 (d, J=8.6 Hz, 1 H).

Compound 34

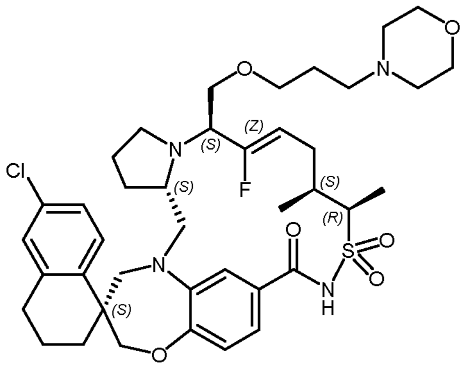

NaH (60 % dispersion in mineral oil, 10 mg, 0.25 mmol, 6.5 eq.) was added cautiously to a stirred solution of Compound 27 (25 mg, 0.0387 mmol) in dry THF (1 mL) at room temperature. After 10 min, 4-(3-bromopropyl)morpholine (CAS [125422-83-5]) (50 mg, 0.173 mmol, 4.5 eq.) was added and the reaction mixture was stirred at 50 °C for 3 h. Upon completion of the reaction, the mixture was cooled to room temperature, diluted with DCM (10 mL) and water was added. Acetic acid was added until the water layer reached pH ca 5. The organic layer was separated, dried ($MgSO_4$), filtered, and evaporated under reduced pressure. The residue was purified by preparative HPLC (Stationary phase: RP XBridge Prep C18 OBD - 10 μm, 30 × 150 mm, Mobile phase: 0.5 % $NH_4Ac$ solution in water + 10 % $CH_3CN$, $CH_3CN$) to give Compound 34 (24 mg, yield: 79 %) as an off-white solid.

$^1H$ NMR (400 MHz, CHLOROFORM-*d*) δ ppm 1.06 (d, J=6.8 Hz, 3 H) 1.41 - 1.46 (m, 4 H) 1.61 - 1.71 (m, 3 H) 1.78 - 1.86 (m, 1 H) 1.90 - 2.03 (m, 5 H) 2.04 - 2.11 (m, 2 H) 2.15 - 2.24 (m, 1 H) 2.68 - 2.80 (m, 8 H) 2.81 - 2.94 (m, 4 H) 3.12 (q, J=7.4 Hz, 1 H) 3.33 (br d, J=14.3 Hz, 2 H) 3.55 - 3.66 (m, 3 H) 3.70 - 3.74 (m, 1 H) 3.87 (br t, J=4.5 Hz, 4 H) 3.90 - 4.02 (m, 3 H) 4.09 (q, J=7.4 Hz, 1 H) 4.18 (d, J=12.3 Hz, 1 H) 4.62 - 4.78 (m, 1 H) 6.94 (s, 2 H) 7.06 - 7.13 (m, 2 H) 7.21 (dd, J=8.6, 2.2 Hz, 1 H) 7.70 (d, J=8.6 Hz, 1 H).

Compound 35

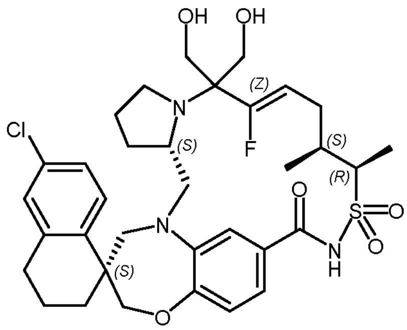

A mixture of Intermediate 63 (100 mg, 0.118 mmol), 1,3-dihydroxyacetone (53 mg, 0.592 mmol, 5 eq.), and $Et_3N$ (0.1 mL, 0.719 mmol, 6 eq.) in DCM (12 mL) was stirred at room temperature. Molecular sieves 4 Å (200 mg) were added and the mixture was stirred at room temperature overnight. The mixture was filtered on dicalite and the filtrate was washed with water, dried on $MgSO_4$, filtered, and evaporated. The residue was purified by preparative HPLC (Stationary phase: RP XBridge Prep C18 OBD-10 μm, 30 × 150 mm, Mobile phase: 0.5 % $NH_4Ac$ solution in water + 10 % $CH_3CN$, $CH_3CN$) to give Compound 35 (13 mg, yield: 16 %).

$^1H$ NMR (400 MHz, CHLOROFORM-*d*) δ ppm 1.03 - 1.07 (m, 3 H) 1.43 - 1.49 (m, 4 H) 1.51 - 1.67 (m, 8 H) 1.77 - 1.89 (m, 4 H) 1.91 - 2.07 (m, 3 H) 2.38 - 2.48 (m, 1 H) 2.73 - 2.81 (m, 2 H) 2.88 - 2.99 (m, 1 H) 3.13 - 3.19 (m, 2 H) 3.21 - 3.28 (m, 1 H) 3.64 - 3.82 (m, 3 H) 3.99 - 4.06 (m, 1 H) 4.12 - 4.19 (m, 1 H) 4.20 - 4.31 (m, 2 H) 4.68 - 4.85 (m, 1 H) 6.81 - 6.87 (m, 1 H) 6.90 - 6.95 (m, 1 H) 7.08 - 7.11 (m, 1 H) 7.16 - 7.23 (m, 2 H) 7.64 - 7.70 (m, 1 H).

Compound 36

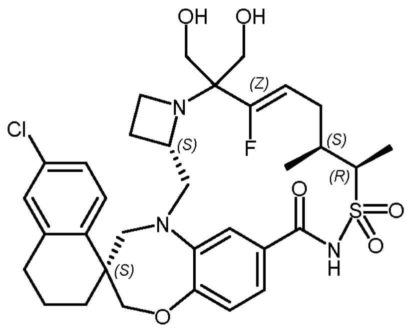

Compound 36 was prepared following an analogous procedure as for Compound 35, using Intermediate 64 instead of Intermediate 63.

$^1$H NMR (400 MHz, CHLOROFORM-*d*) δ ppm 1.03 - 1.08 (m, 3 H) 1.44 - 1.50 (m, 3 H) 1.81 - 2.08 (m, 8 H) 2.18 - 2.27 (m, 2 H) 2.41 - 2.51 (m, 1 H) 2.69 - 2.81 (m, 2 H) 3.21 - 3.33 (m, 2 H) 3.35 - 3.42 (m, 1 H) 3.66 - 3.74 (m, 3 H) 3.77 (s, 2 H) 3.96 (br s, 4 H) 4.02 - 4.15 (m, 2 H) 4.20 - 4.30 (m, 1 H) 4.83 - 5.03 (m, 1 H) 6.89 - 6.95 (m, 2 H) 7.07 - 7.12 (m, 2 H) 7.16 - 7.21 (m, 1 H) 7.65 - 7.70 (m, 1 H).

Compound 37

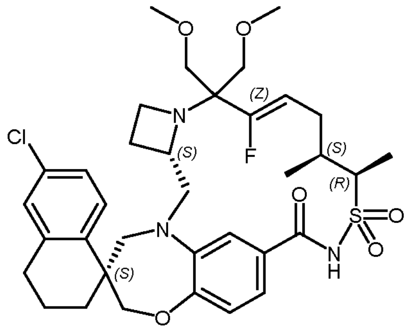

NaH (60 % dispersion in mineral oil, 2 mg, 0.0483 mmol, 4 eq.) was added to a solution of Compound 36 (8 mg, 0.0121 mmol) in THF (1 mL) at room temperature under nitrogen atmosphere. The reaction mixture was stirred for 30 min at room temperature. Iodomethane (8 mg, 0.0604 mmol, 5 eq.) was added and the mixture was stirred for 1 h at 60 °C. The reaction was quenched with water and the THF was evaporated. The aqueous mixture was neutralised with aqueous HCl 1 N (to pH = 6). DCM was added and the mixture was dried by filtration on Extrelut NT3. The filtrate was evaporated and the residue was purified by preparative SFC (Stationary phase: Chiralpak Diacel AD 20 × 250 mm, Mobile phase: $CO_2$, EtOH + 0.4 % $iPrNH_2$) to give Compound 37 (4 mg, yield: 46 %).

$^1$H NMR (400 MHz, CHLOROFORM-*d*) δ ppm 1.02 - 1.09 (m, 3 H) 1.15 - 1.21 (m, 2 H) 1.25 - 1.33 (m, 5 H) 1.39 - 1.45 (m, 3 H) 1.89 - 2.14 (m, 6 H) 2.35 - 2.45 (m, 1 H) 2.65 - 2.84 (m, 2 H) 3.21 - 3.34 (m, 3 H) 3.43 - 3.50 (m, 4 H) 3.50 - 3.66 (m, 3 H) 3.75 - 3.97 (m, 3 H) 3.97 - 4.13 (m, 3 H) 4.96 - 5.19 (m, 1 H) 6.81 - 6.92 (m, 1 H) 6.94 - 7.02 (m, 1 H) 7.05 - 7.10 (m, 1 H) 7.14 - 7.20 (m, 1 H) 7.22 - 7.25 (m, 1 H) 7.66 - 7.72 (m, 1 H).

Compound 37, Compound 38 and Compound 39

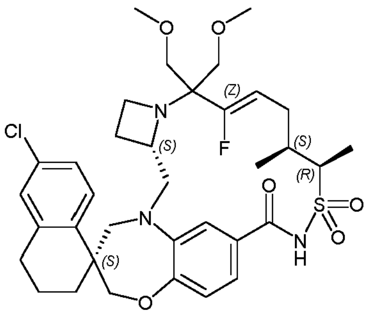

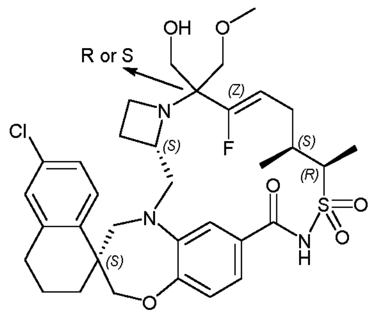

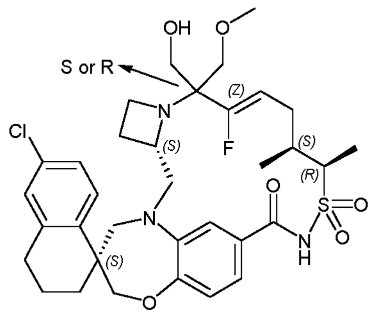

Compound 37 Compound 38 Compound 39

NaH (60 % dispersion in mineral oil, 28 mg, 0.695 mmol, 6 eq.) was added to a solution of Compound 36 (115 mg, 0.174 mmol) in THF (14 mL) at room temperature under nitrogen atmosphere and the mixture was stirred for 30 min at room temperature. Iodomethane (18 mg, 0.13 mmol, 0.75 eq.) was added and the mixture was stirred for 2 h at 80 °C. The reaction was quenched with water and the THF was evaporated. The aqueous solution was extracted with DCM. The organic layer was dried by filtration on ExtrelutNT3, and evaporated. The residue was purified by preparative SFC (Stationary phase: Chiralpak Diacel AD 20 × 250 mm, Mobile phase: $CO_2$, EtOH + 0.4 % $iPrNH_2$) to give Compound 37 (40 mg, yield: 33 %), Compound 38 (4 mg, yield: 4 %), and Compound 39 (13 mg, yield: 11 %).

Compound 38

$^1H$ NMR (400 MHz, CHLOROFORM-*d*) δ ppm 1.07 - 1.13 (m, 3 H) 1.46 - 1.52 (m, 3 H) 1.95 - 2.07 (m, 3 H) 2.08 - 2.17 (m, 1 H) 2.18 - 2.26 (m, 1 H) 2.34 - 2.42 (m, 1 H) 2.46 - 2.57 (m, 2 H) 2.74 - 2.84 (m, 2 H) 3.08 - 3.20 (m, 1 H) 3.30 - 3.50 (m, 2 H) 3.60 - 3.71 (m, 4 H) 3.75 - 3.89 (m, 2 H) 3.92 - 4.09 (m, 3 H) 4.13 - 4.29 (m, 4 H) 4.30 - 4.39 (m, 1 H) 4.56 - 4.77 (m, 1 H) 5.32 - 5.56 (m, 1 H) 5.59 - 5.86 (m, 1 H) 6.90 - 7.00 (m, 2 H) 7.07 - 7.12 (m, 1 H) 7.15 - 7.20 (m, 1 H) 7.28 - 7.32 (m, 1 H) 7.61 - 7.68 (m, 1 H) 7.78 - 7.93 (m, 1 H).

Compound 39

$^1H$ NMR (400 MHz, CHLOROFORM-*d*) δ ppm 1.01 - 1.08 (m, 3 H) 1.37 - 1.42 (m, 1 H) 1.45 - 1.49 (m, 3 H) 1.55 - 1.71 (m, 1 H) 1.74 - 2.10 (m, 7 H) 2.17 (s, 3 H) 2.21 - 2.29 (m, 1 H) 2.42 - 2.52 (m, 1 H) 2.68 - 2.86 (m, 2 H) 3.26 - 3.34 (m, 1 H) 3.37 (s, 3 H) 3.71 - 3.86 (m, 3 H) 3.89 - 4.16 (m, 5 H) 4.18 - 4.47 (m, 1 H) 4.82 - 5.13 (m, 1 H) 5.19 - 5.56 (m, 1 H) 6.89 - 6.97 (m, 2 H) 7.07 - 7.23 (m, 3 H) 7.65 - 7.71 (m, 1 H).

Compound 40, Compound 41 and Compound 42

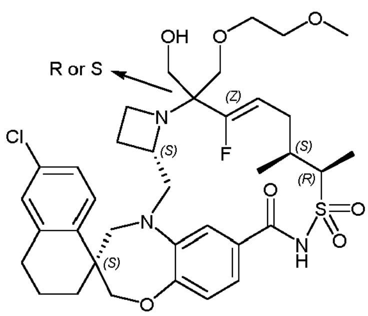

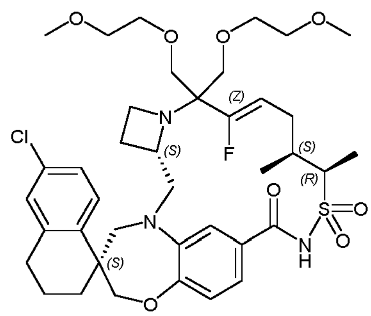

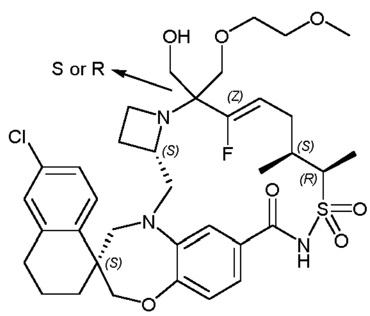

Compound 40 Compound 41 Compound 42

NaH (60 % dispersion in mineral oil, 19 mg, 0.48 mmol, 2 eq.) was added to a solution of Compound 36 (160 mg, 0.24 mmol) in dry DMF (5 mL) and the mixture was stirred at room temperature for 5 min. 2-Bromoethyl methyl ether (67 mg, 0.48 mmol, 2 eq.) was added and the reaction mixture was immediately heated to 80 °C and stirred at

this temperature for 1 h. The reaction mixture was cooled to room temperature and water (100 mL) was added. The mixture was extracted with EtOAc. The organic layer was washed with brine, dried on $MgSO_4$, filtered, and evaporated and coevaporated twice with methylisopropylketone. The residue was purified by column chromatography on silicagel (DCM/MeOH 100/0 to 95/5) followed successively by preparative HPLC (Stationary phase: RP XBridge Prep C18 OBD-10 μm, 50 × 150 mm, Mobile phase: 0.25 % $NH_4HCO_3$ solution in water, $CH_3CN$), and preparative SFC (Stationary phase: Chiralpak Diacel AD 20 × 250 mm, Mobile phase: $CO_2$, EtOH + 0.4 % $iPrNH_2$) to afford Compound 40 (11 mg, yield: 7 %), Compound 41 (10 mg, yield: 5 %), and Compound 42 (29 mg, yield: 17 %).

Compound 40

$^1$H NMR (400 MHz, CHLOROFORM-*d*) δ ppm 0.83 - 0.96 (m, 1 H) 1.02 - 1.08 (m, 3 H) 1.24 - 1.41 (m, 5 H) 1.42 - 1.49 (m, 3 H) 1.75 - 1.90 (m, 1 H) 1.90 - 2.01 (m, 3 H) 2.03 - 2.15 (m, 3 H) 2.35 - 2.48 (m, 1 H) 2.68 - 2.81 (m, 2 H) 3.24 - 3.34 (m, 2 H) 3.54 - 3.62 (m, 2 H) 3.64 - 3.69 (m, 1 H) 3.71 - 3.79 (m, 4 H) 3.80 - 3.93 (m, 5 H) 3.98 - 4.12 (m, 2 H) 4.13 - 4.22 (m, 1 H) 4.90 - 5.09 (m, 1 H) 6.87 - 6.98 (m, 2 H) 7.05 - 7.12 (m, 2 H) 7.14 - 7.20 (m, 1 H) 7.63 - 7.71 (m, 1 H).

Compound 41

$^1$H NMR (400 MHz, CHLOROFORM-*d*) δ ppm 1.01 - 1.11 (m, 3 H) 1.41 - 1.48 (m, 3 H) 1.52 - 1.72 (m, 1 H) 1.76 - 2.13 (m, 8 H) 2.35 - 2.48 (m, 1 H) 2.69 - 2.83 (m, 2 H) 3.26 - 3.29 (m, 3 H) 3.34 (s, 5 H) 3.48 - 3.60 (m, 5 H) 3.61 - 3.73 (m, 5 H) 3.74 - 3.83 (m, 4 H) 3.86 - 3.96 (m, 1 H) 3.97 - 4.05 (m, 2 H) 4.07 - 4.13 (m, 2 H) 4.89 - 5.16 (m, 1 H) 6.85 - 6.91 (m, 1 H) 6.92 - 6.99 (m, 1 H) 7.04 - 7.09 (m, 1 H) 7.12 - 7.21 (m, 2 H) 7.61 - 7.74 (m, 1 H).

Compound 42

$^1$H NMR (400 MHz, CHLOROFORM-*d*) δ ppm 1.00 - 1.10 (m, 3 H) 1.26 - 1.42 (m, 3 H) 1.41 - 1.48 (m, 3 H) 1.77 - 2.10 (m, 7 H) 2.13 - 2.23 (m, 1 H) 2.40 - 2.51 (m, 1 H) 2.69 - 2.84 (m, 2 H) 3.21 - 3.36 (m, 4 H) 3.51 - 3.55 (m, 2 H) 3.56 - 3.61 (m, 2 H) 3.61 - 3.69 (m, 3 H) 3.70 - 3.83 (m, 2 H) 3.83 - 3.91 (m, 2 H) 3.92 - 4.04 (m, 2 H) 4.07 - 4.13 (m, 1 H) 4.14 - 4.26 (m, 1 H) 4.83 - 5.05 (m, 1 H) 6.89 - 6.98 (m, 2 H) 7.06 - 7.13 (m, 2 H) 7.15 - 7.21 (m, 1 H) 7.59 - 7.75 (m, 1 H).

## Analytical Analysis

LCMS

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times ($R_t$) and ions. If not specified differently in the table of data, the reported molecular ion corresponds to the $[M+H]^+$ (protonated molecule) and/or $[M-H]^-$ (deprotonated molecule). In case the compound was not directly ionizable the type of adduct is specified (i.e. $[M+NH_4]^+$, $[M+HCOO]^-$, etc...). For molecules with multiple isotopic patterns (Br, Cl), the reported value is the one obtained for the lowest isotope mass. All results were obtained with experimental uncertainties that are commonly associated with the method used.

Hereinafter, "SQD" means Single Quadrupole Detector, "MSD" Mass Selective Detector, "RT" room temperature, "BEH" bridged ethylsiloxane/silica hybrid, "DAD" Diode Array Detector, "HSS" High Strength silica.

LCMS Method Codes (Flow expressed in mL/min; column temperature (T) in °C; Run time in minutes)

| Method Code | Instrument | column | mobile phase | gradient | Flow<br>----------<br>Col T | Run time |
|---|---|---|---|---|---|---|
| 1 | Waters: Acquity® UPLC® - DAD and SQD | Waters :BEH (1.8 µm, 2.1 * 100 mm) | A: 10 mM $CH_3COONH_4$ in 95 % $H_2O$ + 5 % $CH_3CN$<br>B: $CH_3CN$ | From 100 % A to 5 % A in 2.10 min, to 0 % A in 0.90 min, to 5 % A in 0.5 min | 0.6<br>-------<br>55 | 3.5 |
| 2 | Waters: Acquity® UPLC® - DAD and SQD | Waters :BEH (1.8 µm, 2.1 * 100 mm) | A: 10mM $CH_3COONH_4$ in 95 % $H_2O$ + 5 % $CH_3CN$<br>B: $CH_3CN$ | From 100 % A to 5 % A in 2.10 min, to 0 % A in 0.90 min, to 5 % A in 0.5 min | 0.7<br>-------<br>55 | 3.5 |
| 3 | Waters: Acquity® UPLC® - DAD and SQD | Waters :BEH C18 (1.7 µm, 2.1 * 50 mm) | A: 10 mM $CH_3COONH_4$ in 95 % $H_2O$ + 5 % $CH_3CN$<br>B: $CH_3CN$ | From 95 % A to 5 % A in 1.3 min, held for 0.7 min. | 0.8<br>-------<br>55 | 2 |
| 4 | Waters: Acquity® UPLC® - DAD and SQD | Waters :BEH (1.8 µm, 2.1 * 100 mm) | A: 0.1 % $NH_4HCO_3$ in 95 % $H_2O$ + 5 % $CH_3CN$<br>B: $CH_3CN$ | From 100 % A to 5 % A in 2.10 min, to 0 % A in 0.9 min, to 5 % A in 0.5 min | 0.6<br>-------<br>55 | 4.5 |

(continued)

| Method Code | Instrument | column | mobile phase | gradient | Flow<br>----------<br>Col T | Run time |
|---|---|---|---|---|---|---|
| 5 | Waters: Acquity® UPLC® - DAD and SQD | Waters :BEH (1.8 μm, 2.1 * 100 mm) | A: 0.1 % $NH_4HCO_3$ in 95 % $H_2O$ + 5 % $CH_3CN$<br>B: MeOH | From 100 % A to 5 % A in 2.10 min, to 0 % A in 0.9 min, to 5 % A in 0.5 min | 0.6<br>-------<br>55 | 5.5 |
| 6 | Waters: Acquity® UPLC® - DAD, SQD and ELSD | Waters :HSS T3 (1.8 μm, 2.1 * 100 mm) | A: 10 mM $CH_3COONH_4$ in 95 % $H_2O$ + 5 % $CH_3CN$<br>B: $CH_3CN$ | From 100 % A to 5 % A in 2.10 min, to 0 % A in 0.90 min, to 5 % A in 0.5 min | 0.6<br>-------<br>55 | 3.5 |
| 7 | Waters: Acquity® UPLC® - DAD and SQD | Waters :BEH (1.8 μm, 2.1 * 100 mm) | A: 10 mM $CH_3COONH_4$ in 95 % $H_2O$ + 5 % $CH_3CN$<br>B: MeOH | From 100 % A to 5 % A in 2.10 min, to 0 % A in 0.9 min, to 5 % A in 0.5 min | 0.6<br>-------<br>55 | 3,5 |
| 8 | Waters: Acquity® UPLC® - DAD and SQD | Waters :BEH (1.8 μm, 2.1 * 100 mm) | A: 0.1 % $NH_4HCO_3$ in 95 % $H_2O$ + 5 % $CH_3CN$<br>B: $CH_3CN$ | From 100 % A to 5 % A in 2.10 min, to 0 % A in 0.9 min, to 5 % A in 0.4 min | 0.6<br>-------<br>55 | 3,5 |

LCMS results (RT means retention time)

| Compound number | LCMS results |
|---|---|
| 2 | confirms the MW (RT: 2.25, [M+H]+ 602, LCMS Method 2) |
| 3 | confirms the MW (RT: 2.00, [M+H]+ 584, LCMS Method 2) |
| 4 | confirms the MW (RT: 2.09, [M+H]+ 628, LCMS Method: 1) |

(continued)

| Compound number | LCMS results |
|---|---|
| 5 | confirms the MW (RT: 1.93, [M+H]+ 586, LCMS Method: 1) |
| 6 | confirms the MW (RT: 1.80, [M+H]+ 586, LCMS Method: 1) |
| 7 | confirms the MW (RT: 1.96, [M+H]+ 628, LCMS Method 1) |
| 8 | confirms the MW (RT: 1.98, [M+H]+ 642, LCMS Method 2) |
| 9 | confirms the MW (RT: 1.05, [M+H]+ 632, LCMS Method 3) |
| 10 | confirms the MW (RT: 1.80, [M+H]+ 614, LCMS Method 1) |
| 11 | confirms the MW (RT: 2.14, [M+H]+ 709, LCMS Method 4) |
| 13 | confirms the MW (RT: 1.13, [M+H]+ 646, LCMS Method 3) |
| 14 | confirms the MW (RT: 1.16, [M+H]+ 734, LCMS Method 3) |
| 15 | confirms the MW (RT: 2.03, [M+H]+ 688, LCMS Method 6) |
| 16 | confirms the MW (RT: 1.27, [M+H]+ 660, LCMS Method 3) |
| 17 | confirms the MW (RT: 1.51, [M+H]+ 690, LCMS Method 6) |
| 18 | confirms the MW (RT: 1.15, [M+H]+ 759, LCMS Method 3) |
| 19 | confirms the MW (RT: 1.17, [M+H]+ 765, LCMS Method 3) |
| 20 | confirms the MW (RT: 2.27, [M+H]+ 660, LCMS Method 1) |
| 22 | confirms the MW (RT: 2.16, [M+H]+ 704, LCMS Method 1) |
| 23 | confirms the MW (RT: 2.24, [M+H]+ 744, LCMS Method 6) |
| 24 | confirms the MW (RT: 2.10, [M+H]+ 728, LCMS Method 1) |
| 25 | confirms the MW (RT: 2.06, [M+H]+ 759, LCMS Method 2) |
| 26 | confirms the MW (RT: 2.41, [M+H]+ 700, LCMS Method 1) |
| 27 | confirms the MW (RT: 2.05, [M+H]+ 646, LCMS Method 2) |
| 28 | confirms the MW (RT: 2.15, [M+H]+ 756, LCMS Method 2) |
| 29 | confirms the MW (RT: 1.84, [M+H]+ 690, LCMS Method 6) |
| 30 | confirms the MW (RT: 2.04, [M+H]+ 745, LCMS Method 1) |
| 31 | confirms the MW (RT: 2.52, [M+H]+ 634, LCMS Method 7) |
| 32 | confirms the MW (RT: 1.92, [M+H]+ 769, LCMS Method 8) |
| 33 | confirms the MW (RT: 2.05, [M+H]+ 701, LCMS Method 2) |
| 34 | confirms the MW (RT: 1.21, [M+H]+ 773, LCMS Method 3) |
| 35 | confirms the MW (RT: 1.96, [M+H]+ 676, LCMS Method 2) |
| 36 | confirms the MW (RT: 1.94, [M+H]+ 662, LCMS Method 2) |
| 37 | confirms the MW (RT: 2.15, [M+H]+ 690, LCMS Method 1) |
| 38 | confirms the MW (RT: 1.95, [M+H]+ 676, LCMS Method 1) |
| 39 | confirms the MW (RT: 2.05, [M+H]+ 676, LCMS Method 1) |
| 40 | confirms the MW (RT: 1.02, [M+H]+ 720, LCMS Method 3) |
| 41 | confirms the MW (RT: 2.10, [M+H]+ 778, LCMS Method 1) |
| 42 | confirms the MW (RT: 2.04, [M+H]+ 720, LCMS Method 1) |

SFC-MS methods

The SFC measurement was performed using an Analytical Supercritical fluid chromatography (SFC) system composed by a binary pump for delivering carbon dioxide ($CO_2$) and modifier, an autosampler, a column oven, a diode array detector equipped with a high-pressure flow cell standing up to 400 bars. If configured with a Mass Spectrometer (MS) the flow from the column was brought to the (MS). It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software. Analytical SFC-MS Methods (Flow expressed in mL/min; column temperature (T) in °C; Run time in minutes, Backpressure (BPR) in bars. "$iPrNH_2$" means isopropylamine, "EtOH" means ethanol, "min" mean minutes.

| SFC Method | Column | mobile phase | gradient | Flow<br>-------<br>Col T | Run time<br>-------<br>BPR |
|---|---|---|---|---|---|
| Method 1 | Daicel Chiralpak® AD3 column (3.0 μm, 150 × 4.6 mm) | A:$CO_2$<br>B: EtOH + 0.2 % $iPrNH_2$ | 10%-50% B in 6 min, hold 3.5 min | 2.5<br>-------<br>40 | 9.5<br>-------<br>130 |
| Method 2 | Daicel Chiralpak® AS3 column (3.0 μm, 150 × 4.6 mm) | A:$CO_2$<br>B: EtOH + 0.2 % $iPrNH_2$ | 5 % B hold 6 min, to 50 % in 1 min hold 2.5 min | 2.5<br>-------<br>40 | 9.5<br>-------<br>130 |
| Method 3 | Daicel Chiralpak® AD-H column (3.0 μm, 150 × 4.6 mm) | A:$CO_2$<br>B: EtOH-iPrOH + 0.2 % $iPrNH_2$ | 10%-50% B in 6 min, hold 3.5 min | 2.5<br>-------<br>40 | 9.5<br>-------<br>110 |
| Method 4 | Daicel Chiralpak® IG3 column (3.0 μm, 150 × 4.6 mm) | A:$CO_2$<br>B: EtOH + 0.2 % $iPrNH_2$ | 10%-50% B in 6 min, hold 3.5 min | 2.5<br>-------<br>40 | 9.5<br>-------<br>130 |
| Method 5 | Daicel Chiralpak® AD-H column (3.0 μm, 150 × 4.6 mm) | A:$CO_2$<br>B: EtOH + 0.2 % $iPrNH_2$ | 10%-50% B in 6 min, hold 3.5 min | 2.5<br>-------<br>40 | 9.5<br>-------<br>110 |

Table: Analytical SFC data - $R_t$ means retention time (in minutes), $[M+H]^+$ means the protonated mass of the compound, method refers to the method used for (SFC)MS analysis of enantiomerically pure compounds. No. means number.

| Compound No. | SFC Method | Rt | $[M+H]^+$ |
|---|---|---|---|
| 5 | 1 | 6.38 | 586 |
| 6 | 1 | 7.61 | 586 |

(continued)

| Compound No. | SFC Method | Rt | $[M+H]^+$ |
|---|---|---|---|
| 7 | 2 | 4.08 | 628 |
| 8 | 3 | 6.08 | 642 |
| 28 | 4 | 6.90 | 756 |
| 37 | 5 | 3.60 | 690 |
| 38 | 5 | 4.54 | 676 |
| 39 | 5 | 5.98 | 676 |

NMR

$^1H$ NMR spectra were recorded on Bruker Avance III and Avance NEO spectrometers. $CDCl_3$ was used as solvent, unless otherwise mentioned. The chemical shifts are expressed in ppm relative to tetramethylsilane.

**Pharmacological Analysis**

Biological Example 1

Terbium labeled Myeloid Cell Leukemia 1(Mcl-1) homogeneous time-resolved fluorescence (HTRF) binding assay utilizing the BIM BH3 peptide (H2N-(C/Cy5Mal) WIAQELRRIGDEFN-OH) as the binding partner for Mcl-1.

Apoptosis, or programmed cell death, ensures normal tissue homeostasis, and its dysregulation can lead to several human pathologies, including cancer. Whilst the extrinsic apoptosis pathway is initiated through the activation of cell-surface receptors, the intrinsic apoptosis pathway occurs at the mitochondrial outer membrane and is governed by the binding interactions between pro- and anti-apoptotic Bcl-2 family proteins, including Mcl-1. In many cancers, the anti-apoptotic Bcl-2 protein(s), such as the Mcl-1, are upregulated, and in this way the cancer cells can evade apoptosis. Thus, inhibition of the Bcl-2 protein(s), such as Mcl-1, may lead to apoptosis in cancer cells, providing a method for the treatment of said cancers.

This assay evaluated inhibition of the BH3 domain : Mcl-1 interaction by measuring the displacement of Cy5-labeled BIM BH3 peptide ($H_2N$-(C/Cy5Mal) WIAQELRRIGDEFN-OH) in the HTRF assay format.

Assay Procedure

The following assay and stock buffers were prepared for use in the assay: (a) Stock buffer: 10mM Tris-HCl, pH=7.5 + 150mM NaCl, filtered, sterilized, and stored at 4°C; and (b) 1X assay buffer, where the following ingredients were added fresh to stock buffer: 2 mM dithiothreitol (DTT), 0.0025 % Tween-20, 0.1 mg/mL bovine serum albumin (BSA). The 1X Tb-Mcl-1 + Cy5 Bim peptide solution was prepared by diluting the protein stock solution using the 1X assay buffer (b) to 25 pM Tb-Mcl-1 and 8 nM Cy5 Bim peptide.

Using the Acoustic ECHO, 100 nL of 100x test compound(s) were dispensed into individual wells of a white 384-well Perkin Elmer Proxiplate, for a final compound concentration of 1x and final DMSO concentration of 1 %. Inhibitor control and neutral control (NC, 100 nL of 100 % DMSO) were stamped into columns 23 and 24 of assay plate. Into each well of the plate was then dispensed 10μL of the 1X Tb-Mcl-1 + Cy5 Bim peptide solution. The plate was centrifuged with a cover plate at 1000 rpm for 1 minute, then incubated for 60 minutes at room temperature with plates covered.

The TR-FRET signal was read on an BMG PHERAStar FSX MicroPlate Reader at room temperature using the HTRF optic module (HTRF: excitation: 337nm, light source: laser, emission A: 665nm, emission B: 620nm, integration start: 60 μs, integration time: 400 μs).

Data Analysis

The BMG PHERAStar FSX MicroPlate Reader was used to measure fluorescence intensity at two emission wavelengths - 665nm and 620nm - and report relative fluorescence units (RFU) for both emissions, as well as a ratio of the emissions (665nm/620nm)* 10,000. The RFU values were normalized to percent inhibition as follows:

$$\% \, inhibition = ((NC - IC) - (compound - IC)) / (NC - IC)) * 100$$

where IC (inhibitor control, low signal) = mean signal of 1X Tb-MCl-1 + Cy5 Bim peptide+ inhibitor control or 100 % inhibition of Mcl-1; NC (neutral control, high signal) = mean signal 1X Tb-MCl-1 + Cy5 Bim peptide with DMSO only or 0 % inhibition An 11-point dose response curve was generated to determine $IC_{50}$ values (using GenData) based on the following equation:

$$Y=Bottom + (Top-Bottom)/(1+10^{\wedge}((logIC_{50}-X)*HillSlope))$$

where Y = % inhibition in the presence of X inhibitor concentration; Top = 100 % inhibition derived from the IC (mean signal of Mcl-1 + inhibitor control); Bottom = 0 % inhibition derived from the NC (mean signal of Mcl-1 + DMSO); Hillslope = Hill coefficient; and $IC_{50}$ = concentration of compound with 50 % inhibition in relation to top/neutral control (NC).

$$Ki = IC_{50} / (1 + [L]/Kd)$$

In this assay [L] = 8 nM and Kd = 10 nM

Representative compounds of the present invention were tested according to the procedure as described above, with results as listed in the Table below (n.d. means not determined). The values reported in the table below are subject to error margins associated with the assay used and the equipment.

| Compound | HTRF Ki (nM) |
|---|---|
| 2 | 0.99 |
| 3 | 3.14 |
| 4 | 1.98 |
| 5 | 12.15 |
| 6 | 783.29 |
| 7 | 0.83 |
| 8 | 2.04 |
| 9 | 0.22 |
| 10 | 2.65 |
| 11 | 3.97 |
| 13 | 0.49 |
| 14 | 0.34 |
| 15 | 3.64 |
| 16 | 0.83 |
| 17 | 0.13 |
| 18 | 0.24 |
| 19 | 0.43 |
| 20 | 0.91 |
| 22 | 1.29 |
| 23 | 1.52 |
| 24 | 0.63 |
| 25 | 0.20 |
| 26 | 4.72 |

Representative compounds of the present invention were tested according to the procedure as described above, with results as listed in the Table below (n.d. means not determined). The values reported in the table below

were obtained after recalibration of the equipment. The values are subject to typical error margins, and are averaged values over several runs of a particular compound.

| Compound | HTRF Ki (nM) |
|---|---|
| 1 | 6.39 |
| 2 | 0.84 |
| 3 | n.d. |
| 4 | 3.15 |
| 5 | 5.28 |
| 6 | 2169.84 |
| 7 | 0.61 |
| 8 | 2.64 |
| 9 | 0.23 |
| 10 | n.d. |
| 11 | 2.13 |
| 12 | 2.15 |
| 13 | 0.51 |
| 14 | n.d. |
| 15 | 2.33 |
| 16 | n.d. |
| 17 | 0.17 |
| 18 | 0.24 |
| 19 | 0.36 |
| 20 | n.d. |
| 22 | 0.69 |
| 23 | 0.82 |
| 24 | 0.47 |
| 25 | 0.21 |
| 26 | 2.87 |
| 27 | 0.39 |
| 28 | 0.09 |
| 29 | n.d. |
| 30 | n.d. |
| 31 | 0.86 |
| 32 | n.d. |
| 33 | n.d. |
| 34 | 0.21 |
| 35 | 0.14 |
| 36 | 0.23 |
| 37 | 0.45 |
| 38 | 0.15 |

(continued)

| Compound | HTRF Ki (nM) |
|---|---|
| 39 | 1.84 |
| 40 | 0.16 |
| 41 | 0.25 |
| 42 | 0.90 |

Biological Example 2

MCL-1 is a regulator of apoptosis and is highly over-expressed in tumor cells that escape cell death. The assay evaluates the cellular potency of small-molecule compounds targeting regulators of the apoptosis pathway, primarily MCL-1, Bfl-1, Bcl-2, and other proteins of the Bcl-2 family. Protein-protein inhibitors disrupting the interaction of anti-apoptotic regulators with BH3-domain proteins initiate apoptosis.

Activation of the apoptotic pathway was measured using the CellEvent™ Caspase-3/7 Green ReadyProbes™ Reagent (Thermo Fisher C10423, C10723). This assay produces a green fluorescent stain in cells that enter the apoptosis pathway. CellEvent® Caspase-3/7 Green reagent is a four amino acid peptide (DEVD) conjugated to a nucleic acid-binding dye that is non-fluorescent when not bound to DNA. The CellEvent® Caspase-3/7 Green reagent is intrinsically non-fluorescent, as the DEVD peptide inhibits binding of the dye to DNA. Upon activation of caspase-3/7 in apoptotic cells, the DEVD peptide is cleaved and the free dye can bind DNA, generating a bright green fluorescence. The activation of Caspase-3 and Caspase-7 is downstream of inhibition of MCL-1 or other apoptosis inhibiting proteins in cell lines that are dependent on them.

The live-cell readout on the IncuCyte permits tracking over time of the Caspase activation. The kinetic readout was useful as (a) it reveals differences in time of onset that can be related to differences in the mechanism of apoptosis induction, i.e. this being more direct or indirect; and (b) it allows recognition of artifacts resulting from autofluorescent or precipitating compounds. The IncuCyte readout also allows one to normalize for cell number, as the suspension cells are hard to distribute evenly.

Signals were measured every 2h for a duration of 22h. The ratio of the Caspase mask to the Confluence mask, per image, as raw data, was calculated and the kinetic trace for every well was exported to Genedata Screener for analysis.

In Genedata Screener values for 6h, 12h, and 22h from the kinetic traces were extracted. The values were normalized against negative controls (untreated cells). A standard dose-response analysis was performed on the normalized data.

The following data was reported at each of the following three aforementioned time points: (a) The dose-response curve, (b) The qAC50 and qAC50 Mode, and (c) Max Activity.

Materials used in the assay were as listed in the Table below.

Table - Assay Materials

| ***Reagent*** |
|---|
| MOLP8 cell line (mycoplasma test negative) |
| ViewPlate-384 Black |
| CellEvent™ Caspase-3/7 Green Detection Reagent |
| Breathe-EASIERTM |
| DMSO (dimethyl sulfoxide) |
| RPMI Medium 1640 (1X) without Phenol red and L-Glutamine |
| Heat Inactivated FBS (Fetal bovine serum) |
| L-Glutamine solution |
| Gentamicin |

Cells were maintained in culture medium containing 10 % Heat Inactivated (HI) FBS, 2mM L-Glutamine and 50 μg/mL Gentamycin phenol red free RPMI-1640. Cells were split at 0.4 million /mL twice a week.

On Day 1, plates containing individual wells with test compounds at 10 mM concentration, 150 nL per well. The

final concentrations range from 100 μM to 10 pM compound (and no compound control) and compounds were thawed at room temperature for 1 hour. 25 μl of prewarmed medium was added into each well by multidrop (column 1, 3-22, 24), followed by addition of DMSO control (0.6 % DMSO) in column 2. The plate was sealed using Breathe-Easy® sealing membrane and shaken for 30 min at room temperature to dissolve the test compound(s) in medium. The plate was then kept in the incubator for 1 hour at 37 °C, 5 % $CO_2$.

MOLP8 cells in medium at 40000/25 μl (20000/50 μl final in assay) were prepared with CellEvent™ Caspase-3/7 Green Detection Reagent at 4 μM (2 μM final in assay). Once prepared, the cells were added to the test compound plate in an amount of 20000 and the plate was immediately placed in the IncuCyte and imaging started using following settings: 10X objective, 2 s exposure time in green channel, interval of 2 h, acquisition stopped after 22 h.

For analysis in IncuCyte, a Basic Analysis protocol was defined to calculate the "confluence" and "caspase" areas from the "Phase" and "green" images, respectively, as follows: (a) Confluence: Segmentation Adjustment 1, Hole Fill 0, Adjust Size -2, No filters (b) Caspase: Top-Hat segmentation, Radius 10, Threshold 0.3 GCU, Edge Split On with sensitivity 0, Hole Fill 0, Adjust Size 1, and filter on a minimum Area of 20 $\mu m^2$. The analyzer is trained on a sufficient number of positive and negative control wells, as well as compound treated wells, verifying that the "confluence" layer detects both live and dead (condensed) cells. The "Caspase Area / Confluence Area" approximates the fraction of cells that are positive for the Caspase3/7 stain, calculated "Per Image".

Assay analysis was completed in Genedata Screener, using a predefined template. More particularly, the assay-specific settings for the experiment analysis were as follows: (a) Plate layout: Negative control wells contain no compound but DMSO, and were defined to be "Neutral Control", (b) Trace Channel: There should be one trace channel, name "Measured Channel", of type "Measured". This was the raw data from the IncuCyte; and (c) Layers: Three layers of the type "Aggregated: Time Series", with the names "Mean 6 h", "Mean 12 h" and "Mean 22 h". They contained the mean of the measured from values from 5.5 to 6.5 hours, from 11.5 to 12.5, and from 21.5 to 22.5 hours, respectively.

Normalization and Correction: Each of the three layers was normalized to Percent-of-Control, with Neutral Control as central reference, and Stimulator Control as Scale Reference. Or, if $\mu_{CR}$ was the mean of the Central Reference, and $\mu_{SC}$ was the mean of the Scale Reference then the normalized value was calculated as:

$$\%\text{Activation} = 100\ \% \left(\frac{x_{raw} - \mu_{CR}}{\mu_{SC} - \mu_{CR}}\right)$$

Layer Compound Results: A standard fit model was used as below, with $S_{inf}$, $IC_{50}$ and h as free parameters, and $S_0$ fixed to be 0.

$$\%\text{Activation} = S_0 + \frac{S_{inf} - S_0}{1 + \left(\frac{IC_{50}}{\text{concentration}}\right)^h}$$

The Robust Z' Factor or "RZ' Factor" was calculated in Screener. After excluding outlier kinetic traces in control wells (see below), the RZ' value should be RZ ≥ 0.5 for MOLP8 cells tested at any FBS concentration, and for any of the time points (6 h, 12 h, 22 h).

The "Global SD" was calculated in Screener as the robust standard deviation of the positive or negative controls after normalization (whichever was greater). After excluding outlier kinetic traces in control wells (see below), the Global SD should be Global SD ≤ 10 for MOLP8 cells tested at any FBS concentration, and for any of the time points (6 h, 12 h, 22 h).

Representative compounds of Formula (I) of the present invention were tested according to the procedures described in the Biological Example, with results as listed in the Table below. 'NT' means not tested. The values reported in the table below are subject to error margins associated with the assay used and the equipment.

Table: Measured $AC_{50}$ for Representative Compounds of Formula (I).

| Compound | MOLP8 Caspase 3/7 $AC_{50}$ at 6 h (μM) | MOLP8 Caspase 3/7 $AC_{50}$ at 12 h (μM) | MOLP8 Caspase 3/7 $AC_{50}$ at 22 h (μM) |
|---|---|---|---|
| 1 | 7.76 | ~10.47 | 7.94 |
| 2 | ~0.97 | ~1.05 | 1.20 |
| 3 | 2.4 | 2.69 | 3.09 |

(continued)

| Compound | MOLP8 Caspase 3/7 $AC_{50}$ at 6 h (μM) | MOLP8 Caspase 3/7 $AC_{50}$ at 12 h (μM) | MOLP8 Caspase 3/7 $AC_{50}$ at 22 h (μM) |
|---|---|---|---|
| 4 | ~1.31 | 1.20 | ~1.38 |
| 5 | 6.31 | 6.60 | 5.75 |
| 6 | >30 | >30 | >30 |
| 7 | 2.16 | 1.72 | 1.9 |
| 8 | 2.39 | 2.49 | 2.84 |
| 9 | 0.40 | 0.40 | 0.40 |
| 10 | 3.07 | 3.24 | 3.43 |
| 11 | 2.38 | 2.32 | 2.52 |
| 12 | 1.22 | 1.12 | 1.33 |
| 13 | 0.52 | 0.53 | 0.56 |
| 14 | 0.3 | 0.35 | 0.37 |
| 15 | 2.43 | 2.4 | 2.42 |
| 16 | 0.45 | 0.45 | 0.48 |
| 17 | 11.12 | 10.01 | 9.61 |
| 18 | 0.66 | 0.57 | 0.6 |
| 19 | 0.39 | 0.4 | 0.43 |
| 20 | 0.81 | 0.7 | 0.61 |
| 22 | 0.55 | 0.57 | 0.62 |
| 23 | 1.11 | 1.04 | 1.15 |
| 24 | 0.39 | 0.41 | 0.4 |
| 25 | 0.36 | 0.36 | 0.36 |
| 26 | 3.54 | 3.57 | 3.06 |
| 27 | NT | NT | NT |
| 28 | NT | NT | NT |
| 29 | NT | NT | NT |
| 30 | 0.63 | 0.56 | 0.62 |
| 31 | 1.09 | 0.9 | 0.94 |
| 32 | 1.1 | 0.96 | 0.96 |
| 33 | 0.33 | 0.34 | 0.56 |
| 34 | NT | NT | NT |
| 35 | NT | NT | NT |
| 36 | NT | NT | NT |
| 37 | NT | NT | NT |
| 38 | NT | NT | NT |
| 39 | NT | NT | NT |
| 40 | NT | NT | NT |
| 41 | NT | NT | NT |

(continued)

| Compound | MOLP8 Caspase 3/7 $AC_{50}$ at 6 h (μM) | MOLP8 Caspase 3/7 $AC_{50}$ at 12 h (μM) | MOLP8 Caspase 3/7 $AC_{50}$ at 22 h (μM) |
|---|---|---|---|
| 42 | NT | NT | NT |

Biological Example 3

MCL-1 is a regulator of apoptosis and is highly over-expressed in tumor cells that escape cell death. The assay evaluates the cellular potency of small-molecule compounds targeting regulators of the apoptosis pathway, primarily MCL-1, Bfl-1, Bcl-2, and other proteins of the Bcl-2 family. Protein-protein inhibitors disrupting the interaction of anti-apoptotic regulators with BH3-domain proteins initiate apoptosis.

The Caspase-Glo® 3/7 Assay is a luminescent assay that measures caspase-3 and -7 activities in purified enzyme preparations or cultures of adherent or suspension cells. The assay provides a proluminescent caspase-3/7 substrate, which contains the tetrapeptide sequence DEVD. This substrate is cleaved to release aminoluciferin, a substrate of luciferase used in the production of light. Addition of the single Caspase-Glo® 3/7 Reagent in an "add-mix-measure" format results in cell lysis, followed by caspase cleavage of the substrate and generation of a "glow-type" luminescent signal.

This assay uses the MOLP-8 human multiple myeloma cell line, which is sensitive to MCL-1 inhibition.

Materials:

- Perkin Elmer Envision
- Multidrop 384 and small volume dispensing cassettes
- Centrifuge
- Countess automated cell counter
- Countess counting chamber slides
- Assay plate: ProxiPlate-384 Plus, White 384-shallow well Microplate
- Sealing tape: Topseal A plus
- T175 culture flask

| Product | Units | Storage |
|---|---|---|
| RPMI1640 (no L-Glutamine, no phenol red) | 500 mL | 4 °C |
| Foetal Bovine Serum (FBS) (Heat inactivated) | 500 mL | 4 °C |
| L-Glutamine (200 mM) | 100 ml | -20 °C |
| Gentamicin (50 mg/mL) | 100 mL | 4 °C |
| Caspase 3/7 Detection kit | 100 mL<br>10 × 10 mL | -20 °C |

Cell culture media:

| **MOLP8** | |
|---|---|
| | |
| RPMI-1640 medium | 500 mL |
| 20 % FBS (heat inactivated) | 120 mL |
| 2 mM L-Glutamine | 6.2 mL |
| 50 μg/mL Gentamicin | 620 μL |
| | |

(continued)

| **Assay media** | |
|---|---|
| | |
| RPMI-1640 medium | 500 mL |
| 10 % FBS (Heat inactivated) | 57 mL |
| 2 mM L-Glutamine | 5.7 mL |
| 50 μg/mL Gentamicin | 570 μL |

Cell culture:

Cell cultures were maintained between 0.2 and 2.0 $\times 10^6$ cells/mL. The cells were harvested by collection in 50 mL conical tubes. The cells were then pelleted at 500 g for 5 mins before removing supernatant and resuspension in fresh pre-warmed culture medium. The cells were counted and diluted as needed.

Caspase-Glo reagent:

The assay reagent was prepared by transferring the buffer solution to the substrate vial and mixing. The solution may be stored for up to 1 week at 4 °C with negligible loss of signal.

Assay procedure:

Compounds were delivered in assay-ready plates (Proxiplate) and stored at -20°C. Assays always include 1 reference compound plate containing reference compounds. The plates were spotted with 40 nL of compounds (0.5 % DMSO final in cells; serial dilution; 30 μM highest conc. 1/3 dilution, 10 doses, duplicates). The compounds were used at room temperature and 4 μL of pre-warmed media was added to all wells except column 2 and 23. The negative control was prepared by adding 1 % DMSO in media. The positive control was prepared by adding the appropriate positive control compound in final concentration of 60 μM in media. The plate was prepared by adding 4 μL negative control to column 23, 4 μL positive control to column 2 and 4 μL cell suspension to all wells in the plate. The plate with cells was then incubated at 37 °C for 2 hours. The assay signal reagent is the Caspase-Glo solution described above, and 8 μL was added to all wells. The plates were then sealed and measured after 30 minutes.

The activity of a test compound was calculated as percent change in apoptosis induction as follows:

LC = median of the Low Control values

= Central Reference in Screener

= DMSO

=0%

HC = Median of the High Control values

= Scale Reference in Screener
= 30 μM of positive control
= 100 % apoptosis induction

$$\%\text{Effect } (AC_{50}) = 100 - (\text{sample-LC}) / (\text{HC-LC}) * 100$$

$$\%\text{Control} = (\text{sample} / \text{HC}) * 100$$

$$\%\text{Control min} = (\text{sample-LC}) / (\text{HC-LC}) * 100$$

Table: Measured $AC_{50}$ for Representative Compounds of Formula (I). Averaged values are reported over all runs on all batches of a particular compound. 'NT' means not tested.

| Compound | MOLP8 $AC_{50}$ (nM) |
|---|---|
| 1 | NT |
| 2 | 692.5 |
| 3 | NT |
| 4 | 739.6 |
| 5 | 3744.5 |
| 6 | NT |
| 7 | 851.7 |
| 8 | 1331.4 |
| 9 | 143.7 |
| 10 | 1486.9 |
| 11 | 1489.7 |
| 12 | 519.9 |
| 13 | 181.2 |
| 14 | NT |
| 15 | 1860.0 |
| 16 | 206.7 |
| 17 | 5988.2 |
| 18 | 447.6 |
| 19 | 302.3 |
| 20 | NT |
| 22 | 274.0 |
| 23 | 544.9 |
| 24 | 234.4 |
| 25 | 231.1 |
| 26 | 2516.5 |
| 27 | 786.1 |
| 28 | 120.0 |
| 29 | NT |
| 30 | NT |
| 31 | 512.5 |
| 32 | NT |
| 33 | NT |
| 34 | 457.8 |
| 35 | 547.4 |
| 36 | 2393.3 |
| 37 | 253.4 |

(continued)

| Compound | MOLP8 $AC_{50}$ (nM) |
|---|---|
| 38 | 152.0 |
| 39 | 630.1 |
| 40 | 194.4 |
| 41 | 152.4 |
| 42 | 729.5 |

## Claims

1. A compound of Formula (I)

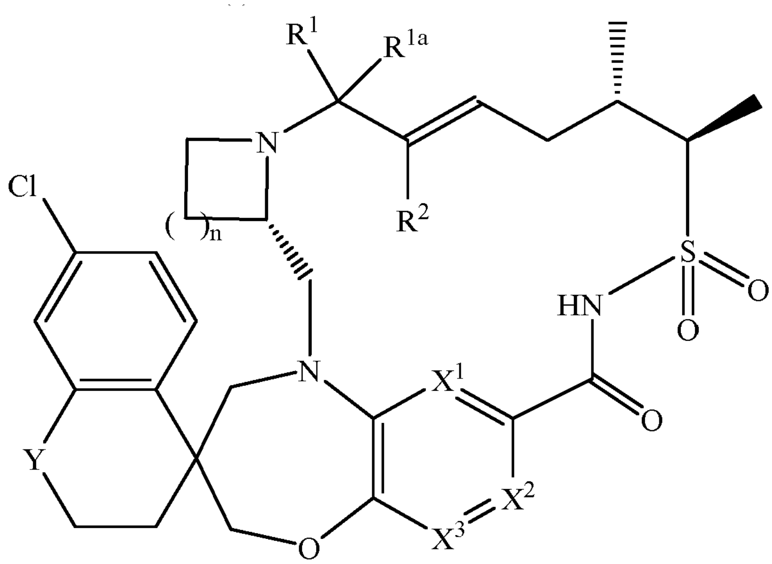

(I)

, wherein

$R^1$ represents hydrogen; $-CH_2OR^a$; $-CH_2F$; or $-CH_2NR^bR^c$;
$R^{1a}$ represents hydrogen; $-CH_2OR^a$; $-CH_2F$; or $-CH_2NR^bR^c$;
$R^a$ is selected from the group consisting of hydrogen; $C_{1-4}$alkyl; $Het^a$;
$C_{1-4}$alkyl substituted with one substituent selected from the group consisting of $-C(=O)-NR^dR^e$, $-C(=O)-OR^f$, $C_{3-6}$cycloalkyl, $Het^b$, and $Het^c$; and
$C_{2-4}$alkyl substituted with one substituent selected from the group consisting of $-(OCH_2CH_2)_m-OCH_3$, $Het^d$, and $-NR^dR^e$;
$R^b$ and $R^c$ are each independently selected from the group consisting of hydrogen, $C_{1-4}$alkyl, and $C_{2-4}$alkyl substituted with one $Het^1$;
or $R^b$ and $R^c$ are taken together to form together with the N-atom to which they are attached a monocyclic 4- to 7-membered fully saturated heterocyclyl containing at least one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing at least one N-atom and optionally one or two additional heteroatoms each independently selected from O, S, and N, wherein said S-atom might be substituted to form S(=O) or $S(=O)_2$;
wherein said monocyclic or fused bicyclic heterocyclyl is optionally substituted on one nitrogen with one $C_{1-4}$alkyl;
wherein said monocyclic or fused bicyclic heterocyclyl is optionally substituted on one carbon atom with one or two substituents each independently selected from the group consisting of $C_{1-4}$alkyl, oxo and halo;
$R^d$ and $R^e$ are each independently selected from the group consisting of hydrogen, $C_{1-4}$alkyl, and $C_{2-4}$alkyl substituted with one $Het^1$;

or $R^d$ and $R^e$ are taken together to form together with the N-atom to which they are attached a monocyclic 4- to 7-membered fully saturated heterocyclyl containing at least one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing at least one N-atom and optionally one or two additional heteroatoms each independently selected from O, S, and N, wherein said S-atom might be substituted to form S(=O) or $S(=O)_2$;

wherein said monocyclic or fused bicyclic heterocyclyl is optionally substituted on one nitrogen with one $C_{1-4}$alkyl;

wherein said monocyclic or fused bicyclic heterocyclyl is optionally substituted on one carbon atom with one or two substituents each independently selected from the group consisting of $C_{1-4}$alkyl, oxo and halo;

$R^f$ represents hydrogen or $C_{1-4}$alkyl;

$R^2$ represents hydrogen or fluoro;

$Het^a$ represents a C-linked 5- or 6-membered monocyclic aromatic heterocyclyl containing one, two or three heteroatoms each independently selected from O, S, and N; wherein one carbon atom is optionally substituted with one $C_{1-4}$alkyl;

$Het^b$ represents a C-linked 4- to 7-membered monocyclic fully saturated heterocyclyl containing one or two heteroatoms each independently selected from O, S, and N; wherein said S-atom might be substituted to form S(=O) or $S(=O)_2$;

$Het^c$ represents a C-linked 5- or 6-membered monocyclic aromatic heterocyclyl containing one, two or three heteroatoms each independently selected from O, S, and N; wherein one carbon atom is optionally substituted with one $C_{1-4}$alkyl;

$Het^d$ represents a N-linked 5-membered monocyclic aromatic heterocyclyl containing one, two or three N-atoms; wherein one carbon atom is optionally substituted with one $C_{1-4}$alkyl; $Het^1$ represents a 4- to 7-membered monocyclic fully saturated heterocyclyl containing one or two heteroatoms each independently selected from O, S, and N; wherein said S-atom might be substituted to form S(=O) or $S(=O)_2$;

n is 1 or 2;

m is 0, 1 or 2;

Y represents O or $CH_2$;

$X^1$ represents CH;

$X^2$ represents CH;

$X^3$ represents CH;

or a pharmaceutically acceptable salt, or a solvate thereof.

**2.** The compound according to claim 1, wherein

$R^{1a}$ represents hydrogen or $-CH_2OR^a$;

$R^b$ and $R^c$ are taken together to form together with the N-atom to which they are attached a monocyclic 4- to 7-membered fully saturated heterocyclyl containing at least one N-atom and optionally one additional heteroatom selected from O, S, and N, wherein said S-atom might be substituted to form S(=O) or $S(=O)_2$; or a fused bicyclic 6- to 11-membered fully saturated heterocyclyl containing at least one N-atom and optionally one or two additional heteroatoms each independently selected from O, S, and N, wherein said S-atom might be substituted to form S(=O) or $S(=O)_2$;

wherein said monocyclic or fused bicyclic heterocyclyl is optionally substituted on one nitrogen with one $C_{1-4}$alkyl;

$R^d$ and $R^e$ are taken together to form together with the N-atom to which they are attached a monocyclic 4- to 7-membered fully saturated heterocyclyl containing at least one N-atom and optionally one additional heteroatom selected from O, S, and N, wherein said S-atom might be substituted to form S(=O) or $S(=O)_2$;

wherein said monocyclic heterocyclyl is optionally substituted on one carbon atom with one or two halo substituents;

$R^f$ represents hydrogen;

$Het^a$ represents a C-linked 5- or 6-membered monocyclic aromatic heterocyclyl containing one, two or three heteroatoms each independently selected from O, S, and N;

m is 0 or 1.

**3.** The compound according to claim 1, wherein

$R^1$ represents hydrogen; $-CH_2OR^a$; or $-CH_2NR^bR^c$;

$R^a$, $R^b$, and $R^c$, are each independently selected from the group consisting of hydrogen and $C_{1-4}$alkyl;

Y represents O or $CH_2$.

4. The compound according to claim 1 or 2, wherein

$R^1$ represents hydrogen or $-CH_2OR^a$;
$R^a$ represents $C_{1-4}$alkyl;
n is 1.

5. The compound according to claim 1, 2 or 3, wherein Y represents O.

6. The compound according to claim 1, 2 or 3, wherein Y represents $CH_2$.

7. The compound according to any one of the preceding claims wherein $R^{1a}$ represents hydrogen.

8. A pharmaceutical composition comprising a compound as claimed in any one of claims 1 to 7 and a pharmaceutically acceptable carrier or diluent.

9. A process for preparing a pharmaceutical composition as defined in claim 8 comprising mixing a pharmaceutically acceptable carrier with a therapeutically effective amount of a compound according to any one of claims 1 to 7.

10. A compound as claimed in any one of claims 1 to 7 or a pharmaceutical composition as claimed in claim 8 for use as a medicament.

11. A compound as claimed in any one of claims 1 to 7 or a pharmaceutical composition as claimed in claim 8 for use in the prevention or treatment of cancer.

12. The compound or a pharmaceutical composition for use according to claim 11, wherein cancer is selected from prostate, lung, pancreatic, breast, ovarian, cervical, melanoma, B-cell chronic lymphocytic leukemia (CLL), acute myeloid leukemia (AML), and acute lymphoblastic leukemia (ALL).

## Patentansprüche

1. Verbindung von Formel (I)

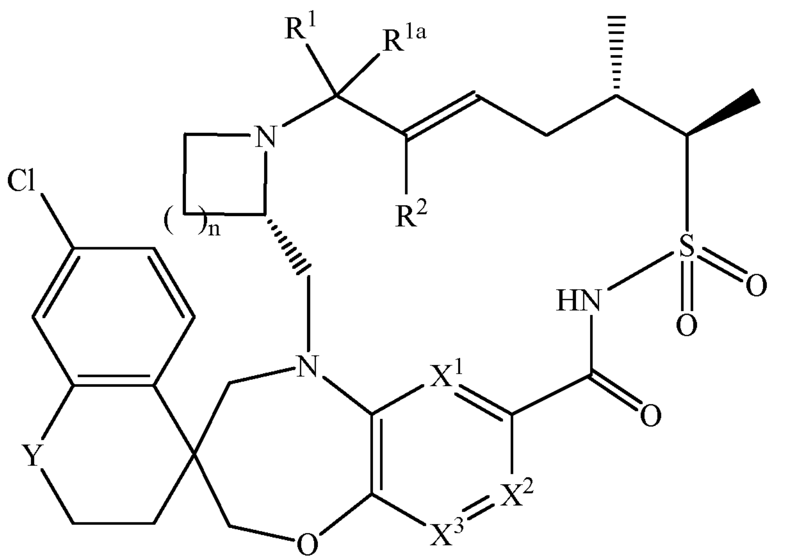

(I)

,

wobei

$R^1$ für Wasserstoff; $-CH_2OR^a$; $-CH_2F$; oder $-CH_2NR^bR^c$ steht;
$R^{1a}$ für Wasserstoff; $-CH_2OR^a$; $-CH_2F$; oder $-CH_2NR^bR^c$ steht;
$R^a$ aus der Gruppe ausgewählt ist, bestehend aus Wasserstoff; $C_{1-4}$-Alkyl; $Het^a$;

$C_{1-4}$-Alkyl, das mit einem Substituenten substituiert ist, der aus der Gruppe ausgewählt ist, bestehend aus $-C(=O)-NR^dR^e$, $-C(=O)-OR^f$, $C_{3-6}$-Cycloalkyl, $Het^b$, und $Het^c$; und

$C_{2-4}$-Alkyl, das mit einem Substituenten substituiert ist, ausgewählt aus der Gruppe, bestehend aus $-(OCH_2CH_2)_m-OCH_3$, $Het^d$, und $-NR^dR^e$;

$R^b$ und $R^c$ unabhängig aus der Gruppe ausgewählt sind, bestehend aus Wasserstoff, $C_{1-4}$-Alkyl und $C_{2-4}$-Alkyl, das mit einem $Het^1$ substituiert ist;

oder $R^b$ und $R^c$ zusammengenommen werden, um zusammen mit dem N-Atom, an das sie gebunden sind, ein monocyclisches 4- bis 7-gliedriges vollständig gesättigtes Heterocyclyl, das mindestens ein N-Atom und gegebenenfalls ein zusätzliches Heteroatom enthält, das aus O, S und N ausgewählt ist, wobei das S-Atom substituiert sein kann, um S(=O) oder $S(=O)_2$ auszubilden; oder ein kondensiertes bicyclisches 6- bis 11-gliedriges vollständig gesättigtes Heterocyclyl auszubilden, das mindestens ein N-Atom und gegebenenfalls ein oder zwei zusätzliche Heteroatome enthält, die jeweils unabhängig aus O, S und N ausgewählt sind, wobei das S-Atom substituiert sein kann, um S(=O) oder $S(=O)_2$ auszubilden;

wobei das monocyclische oder kondensierte bicyclische Heterocyclyl gegebenenfalls an einem Stickstoff mit einem $C_{1-4}$-Alkyl substituiert ist;

wobei das monocyclische oder kondensierte bicyclische Heterocyclyl gegebenenfalls an einem Kohlenstoffatom mit einem oder zwei Substituenten substituiert ist, die jeweils unabhängig aus der Gruppe ausgewählt sind, bestehend aus $C_{1-4}$-Alkyl, Oxo und Halogen;

$R^d$ und $R^e$ unabhängig aus der Gruppe ausgewählt sind, bestehend aus Wasserstoff, $C_{1-4}$-Alkyl und $C_{2-4}$-Alkyl, das mit einem $Het^1$ substituiert ist;

oder $R^d$ und $R^e$ zusammengenommen werden, um zusammen mit dem N-Atom, an das sie gebunden sind, ein monocyclisches 4- bis 7-gliedriges vollständig gesättigtes Heterocyclyl, das mindestens ein N-Atom und gegebenenfalls ein zusätzliches Heteroatom enthält, das aus O, S und N ausgewählt ist, wobei das S-Atom substituiert sein kann, um S(=O) oder $S(=O)_2$ auszubilden; oder ein kondensiertes bicyclisches 6- bis 11-gliedriges vollständig gesättigtes Heterocyclyl auszubilden, das mindestens ein N-Atom und gegebenenfalls ein oder zwei zusätzliche Heteroatome enthält, die jeweils unabhängig aus O, S und N ausgewählt sind, wobei das S-Atom substituiert sein kann, um S(=O) oder $S(=O)_2$ auszubilden;

wobei das monocyclische oder kondensierte bicyclische Heterocyclyl gegebenenfalls an einem Stickstoff mit einem $C_{1-4}$-Alkyl substituiert ist;

wobei das monocyclische oder kondensierte bicyclische Heterocyclyl gegebenenfalls an einem Kohlenstoffatom mit einem oder zwei Substituenten substituiert ist, die jeweils unabhängig aus der Gruppe ausgewählt sind, bestehend aus $C_{1-4}$-Alkyl, Oxo und Halogen;

$R^f$ für Wasserstoff oder $C_{1-4}$-Alkyl steht;

$R^2$ für Wasserstoff oder Fluor steht;

$Het^a$ für ein C-verknüpftes 5- oder 6-gliedriges monocyclisches aromatisches Heterocyclyl steht, das ein, zwei oder drei Heteroatome enthält, die jeweils unabhängig aus O, S und N ausgewählt sind; wobei ein Kohlenstoffatom gegebenenfalls mit einem $C_{1-4}$-Alkyl substituiert ist;

$Het^b$ für ein C-verknüpftes 4- bis 7-gliedriges monocyclisches vollständig gesättigtes Heterocyclyl steht, das ein oder zwei Heteroatome enthält, die jeweils unabhängig aus O, S und N ausgewählt sind; wobei das S-Atom substituiert sein kann, um S(=O) oder $S(=O)_2$ auszubilden;

$Het^c$ für ein C-verknüpftes 5- oder 6-gliedriges monocyclisches aromatisches Heterocyclyl steht, das ein, zwei oder drei Heteroatome enthält, die jeweils unabhängig aus O, S und N ausgewählt sind; wobei ein Kohlenstoffatom gegebenenfalls mit einem $C_{1-4}$-Alkyl substituiert ist;

$Het^d$ für ein N-verknüpftes 5-gliedriges monocyclisches aromatisches Heterocyclyl steht, das ein, zwei oder drei N-Atome enthält; wobei ein Kohlenstoffatom gegebenenfalls mit einem $C_{1-4}$-Alkyl substituiert ist;

$Het^1$ für ein 4- bis 7-gliedriges monocyclisches vollständig gesättigtes Heterocyclyl steht, das ein oder zwei Heteroatome enthält, die jeweils unabhängig aus O, S und N ausgewählt sind; wobei das S-Atom substituiert sein kann, um S(=O) oder $S(=O)_2$ auszubilden;

n 1 oder 2 ist;

m 0, 1 oder 2 ist;

Y für O oder $CH_2$ steht;

$X^1$ für CH steht;

$X^2$ für CH steht;

$X^3$ für CH steht;

oder ein pharmazeutisch verträgliches Salz oder ein Solvat davon.

**2.** Verbindung nach Anspruch 1, wobei

$R^{1a}$ für Wasserstoff oder $-CH_2OR^a$ steht;
$R^b$ und $R^c$ zusammengenommen werden, um zusammen mit dem N-Atom, an das sie gebunden sind, ein monocyclisches 4- bis 7-gliedriges vollständig gesättigtes Heterocyclyl, das mindestens ein N-Atom und gegebenenfalls ein zusätzliches Heteroatom enthält, das aus O, S und N ausgewählt ist, wobei das S-Atom substituiert sein kann, um S(=O) oder $S(=O)_2$ auszubilden; oder ein kondensiertes bicyclisches 6- bis 11-gliedriges vollständig gesättigtes Heterocyclyl auszubilden, das mindestens ein N-Atom und gegebenenfalls ein oder zwei zusätzliche Heteroatome enthält, die jeweils unabhängig aus O, S und N ausgewählt sind, wobei das S-Atom substituiert sein kann, um S(=O) oder $S(=O)_2$ auszubilden;
wobei das monocyclische oder kondensierte bicyclische Heterocyclyl gegebenenfalls an einem Stickstoff mit einem $C_{1-4}$-Alkyl substituiert ist;
$R^d$ und $R^e$ zusammengenommen werden, um zusammen mit dem N-Atom, an das sie gebunden sind, ein monocyclisches 4- bis 7-gliedriges vollständig gesättigtes Heterocyclyl auszubilden, das mindestens ein N-Atom und optional ein zusätzliches Heteroatom enthält, das aus O, S und N ausgewählt ist, wobei das S-Atom substituiert sein kann, um S(=O) oder $S(=O)_2$ auszubilden;
wobei das monocyclische Heterocyclyl gegebenenfalls an einem Kohlenstoffatom mit einem oder zwei Halogensubstituenten substituiert ist;
$R^f$ für Wasserstoff steht;
$Het^a$ für ein C-verknüpftes 5- oder 6-gliedriges monocyclisches aromatisches Heterocyclyl steht, das ein, zwei oder drei Heteroatome enthält, die jeweils unabhängig aus O, S und N ausgewählt sind;
m 0 oder 1 ist.

**3.** Verbindung nach Anspruch 1, wobei

$R^1$ für Wasserstoff; $-CH_2OR^a$; oder $-CH_2NR^bR^c$ steht;
$R^a$, $R^b$, und $R^c$ jeweils unabhängig aus der Gruppe ausgewählt sind, bestehend aus Wasserstoff und $C_{1-4}$-Alkyl;
Y für O oder $CH_2$ steht.

**4.** Verbindung nach Anspruch 1 oder 2, wobei

$R^1$ für Wasserstoff oder $-CH_2OR^a$ steht;
$R^a$ für $C_{1-4}$-Alkyl steht;
n 1 ist.

**5.** Verbindung nach Anspruch 1, 2 oder 3, wobei Y für O steht.

**6.** Verbindung nach Anspruch 1, 2 oder 3, wobei Y für $CH_2$ steht.

**7.** Verbindung nach einem der vorstehenden Ansprüche, wobei $R^{1a}$ für Wasserstoff steht.

**8.** Pharmazeutische Zusammensetzung, umfassend eine Verbindung nach einem der Ansprüche 1 bis 7 und einen pharmazeutisch verträglichen Träger oder Verdünner.

**9.** Verfahren zum Herstellen einer pharmazeutischen Zusammensetzung wie in Anspruch 8 definiert, umfassend ein Mischen eines pharmazeutisch verträglichen Trägers mit einer therapeutisch wirksamen Menge einer Verbindung nach einem der Ansprüche 1 bis 7.

**10.** Verbindung nach einem der Ansprüche 1 bis 7 oder eine pharmazeutische Zusammensetzung nach Anspruch 8 zur Verwendung als ein Medikament.

**11.** Verbindung nach einem der Ansprüche 1 bis 7 oder eine pharmazeutische Zusammensetzung nach Anspruch 8 zur Verwendung bei einer Vorbeugung oder Behandlung von Krebs.

**12.** Verbindung oder pharmazeutische Zusammensetzung zur Verwendung nach Anspruch 11, wobei der Krebs aus Prostata, Lunge, Bauchspeicheldrüse, Brust, Eierstock, Gebärmutterhals, Melanom, chronischer lymphatischer Leukämie (CLL) vom B-Zell-Typ, akuter myeloischer Leukämie (AML) und akuter lymphoblastischer Leukämie (ALL) ausgewählt ist.

## Revendications

**1.** Composé de Formule (I)

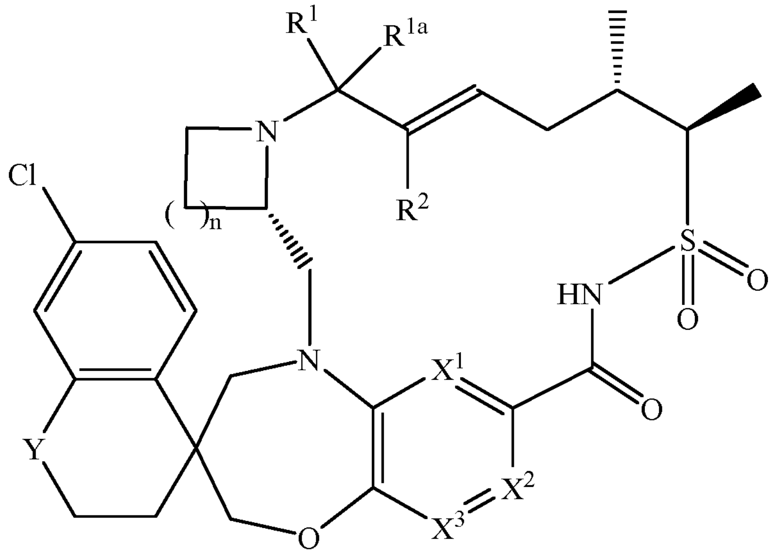

(I)

,

dans lequel

$R^1$ représente hydrogène ; $-CH_2OR^a$ ; $-CH_2F$ ; ou $-CH_2NR^bR^c$ ;
$R^{1a}$ représente hydrogène ; $-CH_2OR^a$ ; $-CH_2F$ ; ou $-CH_2NR^bR^c$ ;
$R^a$ est choisi dans le groupe constitué d'hydrogène ; $C_{1-4}$alkyle ; $Het^a$ ;
$C_{1-4}$alkyle substitué par un substituant choisi dans le groupe constitué de - $C(=O)-NR^dR^e$, $-C(=O)-OR^f$, $C_{3-6}$cycloalkyle, $Het^b$, et $Het^c$ ; et
$C_{2-4}$alkyle substitué par un substituant choisi dans le groupe constitué de - $(OCH_2CH_2)_m-OCH_3$, $Het^d$, et $-NR^dR^e$ ;
$R^b$ et $R^c$ sont chacun choisis indépendamment dans le groupe constitué d'hydrogène, $C_{1-4}$alkyle, et $C_{2-4}$alkyle substitué par un $Het^1$ ;
ou $R^b$ et $R^c$ sont pris ensemble pour former conjointement avec l'atome N auquel ils sont attachés un hétérocyclyle complètement saturé à 4 à 7 chaînons monocyclique contenant au moins un atome N et facultativement un hétéroatome supplémentaire choisi parmi O, S, et N, dans lequel ledit atome S pourrait être substitué pour former S(=O) ou $S(=O)_2$ ; ou un hétérocyclyle totalement saturé à 6 à 11 chaînons bicyclique condensé contenant au moins un atome N et facultativement un ou deux hétéroatomes supplémentaires chacun choisis indépendamment parmi O, S, et N, dans lequel ledit atome S pourrait être substitué pour former S(=O) ou $S(=O)_2$ ;
dans lequel ledit hétérocyclyle monocyclique ou bicyclique condensé est facultativement substitué sur un azote par un $C_{1-4}$alkyle ;
dans lequel ledit hétérocyclyle monocyclique ou bicyclique condensé est facultativement substitué sur un atome de carbone par un ou deux substituants chacun choisis indépendamment dans le groupe constitué de $C_{1-4}$alkyle, oxo et halo ;
$R^d$ et $R^e$ sont chacun choisis indépendamment dans le groupe constitué d'hydrogène, $C_{1-4}$alkyle, et $C_{2-4}$alkyle substitué par un $Het^1$ ;
ou $R^d$ et $R^e$ sont pris ensemble pour former conjointement avec l'atome N auquel ils sont attachés un hétérocyclyle complètement saturé à 4 à 7 chaînons monocyclique contenant au moins un atome N et facultativement un hétéroatome supplémentaire choisi parmi O, S, et N, dans lequel ledit atome S pourrait être substitué pour former S(=O) ou $S(=O)_2$ ; ou un hétérocyclyle totalement saturé à 6 à 11 chaînons bicyclique condensé contenant au moins un atome N et facultativement un ou deux hétéroatomes supplémentaires chacun choisis indépendamment parmi O, S, et N, dans lequel ledit atome S pourrait être substitué pour former S(=O) ou $S(=O)_2$ ;
dans lequel ledit hétérocyclyle monocyclique ou bicyclique condensé est facultativement substitué sur un azote par un $C_{1-4}$alkyle ;
dans lequel ledit hétérocyclyle monocyclique ou bicyclique condensé est facultativement substitué sur un atome de carbone par un ou deux substituants chacun choisis indépendamment dans le groupe constitué de $C_{1-4}$alkyle, oxo et halo ;
$R^f$ représente hydrogène ou $C_{1-4}$alkyle ;

$R^2$ représente hydrogène ou fluoro ;
$Het^a$ représente un hétérocyclyle aromatique monocyclique à 5 ou 6 chaînons lié par C contenant un, deux ou trois hétéroatomes chacun choisis indépendamment parmi O, S, et N ; dans lequel un atome de carbone est facultativement substitué par un $C_{1-4}$alkyle ;
$Het^b$ représente un hétérocyclyle complètement saturé monocyclique à 4 à 7 chaînons lié par C contenant un ou deux hétéroatomes chacun choisis indépendamment parmi O, S, et N ; dans lequel ledit atome S pourrait être substitué pour former S(=O) ou $S(=O)_2$ ;
$Het^c$ représente un hétérocyclyle aromatique monocyclique à 5 ou 6 chaînons lié par C contenant un, deux ou trois hétéroatomes chacun choisis indépendamment parmi O, S, et N ; dans lequel un atome de carbone est facultativement substitué par un $C_{1-4}$alkyle ;
$Het^d$ représente un hétérocyclyle aromatique monocyclique à 5 chaînons lié par N contenant un, deux ou trois atomes N ; dans lequel un atome de carbone est facultativement substitué par un $C_{1-4}$alkyle ;
$Het^1$ représente un hétérocyclyle complètement saturé monocyclique à 4 à 7 chaînons contenant un ou deux hétéroatomes chacun choisis indépendamment parmi O, S, et N ; dans lequel ledit atome S pourrait être substitué pour former S(=O) ou $S(=O)_2$ ;
n vaut 1 ou 2 ;
m vaut 0, 1 ou 2 ;
Y représente O ou $CH_2$ ;
$X^1$ représente CH ;
$X^2$ représente CH ;
$X^3$ représente CH ;
ou un sel pharmaceutiquement acceptable, ou un solvate de celui-ci.

**2.** Composé selon la revendication 1, dans lequel

$R^{1a}$ représente hydrogène ou -CH2ORa ;
$R^b$ et $R^c$ sont pris ensemble pour former conjointement avec l'atome N auquel ils sont attachés un hétérocyclyle complètement saturé à 4 à 7 chaînons monocyclique contenant au moins un atome N et facultativement un hétéroatome supplémentaire choisi parmi O, S, et N, dans lequel ledit atome S pourrait être substitué pour former S(=O) ou $S(=O)_2$ ; ou un hétérocyclyle totalement saturé à 6 à 11 chaînons bicyclique condensé contenant au moins un atome N et facultativement un ou deux hétéroatomes supplémentaires chacun choisis indépendamment parmi O, S, et N, dans lequel ledit atome S pourrait être substitué pour former S(=O) ou $S(=O)_2$ ; dans lequel ledit hétérocyclyle monocyclique ou bicyclique condensé est facultativement substitué sur un azote par un $C_{1-4}$alkyle ;
$R^d$ et $R^e$ sont pris ensemble pour former conjointement avec l'atome N auquel ils sont attachés un hétérocyclyle complètement saturé à 4 à 7 chaînons monocyclique contenant au moins un atome N et facultativement un hétéroatome supplémentaire choisi parmi O, S, et N, dans lequel ledit atome S pourrait être substitué pour former S(=O) ou $S(=O)_2$ ;
dans lequel ledit hétérocyclyle monocyclique est facultativement substitué sur un atome de carbone par un ou deux substituants halo ;
$R^f$ représente hydrogène ;
$Het^a$ représente un hétérocyclyle aromatique monocyclique à 5 ou 6 chaînons lié par C contenant un, deux ou trois hétéroatomes chacun choisis indépendamment parmi O, S, et N ;
m vaut à 0 ou 1.

**3.** Composé selon la revendication 1, dans lequel

$R^1$ représente hydrogène ; $-CH_2OR^a$ ; ou $-CH_2NR^bR^c$ ;
$R^a$, $R^b$, et $R^c$ sont chacun choisis indépendamment dans le groupe constitué d'hydrogène et $C_{1-4}$alkyle ;
Y représente O ou $CH_2$.

**4.** Composé selon la revendication 1 ou 2, dans lequel

$R^1$ représente hydrogène ou $-CH_2OR^a$ ;
$R^a$ représente $C_{1-4}$alkyle ;
n vaut 1.

**5.** Composé selon la revendication 1, 2 ou 3, dans lequel Y représente O.

6. Composé selon la revendication 1, 2 ou 3, dans lequel Y représente $CH_2$.

7. Composé selon l'une quelconque des revendications précédentes, dans lequel $R^{1a}$ représente hydrogène.

8. Composition pharmaceutique comprenant un composé selon l'une quelconque des revendications 1 à 7 et un support ou diluant pharmaceutiquement acceptable.

9. Procédé permettant de préparer une composition pharmaceutique telle que définie dans la revendication 8 comprenant le mélange d'un support pharmaceutiquement acceptable avec une quantité thérapeutiquement efficace d'un composé selon l'une quelconque des revendications 1 à 7.

10. Composé selon l'une quelconque des revendications 1 à 7 ou composition pharmaceutique selon la revendication 8 pour utilisation en tant que médicament.

11. Composé selon l'une quelconque des revendications 1 à 7 ou composition pharmaceutique selon la revendication 8 pour utilisation dans la prévention ou le traitement d'un cancer.

12. Composé ou composition pharmaceutique pour utilisation selon la revendication 11, dans lequel le cancer est choisi parmi cancer de la prostate, du poumon, du pancréas, du sein, de l'ovaire, du col de l'utérus, mélanome, leucémie lymphoïde chronique à lymphocytes B (LLC), leucémie myéloïde aiguë (LMA), et leucémie lymphoblastique aiguë (LLA).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018178226 A
- WO 2017182625 A
- WO 2018178227 A
- WO 2019046150 A
- WO 2016033486 A
- WO 2019036575 A
- WO 2017147410 A
- WO 2018183418 A
- WO 2019222112 A
- WO 2020097577 A


### Non-patent literature cited in the description


- **CHENG et al.** *eLife,* 2016, vol. 5, e17755
- **JULIAN et al.** *Cell Death and Differentiation,* 2017, vol. 24, 1380-1389
- **HANAHAN ; WEINBERG.** *Cell,* 2011, vol. 1, 44
- **BEROUKHIM et al.** *Nature,* 2010, vol. 463 (7283), 899-905
- **YECIES et al.** *Blood,* 2010, vol. 115 (16), 3304-3313
- **ROBERTS et al.** *NEJM,* 2016, vol. 374, 311-322
- **KOTSCHY et al.** *Nature,* 2016, vol. 538, 477-486
- **MERINO et al.** *Sci. Transl. Med,* 2017, (9
- **CHEN et al.** *JCI,* 2018, vol. 128 (1), 500-516
- **WANG et al.** *Genes and Dev,* 2013, vol. 27, 1351-1364
- **STEIMER et al.** *Blood,* 2009, (113), 2805-2815
- **CHARRON, CARLIE L. et al.** *Tetrahedron Lett.,* 2016, vol. 57 (37), 4119-4127
- **AUSTIN R. et al.** *Cancer Letters,* 2016
- **T. W. GREENE ; P. G. M. WUTS.** Protective Groups in Organic Synthesis. Wiley, 2007
- Remington's Pharmaceutical Sciences. **GENNARO et al.** Pharmaceutical preparations and their Manufacture. Mack Publishing Company, 1990
- *CHEMICAL ABSTRACTS,* 72287-26-4
- *CHEMICAL ABSTRACTS,* 54149-17-6
- *CHEMICAL ABSTRACTS,* 68957-94-8
- *CHEMICAL ABSTRACTS,* 3240-94-6
- *CHEMICAL ABSTRACTS,* 38078-09-0
- *CHEMICAL ABSTRACTS,* 63285-62-1
- *CHEMICAL ABSTRACTS,* 125422-83-5